# EUROPEAN PATENT APPLICATION

(11) **EP 4 431 493 A1**
(43) Date of publication of application: **18.09.2024**
(21) Application number: 22894817.0
(22) Date of filing: 16.11.2022
(51) Int. Cl.: C07D 205/04, C07D 213/30, C07D 401/02, C07D 401/14, C07D 409/02, C07D 403/14, C07C 65/26, C07C 63/49, A61K 31/44, A61K 31/4025, A61K 31/397, A61K 31/404, A61P 19/02, A61P 29/00, A61P 35/00, A61P 31/22

(54) **SMALL MOLECULE INHIBITOR AGAINST NASOPHARYNGEAL CARCINOMA, PREPARATION METHOD FOR COMPOUND AND APPLICATION THEREOF**

(30) Priority: 16.11.2021 CN 202111358657
(71) Applicant: Shenzhen Bay Laboratory, Shenzhen, Guangdong 518132 (CN); Peking University Shenzhen Graduate School, Shenzhen, Guangdong 518055 (CN)
(72) Inventor: ZHU, Zhendong, Westborough, MA 01581 (US); XU, Zhengshuang, Shenzhen, Guangdong 518055 (CN); LI, Ting, Shenzhen, Guangdong 518132 (CN); CHE, Chao, Shenzhen, Guangdong 518055 (CN); REN, Xingye, Shenzhen, Guangdong 518132 (CN); CHEN, Sigui, Shenzhen, Guangdong 518132 (CN); ZHANG, Jiayin, Shenzhen, Guangdong 518132 (CN); ZHANG, Ben, Shenzhen, Guangdong 518132 (CN); YANG, Qi, Shenzhen, Guangdong 518132 (CN); ZHANG, Chuanbing, Shenzhen, Guangdong 518132 (CN); YANG, Zhen, Shenzhen, Guangdong 518055 (CN)
(74) Representative: J A Kemp LLP
(86) International application number: PCT/CN2022/132174
(87) International publication number: WO 2023/088285

(57) **Abstract**

A small molecule inhibitor targeting EB virus antigen protein, and/or a pharmaceutical composition comprising same, capable of being used for treating diseases caused by EB virus infection, such as, but not limited to, cancer, infectious mononucleosis, chronic fatigue syndrome, multiple sclerosis, systemic lupus erythematosus or rheumatoid arthritis, especially nasopharyngeal carcinoma. The present invention further provides a small molecule inhibitor against nasopharyngeal carcinoma, and/or a pharmaceutical composition comprising same, capable of being used for treating nasopharyngeal carcinoma.

## Description

### TECHNICAL FIELD

The present invention provides a preparation method for a small molecule inhibitor and a compound against nasopharyngeal carcinoma, and an application for treating and/or preventing tumor disease such as nasopharyngeal carcinoma.

### BACKGROUND

Nasopharyngeal carcinoma is one of multiple tumors in Southern China, which is poorly differentiated or undifferentiated carcinoma derived from nasopharyngeal mucosal epithelial cells and is a highly malignant tumor. Nasopharyngeal carcinoma has obvious racial tendency, with the majority being Chinese and their descendants. In China, its incidence rate is highest in Guangdong Province, followed by Guangxi, Hunan, Fujian and Jiangxi provinces. Nasopharyngeal carcinoma can occur in various age groups, with a peak incidence at the age of 30-50.

Studies have confirmed that the incidence of nasopharyngeal carcinoma is highly correlated with Epstein-Barr virus (EBV) infection. Epstein-Barr virus (EBV) is Human Herpes Virus 4 (HHV-4), belonging to the γ-Herpesvirus subfamily. EBV is a B-lymphotropic virus that commonly infects humans. The initial EBV infection occurs in oropharyngeal squamous epithelial cells, and exists in B lymphocytes in a latent infection state for a long time thereafter. Latent EBV, once activated, can become a pathogenic factor associated with many diseases, including tumors. Studies have found that, besides causing Burkitt's lymphoma, EBV is also associated with Hodgkin's disease, non-Hodgkin's lymphoma, nasopharyngeal carcinoma, NK/T lymphoma, leiomyosarcoma, and malignant epithelial tumor of stomach, breast, and lung and other sites. Therefore, EBV is listed as a Class I human oncogenic virus by IARC (1997).

EBNA1 (viral nuclear antigen 1) is detected in the case where the above-mentioned tumor cells are detected to be infected with EBV Studies have shown that EBNA1 is a protein that is expressed by EBV virus and related to important pathological processes, such as viral DNA replication process, maintainance of virus latency and mutation-induced tumors, initiation of tumor cell migration and induction of immune escape.

In recent years, some progress has been made in the development of targeted chemotherapy drugs targeting EBNA1 for specific tumor, which lays a foundation for target evaluation of EBNA1 protein, structure-based drug design, small molecule activity evaluation, etc. For example, Messick TE et al. applied for two world patents WO2016183534A1 and WO2015073864A1 in 2015 and 2016, disclosing a compound structure of two aromatic rings bearing conjugated alkynes, which were used to inhibit the binding of EBNA1 to DNA and block the replication of EBV-infected cells, thereby having anti-tumor effects. In 2019, the team reported detailed research content, in which its VK series of molecules have good inhibitory activity against EBNA1, showing good effects at the cellular level and in mouse nasopharyngeal carcinoma models. Currently, the series of molecules is at the stage of clinical-phase I (Sci. Transl. Med. 2019, 11, eaau5612.). This achievement proves that EBNA1 protein can be used as a novel drug target for the development of diseases related to EBV infection, including the research of therapeutic drugs for tumors.

The present invention aims to develop small molecule drugs for anti-nasopharyngeal carcinoma, using the structure-based drug design method. The small molecule pharmaceutical compound of the present invention has good selectivity in inhibiting the proliferation of EBV positive tumor cells chains at the cell level, and at the same time exhibits excellent drug ADMET properties and has better druggability. For the inhibitory activities of other EBV positive or negative tumor proliferation, it also shows that the small molecule of the present invention has broad application prospects.

### SUMMARY

The present invention provides a compound of general formula (I), or its enantiomer, diastereomer, tautomer, salt, crystal form, solvate and/or isotope-substituted derivative, as an anti-nasopharyngeal carcinoma inhibitor:

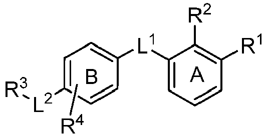

The present invention further provides a pharmaceutical composition, including at least one of the compound of the present invention, or its enantiomer, diastereomer, tautomer, salt, crystal form, solvate and/or isotope-substituted derivative. The present invention further provides a pharmaceutical composition, including at least one of the compound of the present invention, or its enantiomer, diastereomer, tautomer, salt, crystal form, solvate and/or isotope-substituted derivative and at least one of a pharmaceutically acceptable carrier, excipient or diluent. The present invention further provides a method for treating and/or preventing diseases or disorders caused by EBNA1 activity. The present invention further provides a use of preparation of a medicament for treating and/or preventing diseases or disorders caused by EBNA1 activity. The present invention further provides a method for treating and/or preventing nasopharyngeal carcinoma. The present invention further provides a use of preparation of a medicament for treating and/or preventing nasopharyngeal carcinoma. The present invention further provides a method for treating and/or preventing lytic and/or latent EBV infection. The present invention further provides a use of preparation of a medicament for treating and/or preventing diseases caused by lytic and/or latent EBV infection. The present invention further provides a use of preparation of a medicament for treating and/or preventing diseases caused by other non-EBV infecton. The present invention further provides a method for preparing the compound of general formula (I) of the present invention.

The present invention relates to a compound of general formula (I) or its enantiomers, diastereomers, tautomers, salts, crystal forms, solvates and/or isotope-substituted derivatives: 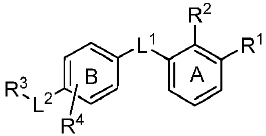 where:
R¹ is selected from -H, -COOH, -C(=O)-O-R^{1a}, -C(=O)-NHR^{1b}, and -C(=O)-NR^{1b}R^{1c};
where,
R^{1a}, R^{1b}, and R^{1c} are the same or different and are each independently selected from: hydrogen, optionally substituted C1-C4 linear alkyl, optionally substituted C3-C4 branched alkyl, optionally substituted C3-C4 cycloalkyl, optionally substituted halogenated C1-C4 linear alkyl, optionally substituted halogenated C3-C4 branched alkyl, and optionally substituted halogenated C3-C4 cycloalkyl; or,
R^{1b} is selected from: hydroxyl, and C1-C4 alkoxy; or,
R^{1b} and R^{1c}, taken together with atoms to which they are attached, form a cyclic group; the cyclic group being selected from: optionally substituted pyrrolidinyl, optionally substituted piperidinyl, optionally substituted piperazinyl, or optionally substituted morpholinyl;
preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, optionally substituted C1-C4 linear alkyl, optionally substituted C3-C4 branched alkyl, optionally substituted halogenated C1-C4 linear alkyl, and optionally substituted halogenated C3-C4 branched alkyl; or,
R^{1b} is selected from: hydroxyl, and C1-C4 alkoxy; or,
R^{1b} and R^{1c}, taken together with atoms to which they are attached, form a cyclic group; the cyclic group is selected from: optionally substituted pyrrolidinyl, and optionally substituted piperidinyl;
more preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, methyl, ethyl, propyl, isopropyl, and butyl; or,
R^{1b} is selected from: hydroxyl, methoxy, ethoxy, and propyloxy; or,
R^{1b} and R^{1c}, taken together with atoms to which they are attached, form a cyclic group; the cyclic group is selected from: pyrrolidinyl;
more preferably, R¹ is selected from -H, -COOH, -COOCH₃, -COOCH₂CH₃, -CONHOH, -CONHCH₃, -CON(CH₃)₂, -CON(c-C₄H₈), and -CONHOCH₃;
R² is selected from the following groups:
   hydrogen, halogen, optionally substituted C1-C4 alkyl, optionally substituted pyrrolyl, optionally substituted indolyl, and optionally substituted phenyl;
   preferably, R² is selected from: hydrogen, chlorine, methyl, pyrrolyl, indolyl, phenyl, chlorophenyl, hydroxyphenyl, and hydroxyalkoxyphenyl;
L¹ is selected from the following groups: 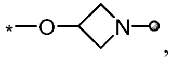 and ethynyl;
where, the round dot 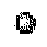 represents a junction where L¹ is linked to the A ring in the compound of general formula (I), the A ring being located on a right side of L¹;
where, the asterisk * represents a junction where L¹ is linked to the B ring in the compound of general formula (I), the B ring being located on a left side of L¹;
L² is selected from the following groups:
   •-(CH₂)_{q}-O-(CH₂)ₚ-*, •-(CH₂)_{q}-NH-(CH₂)ₚ-*, •-NH-C(=O)-NH-*, •-N(CH₃)-C(=O)-NH-*, -CH=CH-, -(CH₂)ₙ-, •-NH-C(=O)-(CH₂)ₚ-*, and •-(CH₂)_{q}-C(=O)-NH-*;
preferably, L² is selected from: •-CH₂-O-*, •-O-CH₂-*, -O-, -CH₂-O-CH₂-, •-CH₂-NH-*, •-CH₂-NH-CH₂-*, •-NH-CH₂-*, -NH-C(=O)-NH-, •-N(CH₃)-C(=O)-NH-*, -CH=CH-, -CH₂-, -CH₂-CH₂-, •-NH-C(=O)-*, •-NH-C(=O)-CH₂-*, •-C(=O)-NH-*, and •-CH₂-C(=O)-NH-*;
further preferably, L² is selected from: •-CH₂-O-*, -O-CH₂-*, -O-, -CH₂-O-CH₂-, •-CH₂-NH-*, •-NH-C(=O)-NH-*, •-N(CH₃)-C(=O)-NH-*, -CH=CH-, -CH₂-, -CH₂-CH₂-, •-NH-C(=O)-*, •-NH-C(=O)-CH₂-*, and •-CH₂-C(=O)-NH-*;
where the round dot • represents a junction where L² is linked to the B ring in the compound of general formula (I), the B ring being located on a right side of L²;
where the asterisk * represents a junction where L² is linked to R³ in the compound of general formula (I) , the R³ being located on a left side of L²;
p and q are each independently 0 or 1 or 2;
n is 1 or 2 or 3;
R³ is selected from: optionally substituted aryl and heteroaryl;
the aryl is selected from: phenyl;
the heteroaryl is selected from: thienyl, pyrazolyl, imidazolyl, isothiazolyl, pyridyl, pyrimidyl, pyrazinyl, and quinolinyl;
the aryl and heteroaryl are optionally substituted by hydrogen, fluorine, chlorine, cyano, C1-C4 alkyl, halogenated C1-C4 alkyl, C1-C4 alkoxy, and halogenated C1-C4 alkoxy one or more times identically or differently;
preferably, the aryl and heteroaryl group is optionally substituted by hydrogen, fluorine, cyano, methyl, methoxy, and trifluoromethyl one or more times identically or differently;
more preferably, the aryl is optionally substituted by hydrogen, cyano, and trifluoromethyl one or more times identically or differently;
more preferably, the heteroaryl is optionally substituted by hydrogen, fluorine, methyl, and methoxy one or more times identically or differently;
further preferably, the pyridyl is optionally substituted by hydrogen, fluorine, methyl, and methoxy one or more times identically or differently;
more further preferably, R³ is selected from: phenyl, thienyl, pyrazolyl, imidazolyl, isothiazolyl, pyridyl, pyrimidinyl, pyrazinyl, quinolinyl, cyanophenyl, trifluoromethylphenyl, fluoropyridyl, methylpyridyl, methoxypyridyl, methylpyrazolyl; R⁴ is selected from: hydrogen, halogen, optionally substituted C1-C4 linear alkyl, optionally substituted C3-C4 branched alkyl, optionally substituted halogenated C1-C4 linear alkyl, optionally substituted halogenated C3-C4 branched alkyl, and hydroxyl;
preferably, R⁴ is selected from: hydrogen, fluorine, chlorine, methyl, and hydroxyl, substituted one or more times identically or differently.

The present invention further relates to a pharmaceutical composition, including the compound of the above general formula (I), or its enantiomer, diastereomer, tautomer, salt, crystal form, solvate and/or isotope-substituted derivative.

The present invention further relates to a pharmaceutical composition, including the compound of the above general formula (I), or its enantiomer, diastereomer, tautomer, salt, crystal form, solvate and/or isotope-substituted derivative, and a pharmaceutically acceptable carrier, excipient or diluent.

The present invention further relates to a method for treating and/or preventing diseases or disorders caused by EBNA1 activity, and the method includes administering to a subject an effective amount of the compound of the above general formula (I), or its enantiomer, diastereomer, tautomer, salt, crystal form, solvate and/or isotope-substituted derivative, or a pharmaceutical composition thereof.

The present invention further relates to a use of the compound of the above general formula (I), or its enantiomer, diastereomer, tautomer, salt, crystal form, solvate and/or isotope-substituted derivative or a pharmaceutical composition thereof for preparation of a medicament for treating and/or preventing diseases caused by EBNA1 activity.

The present invention further relates to a method for treating and/or preventing nasopharyngeal carcinoma, and the method includes administering to a subject an effective amount of the compound of the above general formula (I), or its enantiomer, diastereomer, tautomer, salt, crystal form, solvate and/or isotope-substituted derivative, or a pharmaceutical composition thereof.

The present invention further relates to a use of the compound of the above general formula (I), or its enantiomer, diastereomer, tautomer, salt, crystal form, solvate and/or isotope-substituted derivative, or pharmaceutical composition thereof for preparation of a medicament for treating and/or preventing nasopharyngeal carcinoma.

The present invention relates to the compound of the above general formula (I), or its enantiomer, diastereomer, tautomer, salt, crystal form, solvate and/or isotope-substituted derivative, or pharmaceutical composition thereof, and in the above method, it can be used to treat and/or prevent the following diseases: disease or disorder caused by EBNA1 activity. The aforementioned disease or disorder is at least one selected from the following: cancer, infectious mononucleosis, chronic fatigue syndrome, multiple sclerosis, systemic lupus erythematosus, rheumatoid arthritis and the like. Particularly, the cancer is nasopharyngeal carcinoma, non-Hodgkin's lymphoma, anaplastic large cell lymphoma, angioimmunoblastic T-cell lymphoma, hepatosplenic T-cell lymphoma, B-cell lymphoma, Burkitt's lymphoma , reticuloendothelial proliferation, reticulocytosis, diffuse large B-cell lymphoma, extranodal T/NK lymphoma/angiocentric lymphoma, follicular lymphoma, immunoblastic lymphoma, mucosa-associated lymphoma tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma lymphoma, primary effusion lymphoma, lymphomatoid granuloma, angioimmunoblastic lymphadenopathy, X-linked lymphoproliferative disease, post-transplant lymphoproliferative disease, or Hodgkin lymphoma and the like.

### DESCRIPTION OF EMBODIMENTS

In the detailed description of the present invention, numerous specific details have been elaborated for explanatory purposes to enable the person skilled in the art to understand the disclosed embodiments. However, the person skilled in the art will understand that the specific details of these embodiments do not constitute limitations on the scope of protection of the present invention. In addition, the person skilled in the art can easily understand that the specific order of the relevant descriptions and implementation methods of the present invention is only illustrative, and the relevant order may be changed, but it is still within the spirit and scope of the disclosed embodiments of the present invention.

Unless defined otherwise, all technical and scientific terms used herein have the same meaning as commonly understood by the person skilled in the art. When the definitions of terms used in incorporated references differ from those provided in the description of the present invention, the definitions provided in the present description shall prevail.

Throughout the description and the claims, the following terms take the meanings explicitly associated herein, unless the context clearly dictates otherwise.

The phrase "in one embodiment" as used herein does not necessarily refer to the same embodiment, although it may refer to the same embodiment. Furthermore, the phrase "in another embodiment" as used herein does not necessarily refer to a different embodiment, although it may refer to a different embodiment. Accordingly, various embodiments of the present invention may be easily combined without departing from the scope or spirit of the present invention.

As used herein, the term "EBNA1 (Epstein-Barr virus nuclear antigen1) inhibitor" refers to a compound that inhibits EBNA1.

As used herein, the term "EBV" refers to EB (Epstein-Barr) virus.

As used herein, an "effective amount", "therapeutically effective amount" or "pharmaceutically effective amount" of a compound is an amount of the compound sufficient to provide a beneficial effect to the subject to whom the compound is administered.

As used herein, the term "pharmaceutically acceptable carrier" means a pharmaceutically acceptable material, composition or carrier, such as a liquid or solid filler, stabilizer, dispersant, suspending agent, diluent, excipient, thickener, solvent or encapsulating material, involved in carrying or delivering a compound that can be used in the present invention within or to a subject so that it can perform its intended function.

As used herein, the term "alkyl", refers to a linear, branched, and cyclic alkyl having the specified number of carbon atom (i.e., C1-8 refers to 1 to 8 carbons). Examples of alkyl include methyl, ethyl, n-propyl, isopropyl, n-butyl, t-butyl, isobutyl, sec-butyl, n-pentyl, n-hexyl, n-heptyl, n-octyl, etc. Alkyl groups can be optionally substituted. Non-limiting examples of substituted alkyl groups include hydroxymethyl, chloromethyl, trifluoromethyl, aminomethyl, 1-chloroethyl, 2-hydroxyethyl, 1,2-difluoroethyl, 3-carboxypropyl, etc.

As used herein, the term "alkoxy", refers to the group -O-alkyl, where the alkyl group is defined as above. Alkoxy groups can be optionally substituted. The term C3-C8 cycloalkoxy refers to a ring containing 3 to 8 carbon atoms and at least one oxygen atom (e.g., tetrahydrofuran, tetrahydro-2H-pyran). The C3-C8 cycloalkoxy group can be optionally substituted.

As used herein, the term "haloalkyl" refers to a linear and branched saturated aliphatic hydrocarbon group with a designated number of carbon atoms, which is substituted by one or more halogens. The haloalkyl group include a perhaloalkyl group in which all hydrogens of the alkyl group have been substituted by halogen (e.g., -CF₃, CF₂CF₃). The haloalkyl group can be optionally substituted with one or more substituents other than halogen. Examples of haloalkyl groups include, but are not limited to, fluoromethyl, dichloroethyl, trifluoromethyl, trichloromethyl, pentafluoroethyl, and pentachloroethyl groups.

As used herein, the term "halogen" refers to chlorine, bromine, fluorine and iodine.

As used herein, the term "heteroaryl" refers to a ring group in which at least one ring member is a heteroatom selected from N, O and S, preferably, the number of the heteroatom is 1, 2, 3 or 4. For example, the heteroaryl may be a 5-10 membered oxygen-containing heteroaryl, a 5-10 membered sulfur-containing heteroaryl, and a 5-10 membered nitrogen-containing heteroaryl. Specific examples include, but are not limited to, thienyl, furyl, pyrrolyl, pyrazolyl, imidazolyl, isothiazolyl, pyrazinyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, benzothiazolyl, etc.

As used herein, the term "optionally substituted" refers to the substitution of any substituent that is replaceable, or the substitution that does not occur.

At various places in the present description, substituents of compounds are disclosed as a group or a range. Specifically, the description includes each individual sub-combination of members of such group and range. For example, the term "C1-C8 alkyl" is specifically intended to disclose individually C1, C2, C3, C4, C5, C6, C7, C8, C1-C8, C1-C7, C1-C6, C1-C5, C1- C4, C1-C3, C1-C2, C2-C8, C2-C7, C2-C6, C2-C5, C2-C4, C2-C3, C3-C8, C3-C7, C3-C6, C3-C5, C3-C4, C4-C8, C4-C7, C4-C6, C4-C5, C5-C8, C5-C7, C5-C6 alkyl. The term "C1-C6 alkyl" is specifically intended to disclose individually C1, C2, C3, C4, C5, C6, C1-C6, C1-C5, C1-C4, C1-C3, C1-C2, C2-C6, C2-C5, C2-C4, C2-C3, C3-C6, C3-C5, C3-C4, C4-C6, C4-C5 and C5-C6 alkyl. The term "C1-C4 alkyl" is specifically intended to disclose individually C1, C2, C3, C4, C1-C4, C1-C3, C1-C2, C2-C4, C2-C3, and C3-C4 alkyl.

At various places in the present description, the term "aryl" refers to phenyl, naphthyl, and anthracenyl; the term "heteroaryl" refers to thienyl, furyl, pyrrolyl, pyrazolyl, imidazolyl, isothiazolyl, pyrazinyl, oxazolyl, triazolyl, thiazolyl, pyridyl, pyrimidinyl, indolyl, quinolinyl, quinazolinyl, indazolyl, benzofuryl, benzothienyl, benzoxazolyl, benzotriazolyl, and benzothiazolyl.

The present invention relates to a compound of general formula (I), or its enantiomer, diastereomer, tautomer, salt, crystal form, solvate and/or isotope-substituted derivative: 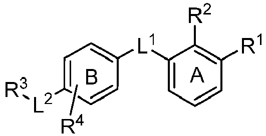 where:
R¹ is selected from -H, -COOH, -C(=O)-O-R^{1a}, -C(=O)-NHR^{1b}, and -C(=O)-NR^{1b}R^{1c};
where,
R^{1a}, R^{1b}, and R^{1c} are the same or different and are each independently selected from: hydrogen, optionally substituted C1-C4 linear alkyl, optionally substituted C3-C4 branched alkyl, optionally substituted C3-C4 cycloalkyl, optionally substituted halogenated C1-C4 linear alkyl, optionally substituted halogenated C3-C4 branched alkyl, and optionally substituted halogenated C3-C4 cycloalkyl; or,
R^{1b} is selected from: hydroxyl and C1-C4 alkoxy; or,
R^{1b} and R^{1c}, taken together with atoms to which they are attached, form a cyclic group; the cyclic group being selected from: optionally substituted pyrrolidinyl, optionally substituted piperidinyl, optionally substituted piperazinyl, and optionally substituted morpholinyl;
preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, optionally substituted C1-C4 linear alkyl, optionally substituted C3-C4 branched alkyl, optionally substituted halogenated C1-C4 linear alkyl, and optionally substituted halogenated C3-C4 branched alkyl; or,
R^{1b} is selected from: hydroxyl, and C1-C4 alkoxy; or,
R^{1b} and R^{1c}, taken together with atoms to which they are attached, form a cyclic group; the cyclic group is selected from: optionally substituted pyrrolidinyl, and optionally substituted piperidinyl;
more preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, methyl, ethyl, propyl, isopropyl, and butyl; or,
R^{1b} is selected from: hydroxyl, methoxy, ethoxy, and propyloxy; or,
R^{1b} and R^{1c}, taken together with atoms to which they are attached, form a cyclic group; the cyclic group is selected from: pyrrolidinyl;
more preferably, R¹ is selected from -H, -COOH, -COOCH₃, -COOCH₂CH₃, -CONHOH, -CONHCH₃, -CON(CH₃)₂, -CON(c-C₄H₈), and -CONHOCH₃;
R² is selected from the following groups:
   hydrogen, halogen, optionally substituted C1-C4 alkyl, optionally substituted pyrrolyl, optionally substituted indolyl, and optionally substituted phenyl;
   preferably, R² is selected from: hydrogen, chlorine, methyl, pyrrolyl, indolyl, phenyl, chlorophenyl, hydroxyphenyl, and hydroxyalkoxyphenyl;
   L¹ is selected from the following groups: 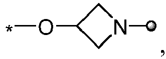 and ethynyl;
   where the round dot 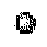 represents where L¹ is linked to the A ring in the compound of general formula (I), the A ring being located on a right side of L¹;
   where the asterisk * represents a junction where L¹ is linked to the B ring in the compound of general formula (I), the B ring being located on a left side of L¹;
   L² is selected from the following groups:
      •-(CH₂)_{q}-O-(CH₂)ₚ-*, •-(CH₂)_{q}-NH-(CH₂)ₚ-*, •-NH-C(=O)-NH-*, •-N(CH₃)-C(=O)-NH-*, -CH=CH-, -(CH₂)ₙ-, •-NH-C(=O)-(CH₂)ₚ-*, and •-(CH₂)_{q}-C(=O)-NH-*;
   preferably, L² is selected from: •-CH₂-O-*, •-O-CH₂-*, -O-, -CH₂-O-CH₂-, •-CH₂-NH-*, •-CH₂-NH-CH₂-*, •-NH-CH₂-*, -NH-C(=O)-NH-, •-N(CH₃)-C(=O)-NH-*, -CH=CH-, -CH₂-, -CH₂-CH₂-, •-NH-C(=O)-*, •-NH-C(=O)-CH₂-*, •-C(=O)-NH-*, and •-CH₂-C(=O)-NH-*;
   further preferably, L² is selected from: •-CH₂-O-*, -O-CH₂-*, -O-, -CH₂-O-CH₂-, •-CH₂-NH-*, •-NH-C(=O)-NH-*, •-N(CH₃)-C(=O)-NH-*, -CH=CH-, -CH₂-, -CH₂-CH₂-, •-NH-C(=O)-*, •-NH-C(=O)-CH₂-*, and •-CH₂-C(=O)-NH-*;
where the round dot • represents a junction where L² is linked to the B ring in the compound of general formula (I), the B ring being located on a right side of L²;
where the asterisk * represents a junction where L² is linked to R³ in the compound of general formula (I) , the R³ being located on a left side of L²;
p and q are each independently 0 or 1 or 2;
n is 1 or 2 or 3;
R³ is selected from: optionally substituted aryl and heteroaryl;
the aryl is selected from: phenyl;
the heteroaryl is selected from: thienyl, pyrazolyl, imidazolyl, isothiazolyl, pyridyl, pyrimidyl, pyrazinyl, and quinolinyl;
the aryl and heteroaryl are optionally substituted by hydrogen, fluorine, chlorine, cyano, C1-C4 alkyl, halogenated C1-C4 alkyl, C1-C4 alkoxy, or halogenated C1-C4 alkoxy one or more times identically or differently;
preferably, the aryl and heteroaryl groups are optionally substituted by hydrogen, fluorine, cyano, methyl, methoxy, or trifluoromethyl one or more times identically or differently;
more preferably, the aryl is optionally substituted by hydrogen, cyano, or trifluoromethyl one or more times identically or differently;
more preferably, the heteroaryl is optionally substituted by hydrogen, fluorine, methyl, or methoxy one or more times identically or differently;
further preferably, the pyridyl is optionally substituted by hydrogen, fluorine, methyl, or methoxy one or more times identically or differently;
further more preferably, R³ is selected from: phenyl, thienyl, pyrazolyl, imidazolyl, isothiazolyl, pyridyl, pyrimidinyl, pyrazinyl, quinolinyl, cyanophenyl, trifluoromethylphenyl, fluoropyridyl, methylpyridyl, methoxypyridyl, and methylpyrazolyl;
R⁴ is selected from: hydrogen, halogen, optionally substituted C1-C4 linear alkyl, optionally substituted C3-C4 branched alkyl, optionally substituted halogenated C1-C4 linear alkyl, optionally substituted halogenated C3-C4 branched alkyl, and hydroxyl;
preferably, R⁴ is selected from: hydrogen, fluorine, chlorine, methyl and hydroxyl, substituted one or more times identically or differently.

In another preferred embodiment, the present invention relates to the compound of above general formula (I), or its enantiomer, diastereomer, tautomer, salt, crystal form, solvate and/or isotope-substituted derivative, where:
when L² is selected from -(CH₂)_{q}-O-(CH₂)ₚ-,
preferably, L² is selected from: •-CH₂-O-*, •-O-CH₂-*, -O- or -CH₂-O-CH₂-;
R³ is selected from aryl and heteroaryl;
the aryl is selected from: phenyl;
the heteroaryl is selected from: thienyl, pyrazolyl, isothiazolyl, pyridyl, pyrimidyl, pyrazinyl, and quinolinyl;
the aryl and heteroaryl is optionally substituted by hydrogen, C1-C4 alkyl, or C1-C4 alkoxy one or more times identically or differently;
preferably, the aryl and heteroaryl is optionally substituted by hydrogen, methyl or methoxy one or more times identically or differently;
preferably, the R³ is selected from: phenyl, thienyl, pyrazolyl, isothiazolyl, pyridyl, pyrimidyl, pyrazinyl, quinolinyl, methylpyridinyl, methoxypyridyl, and methylpyrazolyl;
more preferably, L² is selected from: •-CH₂-O-*; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl;
more preferably, L² is selected from: •-CH₂-O-*; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl; further more preferably, R³ is selected from: pyridyl;
more preferably, L² is selected from: -O-; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl; further more preferably, R³ is selected from: pyridyl;
more preferably, L² is selected from: -CH₂-O-CH₂-; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl; further more preferably, R³ is selected from: pyridyl;
   or
when L² is selected from -(CH₂)_{q}-NH-(CH₂)ₚ-, •-NH-C(=O)-(CH₂)ₚ-*, •-(CH₂)_{q}-C(=O)-NH-*, and -CH=CH-,
preferably, L² is selected from: •-CH₂-NH-*, •-CH₂-NH-CH₂-*, •-NH-CH₂-*, •-NH-C(=O)-*, •-NH-C(=O)-CH₂-*, •-C(=O)-NH-*, •-CH₂-C(=O)-NH-*, and -CH=CH-;
R³ is selected from aryl and heteroaryl;
the aryl is selected from: phenyl;
the heteroaryl is selected from: pyridyl;
more preferably, L² is selected from: •-CH₂-NH-*, •-CH₂-NH-CH₂-*, and •-NH-CH₂-*; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl;
more preferably, L² is selected from: •-CH₂-NH-*, R³ is selected from: pyridyl, methylpyridinyl, and methoxypyridyl; further more preferably, L² is selected from: •-CH₂-NH-*, R³ is selected from: pyridyl;
more preferably, L² is selected from: •-NH-C(=O)-*, and •-NH-C(=O)-CH₂-*; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl; further more preferably, R³ is selected from: pyridyl;
more preferably, L² is selected from: •-C(=O)-NH-* and •-CH₂-C(=O)-NH-*; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, L² is selected from: •-CH₂-C(=O)-NH-*, R³ is selected from: phenyl, pyridyl, methylpyridyl, and methoxypyridyl; further more preferably, L² is selected from: •-CH₂-C(=O)-NH-*, and R³ is selected from: phenyl, and pyridyl;
more preferably, L² is selected from: -CH=CH-; R³ is selected from: phenyl, thienyl, pyrazolyl, isothiazolyl, pyridyl, pyrimidinyl, pyrazinyl, quinolinyl, methylpyridinyl, methoxypyridyl, and methylpyrazolyl; further preferably, R³ is selected from: phenyl, pyridyl, methylpyridyl, and methoxypyridyl; further more preferably, R³ is selected from: pyridyl, methylpyridyl, and methoxypyridyl; further more preferably, R³ is selected from: pyridyl;
   or
when L² is selected from -NH-C(=O)-NH- and •-N(CH₃)-C(=O)-NH-*,
R³ is selected from aryl and heteroaryl;
the aryl is selected from: phenyl;
the heteroaryl is selected from: pyridyl;
the aryl and heteroaryl are substituted by hydrogen, fluorine, cyano, C1-C4 alkyl, or halogenated C1-C4 alkyl one or more times identically or differently;
preferably, the aryl and heteroaryl are substituted by hydrogen, fluorine, cyano, methyl, methoxy, or trifluoromethyl one or more times identically or differently; preferably, the R³ is selected from: cyanophenyl, trifluoromethylphenyl, pyridyl, fluoropyridyl, methylpyridyl, methoxypyridyl, trifluoromethylpyridyl, and trifluoromethoxypyridyl;
more preferably, L² is selected from: -NH-C(=O)-NH-; R³ is selected from: cyanophenyl, trifluoromethylphenyl, pyridyl, fluoropyridyl, methylpyridyl, methoxypyridyl, trifluoromethylpyridyl, and trifluoromethoxypyridyl; further preferably, R³ is selected from: trifluoromethylphenyl, pyridyl, fluoropyridyl, and methylpyridyl.
more preferably, L² is selected from: •-N(CH₃)-C(=O)-NH-*; R³ is selected from: cyanophenyl, trifluoromethylphenyl, pyridyl, fluoropyridyl, methylpyridyl, methoxypyridyl, trifluoromethylpyridyl, and trifluoromethoxypyridyl; further preferably, R³ is selected from: cyanophenyl, trifluoromethylphenyl, pyridyl, fluoropyridyl, and methylpyridyl; further more preferably, R³ is selected from: cyanophenyl;
   or
when L² is selected from -(CH₂)ₙ-;
preferably, L² is selected from: -CH₂-, and -CH₂-CH₂-;
R³ is selected from aryl, and heteroaryl;
the aryl is selected from: phenyl;
the heteroaryl is selected from: pyridyl, and imidazolyl;
more preferably, L² is selected from: -CH₂-; R³ is selected from: phenyl, pyridyl, and imidazolyl; further preferably, R³ is selected from: imidazolyl;
more preferably, L² is selected from: -CH₂-CH₂-; R³ is selected from: phenyl, pyridyl, and imidazolyl; further preferably, R³ is selected from: pyridyl.

In another preferred embodiment, the present invention relates to the compound of above general formula (I), or its enantiomer, diastereomer, tautomer, salt, crystal form, solvate and/or isotope-substituted derivative, where:
when L¹ is selected from oxy-azetidinyl,
R² is selected from: hydrogen, halogen, optionally substituted C1-C4 alkyl, optionally substituted pyrrolyl, optionally substituted indolyl, and optionally substituted phenyl;
preferably, R² is selected from: hydrogen, chlorine, methyl, pyrrolyl, indolyl, phenyl, and chlorophenyl;
L² is selected from: -(CH₂)_{q}-O-(CH₂)ₚ-, -(CH₂)_{q}-NH-(CH₂)ₚ-, -NH-C(=O)-NH-, •-N(CH₃)-C(=O)-NH-*, -CH=CH-, -(CH₂)ₙ-, •-NH-C(=O)-(CH₂)ₚ-* and •-(CH₂)_{q}-C(=O)-NH-*;
R³ is selected from: phenyl, thienyl, pyrazolyl, imidazolyl, isothiazolyl, pyridyl, pyrimidinyl, pyrazinyl, quinolinyl, cyanophenyl, trifluoromethylphenyl, fluoropyridyl, methylpyridyl, methoxypyridyl, and methylpyrazolyl;
more preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, optionally substituted C1-C4 linear alkyl, optionally substituted C3-C4 branched alkyl, optionally substituted halogenated C1-C4 linear alkyl, and optionally substituted halogenated C3-C4 branched alkyl; or,
R^{1b} is selected from: hydroxyl, and C1-C4 alkoxy; or,
R^{1b} and R^{1c}, taken together with atoms to which they are attached, form a cyclic group; the cyclic group being selected from: optionally substituted pyrrolidinyl, and optionally substituted piperidinyl;
more preferably, R¹ is selected from -H, -COOH, -C(=O)-O-R^{1a}, -C(=O)-NHR^{1b}, and -C(=O)-NR^{1b}R^{1c};
R^{1a}, R^{1b}, and R^{1c} are the same or different and are each independently selected from: hydrogen, methyl, ethyl, propyl, isopropyl, and butyl; or,
R^{1b} is selected from: hydroxyl, methoxy, ethoxy, and propoxy; or,
R^{1b} and R^{1c}, taken together with atoms to which they are attached, form a cyclic group; the cyclic group being selected from: pyrrolidinyl;
further preferably, R¹ is selected from -H, -COOH, -COOCH₃, -COOCH₂CH₃, -CONHOH, -CONHCH₃, -CON(CH₃)₂, -CON(c-C₄H₈), and -CONHOCH₃;
more preferably, when R³ is selected from: phenyl, cyanophenyl, and trifluoromethylphenyl, L² is selected from: -NH-C(=O)-NH-, •-N(CH₃)-C(=O)-NH-*, and •-(CH₂)_{q}-C(=O)-NH-*; R² is selected from: pyrrolyl, and chlorophenyl; R¹ is selected from -H, -COOH, -COOCH₃, and -CONHOH;
more preferably, when R³ is selected from: pyridyl, fluoropyridyl, methylpyridyl, and methoxypyridyl, L² is selected from: -(CH₂)_{q}-O-(CH₂)ₚ-, -(CH₂)_{q}-NH-(CH₂)ₚ-, -NH-C(=O)-NH-, •-N(CH₃)-C(=O)-NH-*, -CH=CH-, -(CH₂)ₙ-, •-NH-C(=O)-(CH₂)ₚ-*, and •-(CH₂)_{q}-C(=O)-NH-*; R² is selected from: hydrogen, chlorine, methyl, pyrrolyl, indolyl , phenyl, and chlorophenyl; R¹ is selected from -H, -COOH, -COOCH₃, -COOCH₂CH₃, -CONHOH, -CONHCH₃, -CON(CH₃)₂, -CON(c-C₄H₈), and -CONHOCH₃;
further preferably, when L² is selected from: •-CH₂-O-*, •-O-CH₂-*, -O-, and -CH₂-O-CH₂-; R³ is selected from: pyridyl, methylpyridyl, and methoxypyridyl; R² is selected from: pyrrolyl, phenyl, and chlorophenyl; R¹ is selected from: -H, -COOH, -COOCH₃, -COOCH₂CH₃, -CONHOH, -CONHCH₃, -CON(CH₃)₂, and -CON(c-C₄H₈);
further preferably, when L² is selected from: •-CH₂-NH-*, •-CH₂-NH-CH₂-*, and •-NH-CH₂-*; R³ is selected from: pyridyl, methylpyridyl, and methoxypyridyl; R² is selected from: pyrrolyl, phenyl, and chlorophenyl; R¹ is selected from -H, -COOH, -COOCH₃, and -CONHOH; further more preferably, R³ is selected from: pyridyl;
further preferably, when L² is selected from: -NH-C(=O)-NH-, and •-N(CH₃)-C(=O)-NH-*; R³ is selected from: pyridyl, fluoropyridyl, and methylpyridyl; R² is selected from: hydrogen, pyrrolyl, phenyl, and chlorophenyl; R¹ is selected from -H, -COOH, -COOCH₃, -CONHOH, and -CONHCH₃;
further preferably, when L² is selected from: -CH=CH-; R³ is selected from: pyridyl, methylpyridyl, and methoxypyridyl; R² is selected from: pyrrolyl, and phenyl; R¹ is selected from -H, - COOH, -COOCH₃, and -CONHOH; further more preferably, R³ is selected from: pyridyl;
further preferably, when L² is selected from: -CH₂- and -CH₂-CH₂-; R³ is selected from: pyridyl, methylpyridyl and methoxypyridyl; R² is selected from: pyrrolyl; R¹ is selected from -H, -COOH and -COOCH₃; further more preferably, R³ is selected from: pyridyl;
further preferably, when L² is selected from: •-NH-C(=O)-* and •-NH-C(=O)-CH₂-*; R³ is selected from: pyridyl, methylpyridyl and methoxypyridyl; R² is selected from: hydrogen, chlorine, methyl, pyrrolyl, phenyl and chlorophenyl; R¹ is selected from -H, -COOH, -COOCH₃, -CONHOH, -CONHCH₃ and -CONHOCH₃; further more preferably, R³ is selected from: pyridyl;
further preferably, when L² is selected from: •-C(=O)-NH-* and •-CH₂-C(=O)-NH-*; R³ is selected from: pyridyl, methylpyridyl or methoxypyridyl; R² is selected from: hydrogen, indolyl and phenyl; R¹ is selected from -H, -COOH, -COOCH₃, -CONHOH and -CONHCH₃; further more preferably, R³ is selected from: pyridyl;
   or
when L¹ is selected from ethynyl,
R² is selected from: optionally substituted indolyl and optionally substituted phenyl;
L² is selected from: -(CH₂)_{q}-O-(CH₂)ₚ-, -NH-C(=O)-NH-, •-N(CH₃)-C(=O)-NH-*, •-NH-C(=O)-(CH₂)ₚ-* and •-(CH₂)_{q}-C(=O)-NH-*;
preferably, R² is selected from: indolyl, phenyl, chlorophenyl, hydroxyphenyl and hydroxyalkoxyphenyl;
R³ is selected from: pyridyl, methylpyridyl and methoxypyridyl;
more preferably, when L² is selected from: •-CH₂-O-*, •-O-CH₂-*, -O- and -CH₂-O-CH₂-; R³ is selected from: pyridyl; R² is selected from: indolyl; R¹ is selected from -H, -COOH, -COOCH₃ and -CONHOH;
more preferably, when L² is selected from: -NH-C(=O)-NH- and •-N(CH₃)-C(=O)-NH-*; R³ is selected from: pyridyl, fluoropyridyl and methylpyridyl; R² is selected from: indolyl, phenyl, hydroxyphenyl and hydroxyalkoxyphenyl; R¹ is selected from -H, -COOH, -COOCH₃ and -CONHOH; further more preferably, R³ is selected from: pyridyl;
more preferably, when L² is selected from: •-NH-C(=O)-* and •-NH-C(=O)-CH₂-*; R³ is selected from: pyridyl, methylpyridyl and methoxypyridyl; R² is selected from: phenyl; R¹ is selected from: -H, -COOH and -COOCH₃; further more preferably, R³ is selected from: pyridyl;
further preferably, when L² is selected from: •-C(=O)-NH-* and •-CH₂-C(=O)-NH-*; R³ is selected from: pyridyl, methylpyridyl and methoxypyridyl; R² is selected from: indolyl and phenyl; R¹ is selected from -H, -COOH, -COOCH₃ and -CONHOH; further more preferably, R³ is selected from: pyridyl.

In another preferred embodiment, the present invention relates to the compound of above general formula (I), or its enantiomer, diastereomer, tautomer, salt, crystal form, solvate and/or isotope-substituted derivative, and the compound is selected from:
methyl 3 -(3 -(4-((pyridin-3 -yloxy)methyl)phenoxy)azetidin-1 -yl)-2-(1H-pyrrol-1 -yl) benzoate, 3-(3-(4-((pyridin-3-yloxy)methyl)phenoxy)azetidin-1-yl)-2-(1H-pyrrol-1-yl) benzoic acid, ethyl 3-(3-(4-((pyridin-3-yloxy)methyl)phenoxy)azetidin-1-yl)-2-(1H-pyrrol-1-yl) benzoate, N-hydroxy-3-(3-(4-((pyridin-3-yloxy)methyl)phenoxy)azetidin-1-yl)-2-(1H-pyrrole-1-yl) benzamide, N-methyl-3-(3-(4-((pyridin-3-yloxy)methyl)phenoxy)azetidin-1-yl)-2-(1H-pyrrol-1-yl) benzamide, N,N-dimethyl-3-(3-(4-((pyridin-3-yloxy)methyl)phenoxy)azetidin- 1 -yl)-2-(1 H-pyrrol-1 -yl) benzamide, (3 -(3 -(4-((pyridin-3 -yloxy)methyl)phenoxy)azetidin-1 -yl)-2-(1 H-pyrrol-1 -yl)phenyl)(pyrrolid in-1 -yl) methanone, 3 -((4-((1 -(2-(1H-pyrrole-1 -yl)phenyl)azetidin-3 -yl)oxy)benzyl)oxy) pyridine, methyl 3 -(3 -(4-((4-methylpyridin-3 -yloxy)methyl)phenoxy)azetidin-1 -yl)-2-(1 H-pyrrol-1 -yl) benzoate, 3-(3-(4-((4-methylpyridin-3-yloxy)methyl)phenoxy)azetidin-1-yl)-2-(1H-pyrrol-1-yl) benzoic acid, methyl 3-(3-(4-((5-methylpyridin-3-yloxy)methyl)phenoxy)azetidin-1-yl)-2-(1H-pyrrol-1-yl) benzoate, 3-(3-(4-((5-methylpyridin-3-yloxy)methyl)phenoxy)azetidin-1-yl)-2-(1H-pyrrol-1-yl) benzoic acid, methyl 3-(3-(4-(((6-methoxypyridin-3-yl)oxy)methyl)phenoxy)azetidin-1-yl)-2-(1H-pyrrole-1-yl) benzoate, 3-(3-(4-(((6-methoxypyridin-3-yl)oxy)methyl)phenoxy)azetidin-1-yl)-2-(1H-pyrrol-1-yl) benzoic acid, methyl 3-(3-(4-((6-methylpyridin-3-yloxy)methyl)phenoxy)azetidin-1-yl)-2-(1H-pyrrol-1-yl) benzoate, 3-(3-(4-((6-methylpyridin-3-yloxy)methyl)phenoxy)azetidin-1-yl)-2-(1H-pyrrol-1-yl) benzoic acid, methyl 3-(3-(4-(((5-methoxypyridin-3-yl)oxy)methyl)phenoxy)azetidin-1-yl)-2-(1H-pyrrol-1-yl) benzoate, 3-(3-(4-(((5-methoxypyridin-3-yl)oxy)methyl)phenoxy)azetidin-1-yl)-2-(1H-pyrrol-1-yl) benzoic acid, methyl 3-(3-(4-(3-(3-cyanophenyl)-1-methylureido)phenoxy)azetidin-1-yl)-2-(1H-pyrrol-1-yl) benzoate, 3-(3-(4-(3-(3-cyanophenyl)-1-methylureido)phenoxy)azetidin-1-yl)-2-(1H-pyrrole-1-yl) benzoic acid, methyl 3 -(3 -(4-((pyridin-4-yloxy)methyl)phenoxy)azetidin-1 -yl)-2-(1H-pyrrole-1 -yl) benzoate, 3-(3-(4-((pyridin-4-yloxy)methyl)phenoxy)azetidin-1-yl)-2-(1H-pyrrole-1-yl) benzoic acid, methyl 2-(1H-pyrrol-1-yl)-3-(3-(4-((quinolin-3-yloxy)methyl)phenoxy)azetidin-1-yl) benzoate, 2-(1H-pyrrol-1 -yl)-3 -(3 -(4-((quinolin-3 -yloxy)methyl)phenoxy)azetidin-1 -yl) benzoic acid, methyl 2-(1H-pyrrol-1-yl)-3-(3-(4-(3-(3-(trifluoromethyl)phenyl)ureido)phenoxy)azetidin-1-yl) benzoate, 2-(1H-pyrrol-1-yl)-3-(3-(4-(3-(3-(trifluoromethyl)phenyl)ureido)phenoxy)azetidin-1-yl) benzoic acid, N-hydroxy-2-(1 H-pyrrol-1-yl)-3-(3-(4-(3-(3-(trifluoromethyl)phenyl)ureido)phenoxy)azetidin -1-yl) benzamide, methyl 3 -(3 -(4-((pyrimidin-2-yloxy)methyl)phenoxy)azetidin-1 -yl)-2-(1H-pyrrol-1 -yl) benzoate, 3-(3-(4-((pyrimidin-2-yloxy)methyl)phenoxy)azetidin-1-yl)-2-(1H-pyrrol-1-yl) benzoic acid, methyl 3-(3-(4-((pyridin-2-yloxy)methyl)phenoxy)azetidin-1-yl)-2-(1H-pyrrol-1-yl) benzoate, 3-(3-(4-((pyridin-2-yloxy)methyl)phenoxy)azetidin-1-yl)-2-(1H-pyrrol-1-yl) benzoic acid, methyl 3-(3-(4-((pyrazin-2-yloxy)methyl)phenoxy)azetidin-1-yl)-2-(1H-pyrrol-1-yl) benzoate, 3-(3-(4-((pyrazin-2-yloxy)methyl)phenoxy)azetidin-1-yl)-2-(1H-pyrrol-1-yl) benzoic acid, methyl 3 -(3 -(4-((pyrimidin-5 -yloxy)methyl)phenoxy)azetidin-1 -yl)-2-(1H-pyrrol-1 -yl) benzoate, 3-(3-(4-((pyrimidin-5-yloxy)methyl)phenoxy)azetidin-1-yl)-2-(1H-pyrrol-1-yl) benzoic acid, N-hydroxy-3-(3-(4-((pyrimidin-5-oxy)methyl)phenoxy)azetidin-1-yl)-2-(1H-pyrrol-1-yl) benzamide, methyl 3-(3-(3-methyl-4-((pyridin-3-yloxy)methyl)phenoxy)azetidin-1-yl)-2-(1H-pyrrol-1-yl) benzoate, 3-(3-(3-methyl-4-((pyridin-3-yloxy)methyl)phenoxy)azetidin-1 -yl)-2-(1H-pyrrol-1 -yl) benzoic acid, methyl 3-(3-(2-methyl-4-((pyridin-3-yloxy)methyl)phenoxy)azetidin-1 -yl)-2-(1H-pyrrol-1 -yl) benzoate, 3-(3-(2-methyl-4-((pyridin-3-yloxy)methyl)phenoxy)azetidin-1 -yl)-2-(1H-pyrrol-1 -yl) benzoic acid, methyl 6-(3-(4-((pyridin-3-yloxy)methyl)phenoxy)azetidin-1-yl)-[1,1'-biphenyl]-2-carboxylate, 6-(3-(4-((pyridin-3-yloxy)methyl)phenoxy)azetidin-1 -yl)-[1,1 '-biphenyl]-2-carboxylic acid, N-hydroxy-6-(3-(4-((pyridin-3-yloxy)methyl) phenoxy)azetidin-1 -yl)-[1,1 '-biphenyl]-2-carboxamide, methyl 3'-chloro-6-(3-(4-((pyridin-3-yloxy)methyl)phenoxy)azetidin-1-yl)-[1,1'-biphenyl]-2-carboxyl ate, methyl 3-(3-(3-chloro-4-((pyridin-3-yloxy)methyl)phenoxy)azetidin-1 -yl)-2-(1 H-pyrrol-1 -yl) benzoate, 3-(3-(3-chloro-4-((pyridin-3-yloxy)methyl)phenoxy)azetidin-1-yl)-2-(1H-pyrrol-1-yl) benzoic acid, methyl 3-(3-(3-fluoro-4-((pyridin-3-yloxy)methyl)phenoxy)azetidin-1-yl)-2-(1H-pyrrol-1-yl) benzoate, 3-(3-(3-fluoro-4-((pyridin-3-yloxy)methyl)phenoxy)azetidin-1-yl)-2-(1H-pyrrole-1-yl) benzoic acid, methyl 3-(3-(3-((pyridin-3-yloxy)methyl)phenoxy)azetidin-1-yl)-2-(1H-pyrrole-1-yl) benzoate, methyl 6-((3-hydroxy-4-(3-(pyridin-3-yl)ureido)phenyl)ethynyl)-[1,1'-biphenyl]-2-carboxylate, methyl 3 -(3 -(4-((pyridin-3 -ylamino)methyl)phenoxy)azetidin-1 -yl)-2-(1H-pyrrole-1 -yl) benzoate, 3-(3-(4-((pyridin-3-ylamino)methyl)phenoxy)azetidin-1-yl)-2-(1H-pyrrole-1-yl) benzoic acid, N-hydroxy-3-(3-(4-((pyridin-3-ylamino)methyl)phenoxy)azetidin-1-yl)-2-(1H-pyrrole-1-yl) benzamide, methyl 3-(3-(4-(pyridin-3-ylmethoxy)phenoxy)azetidin-1-yl)-2-(1H-pyrrole-1-yl) benzoate, 3-(3-(4-(pyridin-3-ylmethoxy)phenoxy)azetidin-1-yl)-2-(1H-pyrrole-1-yl) benzoic acid, N-hydroxy-3-(3-(4-(pyridin-3-ylmethoxy)phenoxy)azetidin-1-yl)-2-(1H-pyrrole-1-yl) benzamide, methyl 3-(3-(4-(pyridin-3-yloxy)phenoxy)azetidin-1-yl)-2-(1H-pyrrol-1-yl) benzoate, 3-(3-(4-(pyridin-3-yloxy)phenoxy)azetidin-1-yl)-2-(1H-pyrrol-1-yl) benzoic acid, methyl 3-(3-(4-(pyridin-4-yloxy)phenoxy)azetidin-1-yl)-2-(1H-pyrrol-1-yl) benzoate, 3-(3-(4-(pyridin-4-yloxy)phenoxy)azetidin-1-yl)-2-(1H-pyrrol-1-yl) benzoic acid, methyl 3-(3-(4-((pyridin-3-ylmethoxy)methyl)phenoxy)azetidin-1-yl)-2-(1H-pyrrol-1-yl) benzoate, 3-(3-(4-((pyridin-3-ylmethoxy)methyl)phenoxy)azetidin-1-yl)-2-(1H-pyrrol-1-yl) benzoic acid, N-hydroxy-3-(3-(4-((pyridin-3-ylmethoxy)methyl)phenoxy)azetidin-1-yl)-2-(1H-pyrrol-1-yl) benzamide, methyl (E)-3 -(3 -(4-(2-(pyridin-3 -yl)vinyl)phenoxy)azetidin-1 -yl)-2-(1H-pyrrole-1 -yl) benzoate, (E)-3-(3-(4-(2-(pyridin-3-yl)vinyl)phenoxy)azetidin-1-yl)-2-(1H-pyrrol-1-yl) benzoic acid, (E)-N-hydroxy-3-(3-(4-(2-(pyridin-3-yl)vinyl)phenoxy)azetidin-1-yl)-2-(1H-pyrrol-1-yl) benzamide, methyl 3-(3-(4-(2-(pyridin-3-yl)ethyl)phenoxy)azetidin-1-yl)-2-(1H-pyrrol-1-yl) benzoate, 3-(3-(4-(2-(pyridin-3-yl)ethyl)phenoxy)azetidin-1-yl)-2-(1H-pyrrol-1-yl) benzoic acid, methyl 2-(1H-pyrrol-1 -yl)-3-(3-(4-((thiophen-2-yloxy)methyl)phenoxy)azetidin-1 -yl) benzoate, 2-(1H-pyrrol-1 -yl)-3-(3-(4-((thiophen-2-yloxy)methyl)phenoxy)azetidin-1 -yl) benzoic acid, methyl 3-(3-(4-(nicotinamido)phenoxy)azetidin-1-yl)-2-(1H-pyrrol-1-yl) benzoate, 3-(3-(4-(nicotinamido)phenoxy)azetidin-1-yl)-2-(1H-pyrrol-1-yl) benzoic acid, N-(4-((1-(3-(hydroxycarbamoyl)-2-(1H-pyrrol-1-yl)phenyl)azetidin-3-yl)oxy)phenyl) nicotinamide, methyl 3-(3-(4-(((1-methyl-1H-pyrazol-3-yl)oxy)methyl)phenoxy)azetidin-1-yl)-2-(1H-pyrrol-1-yl) benzoate, 3-(3-(4-(((1-methyl-1H-pyrazol-3-yl)oxy)methyl)phenoxy)azetidin-1-yl)-2-(1H-pyrrol-1-yl) benzoic acid, methyl 3-(3-(4-((isothiazol-3-yloxy)methyl)phenoxy)azetidin-1-yl)-2-(1H-pyrrol-1-yl) benzoate, 3-(3-(4-((isothiazol-3-yloxy)methyl)phenoxy)azetidin-1-yl)-2-(1H-pyrrol-1-yl) benzoic acid, methyl 3-(3-(4-(3-(pyridin-3-yl)ureido)phenoxy)azetidin-1-yl)-2-(1H-pyrrol-1-yl) benzoate, 3-(3-(4-(3-(pyridin-3-yl)ureido)phenoxy)azetidin-1-yl)-2-(1H-pyrrol-1-yl) benzoic acid, N-hydroxy-3-(3-(4-(3-(pyridin-3-yl)ureido)phenoxy)azetidin-1-yl)-2-(1H-pyrrol-1-yl) benzamide, methyl 3-(3-(4-(2-oxo-2-(phenylamino)ethyl)phenoxy)azetidin-1-yl)-2-(1H-pyrrol-1-yl) benzoate, 3-(3-(4-(2-oxo-2-(phenylamino)ethyl)phenoxy)azetidin-1-yl)-2-(1H-pyrrol-1-yl) benzoic acid, methyl (E)-6-(3-(4-(2-(pyridin-3-yl)vinyl)phenoxy)azetidin-1-yl)-[1,1'-biphenyl]-2-carboxylate, (E)-6-(3-(4-(2-(pyridin-3-yl)vinyl)phenoxy)azetidin-1-yl)-[1,1'-biphenyl]-2-carboxylic acid, (E)-N-hydroxy-6-(3-(4-(2-(pyridin-3-yl)vinyl)phenoxy)azetidin-1-yl)-[1,1'-biphenyl]-2-carbo xamide, methyl 3'-chloro-6-(3-(4-(3-(3-(trifluoromethyl)phenyl)ureido)phenoxy)azetidin-1 -yl)-[1,1 '-biphenyl] -2-carboxylate, 3'-chloro-6-(3-(4-(3-(3-(trifluoromethyl)phenyl)ureido)phenoxy)azetidin-1 -yl)-[1,1 '-biphenyl] -2-carboxylic acid, methyl 3'-chloro-6-(3-(4-((pyridin-3-ylamino)methyl)phenoxy)azetidin-1 -yl)-[1,1 '-biphenyl]-2-carbo xylate, 3'-chloro-6-(3-(4-((pyridin-3-ylamino)methyl)phenoxy)azetidin-1 -yl)-[1,1 '-biphenyl]-2-carbo xylic acid, 3'-chloro-N-hydroxy-6-(3-(4-((pyridin-3-ylamino)methyl)phenoxy)azetidin-1 -yl)-[1,1 '-biphen yl]-2-carboxamide, methyl 6-(3-(4-((pyridin-3-ylamino)methyl)phenoxy)azetidin-1-yl)-[1,1'-biphenyl]-2-carboxylate, 6-(3-(4-((pyridin-3-ylamino)methyl)phenoxy)azetidin-1-yl)-[1,1'-biphenyl]-2-carboxylic acid, N-hydroxy-6-(3-(4-((pyridin-3-ylamino)methyl)phenoxy)azetidin-1-yl)-[1,1'-biphenyl]-2-carb oxamide, methyl 6-(3-(4-(2-oxo-2-(pyridin-3-ylamino)ethyl)phenoxy)azetidin-1-yl)-[1,1'-biphenyl]-2-carboxyl ate, 6-(3-(4-(2-oxo-2-(pyridin-3-ylamino)ethyl)phenoxy)azetidin-1-yl)-[1,1'-biphenyl]-2-carboxyli c acid, N-hydroxy-6-(3-(4-(2-oxo-2-(pyridine-3-ylamino)ethyl)phenoxy)azetidin-1-yl)-[1,1'-biphenyl ]-2-carboxamide, N-methyl-6-(3-(4-(2-oxo-2-(pyridin-3-ylamino)ethyl)phenoxy)azetidin-1-yl)-[1,1'-biphenyl]-2-carboxamide, methyl 3'-chloro-6-(3-(4-(3-(pyridin-3-yl)ureido)phenoxy)azetidin-1-yl)-[1,1'-biphenyl]-2-carboxylat e, 3'-chloro-6-(3-(4-(3-(pyridin-3-yl)ureido)phenoxy)azetidin-1-yl)-[1,1'-biphenyl]-2-carboxylic acid, 3'-chloro-N-hydroxy-6-(3-(4-(3-(pyridin-3-yl)ureido)phenoxy)azetidin-1-yl)-[1,1'-biphenyl]-2 -carboxamide, methyl 6-(3-(4-(3-(pyridin-3-yl)ureido)phenoxy)azetidin-1 -yl)-[1,1 '-biphenyl]-2-carboxylate, 6-(3-(4-(3-(pyridin-3-yl)ureido)phenoxy)azetidin-1-yl)-[1,1'-biphenyl]-2-carboxylic acid, N-hydroxy-6-(3-(4-(3-(pyridin-3-yl)ureido)phenoxy)azetidin-1 -yl)-[1,1 '-biphenyl]-2-carboxa mide, N-methyl-6-(3-(4-(3-(pyridine-3-yl)ureido)phenoxy)azetidin-1-yl)-[1,1'-biphenyl]-2-carboxa mide, methyl 3-(3-(4-(2-oxo-2-(pyridin-3-ylamino)ethyl)phenoxy)azetidin-1-yl) benzoate, 3-(3-(4-(2-oxo-2-(pyridin-3-ylamino)ethyl)phenoxy)azetidin-1-yl) benzoic acid, N-hydroxy-3-(3-(4-(2-oxo-2-(pyridin-3-ylamino)ethyl)phenoxy)azetidin-1-yl) benzamide, 2-(4-((1-phenylazetidin-3-yl)oxy)phenyl)-N-(pyridin-3-yl) acetamide, methyl 2-(1H-indol-6-yl)-3 -(3 -(4-(2-oxo-2-(pyridin-3 -ylamino)ethyl)phenoxy)azetidin-1 -yl) benzoate, 2-(1H-indol-6-yl)-3-(3-(4-(2-oxo-2-(pyridine-3-ylamino)ethyl)phenoxy)azetidin-1-yl) benzoic acid, methyl 6-(3-(4-(2-(pyridin-3-yl)acetamido)phenoxy)azetidin-1-yl)-[1,1'-biphenyl]-2-carboxylate, 6-(3-(4-(2-(pyridin-3-yl)acetamido)phenoxy)azetidin-1-yl)-[1,1'-biphenyl]-2-carboxylic acid, N-hydroxy-6-(3-(4-(2-(pyridin-3-yl)acetamido)phenoxy)azetidin-1-yl)-[1,1'-biphenyl]-2-carb oxamide, N-methyl-6-(3-(4-(2-(pyridin-3-yl)acetamido)phenoxy)azetidin-1-yl)-[1,1'-biphenyl]-2-carbo xamide, N-methoxy-6-(3-(4-(2-(pyridin-3-yl)acetamido)phenoxy)azetidin-1-yl)-[1,1 '-biphenyl]-2-carb oxamide, N-(4-((1-([1,1'-biphenyl]-2-yl)azetidin-3-yl)oxy)phenyl)-2-(pyridin-3-yl)acetamide, methyl 3 -(3 -(4-(3 -(5 -fluoropyridin-3 -yl)ureido)phenoxy)azetidin-1 -yl)-2-(1H-pyrrol-1 -yl) benzoate, 3-(3-(4-(3-(5-fluoropyridin-3-yl)ureido)phenoxy)azetidin-1-yl)-2-(1H-pyrrol-1-yl) benzoic acid, methyl 3-(3-(4-(3-(5-methylpyridin-3-yl)ureido)phenoxy)azetidin-1 -yl)-2-(1H-pyrrol-1 -yl) benzoate, 3-(3-(4-(3-(5-methylpyridin-3-yl)ureido)phenoxy)azetidin-1-yl)-2-(1H-pyrrol-1-yl) benzoic acid, methyl 3'-chloro-6-(3-(4-(2-(pyridin-3-yl)acetamido)phenoxy)azetidin-1-yl)-[1,1'-biphenyl]-2-carbox ylate, 3'-chloro-6-(3-(4-(2-(pyridin-3-yl)acetamido)phenoxy)azetidin-1-yl)-[1,1'-biphenyl]-2-carbox ylic acid, methyl 3-(3-(4-(2-(pyridin-3-yl)acetamido)phenoxy)azetidin-1-yl) benzoate, 3-(3-(4-(2-(pyridin-3-yl)acetamido)phenoxy)azetidin-1-yl) benzoic acid, methyl 3-(3-(4-(3-(pyridin-3-yl)ureido)phenoxy)azetidin-1-yl) benzoate, 3-(3-(4-(3-(pyridin-3-yl)ureido)phenoxy)azetidin-1-yl) benzoic acid, methyl 3-(3-(4-(2-(pyridin-3-yl)acetamido)phenoxy)azetidin-1-yl)-2-(1H-pyrrol-1-yl) benzoate, 3-(3-(4-(2-(pyridin-3-yl)acetamido)phenoxy)azetidin-1-yl)-2-(1H-pyrrol-1-yl) benzoic acid, methyl 3 -(3 -(3 -fluoro-4-(2-(pyridin-3 -yl)acetamido)phenoxy)azetidin-1 -yl)-2-(1H-pyrrol-1 -yl) benzoate, 3-(3-(3-fluoro-4-(2-(pyridin-3-yl)acetamido)phenoxy)azetidin-1-yl)-2-(1H-pyrrol-1-yl) benzoic acid, methyl 2-methyl-3-(3-(4-(2-(pyridin-3-yl)acetamido)phenoxy)azetidin-1-yl) benzoate, 2-methyl-3-(3-(4-(2-(pyridin-3-yl)acetamido)phenoxy)azetidin-1-yl) benzoic acid, methyl 6-(3-(3-fluoro-4-(2-(pyridin-3-yl)acetamido)phenoxy)azetidin-1 -yl)-[1,1'-biphenyl]-2-carboxy late, 6-(3-(3-fluoro-4-(2-(pyridin-3-yl)acetamido)phenoxy)azetidin-1 -yl)-[1,1'-biphenyl]-2-carboxy lic acid, methyl 2-chloro-3-(3-(4-(2-(pyridin-3-yl)acetamido)phenoxy)azetidin-1-yl) benzoate, 2-chloro-3-(3-(4-(2-(pyridin-3-yl)acetamido)phenoxy)azetidin-1-yl) benzoic acid, methyl 3-(3-(4-((1H-imidazol-1-yl)methyl)phenoxy)azetidin-1-yl)-2-(1H-pyrrol-1-yl) benzoate, 3-(3-(4-((1H-imidazol-1-yl)methyl)phenoxy)azetidin-1-yl)-2-(1H-pyrrol-1-yl) benzoic acid, methyl 3'-(2-hydroxyethoxy)-6-((4-(3-(pyridin-3-yl)ureido)phenyl)ethynyl)-[1,1'-biphenyl]-2-carbox ylate, methyl 6-((4-(3-(pyridin-3-yl)ureido)phenyl)ethynyl)-[1,1'-biphenyl]-2-carboxylate, 6-((4-(3-(pyridin-3-yl)ureido)phenyl)ethynyl)-[1,1'-biphenyl]-2-carboxylic acid, N-hydroxy-6-((4-(3-(pyridin-3-yl)ureido)phenyl)ethynyl)-[1,1'-biphenyl]-2-carboxamide, methyl 2-(1H-indol-6-yl)-3-((4-(3-(pyridin-3-yl)ureido)phenyl)ethynyl) benzoate, 2-(1H-indol-6-yl)-3-((4-(3-(pyridin-3-yl)ureido)phenyl)ethynyl) benzoic acid, methyl 6-((4-(2-oxo-2-(pyridin-3-ylamino)ethyl)phenyl)ethynyl)-[1,1'-biphenyl]-2-carboxylate, 6-((4-(2-oxo-2-(pyridin-3-ylamino)ethyl)phenyl)ethynyl)-[1,1'-biphenyl]-2-carboxylic acid, N-hydroxy-6-((4-(2-oxo-2-(pyridin-3-ylamino)ethyl)phenyl)ethynyl)-[1,1'-biphenyl]-2-carbox amide, methyl 2-(1H-indol-6-yl)-3-((4-(2-oxo-2-(pyridin-3-ylamino)ethyl)phenyl)ethynyl) benzoate, 2-(1H-indol-6-yl)-3-((4-(2-oxo-2-(pyridin-3-ylamino)ethyl)phenyl)ethynyl) benzoic acid, methyl 6-((4-(2-(pyridin-3-yl)acetamido)phenyl)ethynyl)-[1,1'-biphenyl]-2-carboxylate, 6-((4-(2-(pyridin-3-yl)acetamido)phenyl)ethynyl)-[1,1'-biphenyl]-2-carboxylic acid, and methyl 3'-hydroxyl-6-((4-(3-(pyridin-3-yl)ureido)phenyl)ethynyl)-[1,1'-biphenyl]-2-carboxylate.

In another embodiment, the present invention further relates to a pharmaceutical composition, including one or more of the compound of the above general formula (I) according to the present invention, or its enantiomer, diastereomer, tautomer, salt, crystal form, solvate and/or isotope-substituted derivative.

In another embodiment, the present invention further relates to a pharmaceutical composition, including one or more of the compound of the above general formula (I) according to the present invention, or its enantiomer, diastereomer, tautomer, salt, crystal form, solvate and/or isotope-substituted derivative, and a pharmaceutically acceptable carrier, excipient or diluent.

In another embodiment, the present invention further relates to a method for treating and/or preventing diseases or disorders caused by EBNA1 activity, and the method includes administering to a subject an effective amount of the compound of the above general formula (I), or its enantiomer, diastereomer, tautomer, salt, crystal form, solvate and/or isotope-substituted derivative. The compound of the general formula (I) of the present invention shows a better inhibitory effect on EBNA1 at the cellular level; especially, when R³ is selected from pyridyl and phenyl, it shows a more excellent inhibitory activity.

In another embodiment, the present invention further relates to a use of one or more of the compound of the above general formula (I) according to the present invention, or its enantiomer, diastereomer, tautomer, salt, crystal form, solvate and/or isotope-substituted derivative for preparation of a medicament for treating and/or preventing the diseases caused by EBNA1 activity, including administering to a subject an effective amount of at least one compound according to the present invention, or its enantiomer, diastereomer, tautomer, salt, crystal form, solvate and/or isotope-substituted derivative.

In another embodiment, the present invention further relates to a method for treating and/or preventing nasopharyngeal carcinoma, including administering to a subject an effective amount of at least one pharmaceutical composition according to the present invention.

In another embodiment, the present invention further relates to a use of one or more of the above-mentioned pharmaceutical compositions according to the present invention for preparation of a medicament for treating and/or preventing nasopharyngeal carcinoma, including administering to a subject an effective amount of at least one pharmaceutical composition according to the present invention.

In a preferred embodiment, the disease or disorder is: cancer, infectious mononucleosis, chronic fatigue syndrome, multiple sclerosis, systemic lupus erythematosus or rheumatoid arthritis.

In a more preferred embodiment, the cancer is nasopharyngeal carcinoma, non-Hodgkin's lymphoma, anaplastic large cell lymphoma, angioimmunoblastic T-cell lymphoma, hepatosplenic T-cell lymphoma, B-cell lymphoma, Burkitt's lymphoma, reticuloendothelial proliferation, reticulocytosis, diffuse large B-cell lymphoma, extranodal T/NK lymphoma/angiocentric lymphoma, follicular lymphoma, immunoblastic lymphoma, mucosa-associated lymphoma tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma, primary effusion lymphoma, lymphomatoid granuloma, angioimmunoblastic lymphadenopathy, X-linked lymphoproliferative disease, post-transplant lymphoproliferative disease, or Hodgkin lymphoma.

The embodiments and preparation examples provided in the present invention further illustrate the compounds of the present invention and their preparation methods. It should be understood that the scope of the following preparation examples and embodiments does not limit the scope of the present invention in any way.

The following synthetic route describes a preparation method of the derivatives of general formula (I) of the present invention, and the raw materials, reagents, catalysts, solvents, etc. used in the following synthetic route can be prepared by methods well known to the person skilled in the field of organic chemistry or can be obtained by commercially purchase. All final derivatives of the present invention can be prepared by the methods described in the routes or similar methods, which are all well known to the person skilled in the field of organic chemistry. All variables factors applied in these routes are defined below or as defined in the claims.

### Preparation Method

Unless otherwise specified, all reagents are purchased from reagent companies and used directly without purification treatment. Most of the reactions are performed in anhydrous condition under nitrogen protection. Reagents and solvents are purified according to Purification of Laboratory Chemicals (W.L.F. Armarego, Christina Li Lin Chai, Elsevier Inc. 2009). Unless otherwise specified, solvents are re-distilled before use. Tetrahydrofuran (THF) is treated with sodium-benzophenone system; dichloromethane and N,N'-dimethylformamide (DMF) are treated with calcium hydride; methanol is treated with magnesium chips and re-distilled for later use. Triethylamine, diisopropylethylamine (DIPEA) and pyridine are all treated with calcium hydride and re-distilled. The reaction is monitored by thin-layer chromatography (TLC). The thin-layer silica gel plate used is GF254 (60-F250, 0.2 mm); and is developed with UV (wavelength 254 nm) or iodine, or soaked in phosphomolybdic acid or ninhydrin solution, and then heated for color development. Rapid column chromatography is filled with silica gel 60 (230-400 mesh ASTM), generally using ethyl acetate and n-hexane or dichloromethane and methanol system as an eluent. ¹H NMR is determined by DRX-300 or Brüker Avance-400 or Brüker Avance-500 Nuclear Magnetic Resonance instrument, and the chemical shift is determined by residual peaks of deuterated solvent; high-resolution mass spectrometry is performed by ABI Q-star Elite mass spectrometer or Thermo Q Exactive Focus mass spectrometer; and liquid mass spectrometer is Agilent 6125.

### General experimental operation 1:

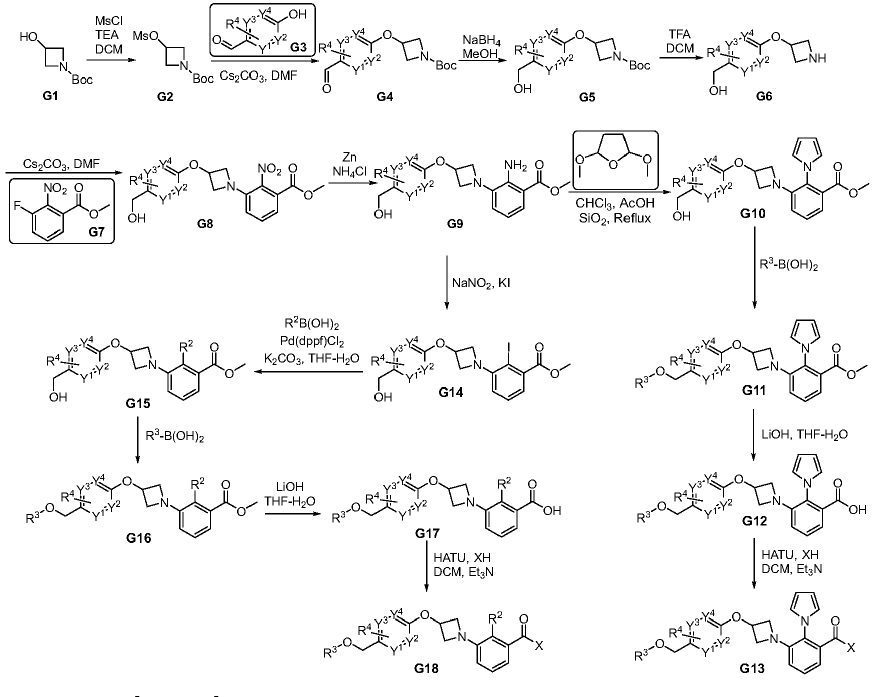

R² and R³ are aromatic rings or aromatic heterocycles, as defined specifically in the description and claims; R⁴ is as defined in the description and claims; X=O-R^{1a}, NHR^{1b} or NR^{1b}R^{1c}, as defined specifically in the description and claims; Y¹, Y², Y³, and Y⁴ are as defined in the specification and claims.

N-Boc-3-hydroxy azetidine G1 (1.0 equivalent) was dissolved in dichloromethane, and under nitrogen protection, triethylamine (1.5 equivalents) was added under cooling in an ice bath; then methyl sulfonyl chloride (1.5 equivalents) was added slowly, and the reaction was brought to room temperature for 2h. The solvent was removed by rotary evaporation under reduced pressure, and the residue was diluted with a large amount of ethyl acetate and then washed once with water. The resulting organic phase was dried with anhydrous sodium sulfate, filtered, and concentrated to obtain a light yellow oily intermediate compound G2. Without further purification, G2 was dissolved in N,N-dimethylformamide and then heated to 100°C for 14 hours under nitrogen protection after addition of G3 (1.2 equivalents) and cesium carbonate (2.2 equivalents). After cooling to room temperature, the reaction solution was diluted with a large amount of ethyl acetate, washed twice with saturated aqueous solution of ammonium chloride, and washed once with saturated aqueous solution of sodium bicarbonate. The organic phase was dried with anhydrous sodium sulfate, filtered, concentrated, and then separated by silica gel column chromatography to obtain a compound G4.

Compound G4 (1 equivalent) was dissolved in a solvent, and sodium borohydride (3.0 equivalents) was added under nitrogen protection and ice bath cooling, and then a small amount of methanol was added. The reaction was carried out at room temperature for 2 hours. The reaction was quenched with saturated aqueous solution of ammonium chloride, and the organic phase was removed under reduced pressure and extracted with a large amount of ethyl acetate. The organic phase was washed with saturated aqueous solution of sodium chloride, dried with anhydrous sodium sulfate, filtered and concentrated to obtain a compound G5. The compound G5 was dissolved in an appropriate amount of dichloromethane and treated with excess trifluoroacetic acid to remove the Boc protection and obtain the trifluoroacetate of compound G6.

The intermediate G6 (1.0 equivalent), methyl 3-fluoro-2-nitrobenzoate G7 (1.0 equivalent), and cesium carbonate (2.5 equivalents) were dissolved in N,N-dimethylformamide, and heated under nitrogen protection to 50°C for 2 hours. After the reaction solution was cooled to room temperature, diluted with a large amount of ethyl acetate, and washed with saturated aqueous solution of ammonium chloride. The resulting organic phase was dried with anhydrous sodium sulfate, filtered, concentrated and then separated by silica gel column chromatography to obtain a compound G8.

Nitro reduction operation: dissolving the nitro-substituted compound G8 (1.0 equivalent) that needs to be reduced in a mixed solution of methanol and dichloromethane at a mixing ratio of 1:1, adding ammonium chloride (5.0 equivalents) under ice bath cooling, and adding reduction zinc powder (3.0 equivalents), restoring to room temperature and stirring for more than 2 hours, and monitoring by the thin-layer chromatography until the reaction is over. The reaction solution was filtered to remove insoluble solid substance; the filter cake was washed with ethyl acetate; and the filtrate was diluted with a large amount of ethyl acetate, and washed with saturated aqueous solution of sodium bicarbonate. The aqueous phase was sequentially extracted with ethyl acetate twice, and the obtained organic phases were combined. The mixture was dried over anhydrous sodium sulfate, filtered, concentrated, and separated by the silica gel column chromatography to obtain a compound G9.

The compound G9 (1.0 equivalent) was dissolved in anhydrous chloroform, and appropriate amount of dry silica gel, compound 2,5-dimethoxytetrahydrofuran (5.0 equivalents) and anhydrous acetic acid (50.0 equivalents) were added respectively, and then the reaction was subjected to refluxing under nitrogen protection for more than 2 hours, and monitored by thin-layer chromatography until the end of the reaction. The reaction solution was cooled to room temperature, and filtered to remove insoluble substance. The filtrate was washed with saturated aqueous solution of sodium bicarbonate. The aqueous phase was sequentially extracted with ethyl acetate twice, and the obtained organic phases were combined. The mixture was dried over anhydrous sodium sulfate, filtered, concentrated, and separated by silica gel column chromatography to obtain a compound G10.

The compound G10 (1.0 equivalent), arylboronic acid compound (R³B(OH)₂) (3.0 equivalents), anhydrous copper acetate (0.3 equivalent) and an appropriate amount of 40-60 mesh 4Å molecular sieve were mixed and dissolved in dichloromethane, and triethylamine (5.0 equivalents) was added. After replacement with oxygen environment, the mixture was stirring at room temperature for 12 hours, and filtered to remove insoluble substance. The filtrate was washed with saturated aqueous solution of ammonium chloride. The aqueous phase was sequentially extracted with ethyl acetate twice, and the obtained organic phases were combined. The mixture was dried over anhydrous sodium sulfate, filtered, concentrated, and separated by silica gel column chromatography to obtain a compound G11.

Hydrolysis operation: dissolving the compound G11 (1.0 equivalent) in an appropriate amount of tetrahydrofuran, adding aqueous solution of lithium hydroxide (1.0 mole per liter, 3.0 equivalents), replacing with nitrogen gas, stirring at room temperature for more than 4 hours, and monitoring by thin-layer chromatography until the end of the reaction. The organic phase was removed under reduced pressure, and diluted hydrochloric acid (1.0 mol per liter) was added dropwise to the residual aqueous phase to adjust pH to 4, a large amount of white solid was precipitated in the system, followed by filtration to obtain the solid. The filter cake was washed with water and dried to obtain carboxylic acid compound G12.

The compound G12 (1.0 equivalent) was dissolved in an appropriate amount of anhydrous dichloromethane, and then triethylamine (3.0 equivalents), nucleophilic reagent XH (1.2 equivalents) and HATU (1.5 equivalents) were added separately under ice bath cooling, followed by restoring to room temperature under nitrogen protection and stirring for more than 4 hours, and monitoring by thin-layer chromatography until the end of the reaction. The reaction solution was diluted with a large amount of ethyl acetate, and washed with saturated aqueous solution of ammonium chloride. The aqueous phase was sequentially extracted with ethyl acetate twice, and the obtained organic phases were combined. The mixture was dried over anhydrous sodium sulfate, filtered, concentrated, and separated by silica gel column chromatography to obtain a compound G13.

The compound G9 (1.0 equivalent) was dissolved in 1,4-dioxane-water, hydroiodic acid (above 20.0 equivalents) was added under ice bath cooling, followed by stirring for 10min and slowly adding aqueous solution of sodium nitrite (1.1 equivalents). An aqueous solution of potassium iodide (1.5 equivalents) was added under ice bath cooling after reaction for 1 hour, and the reaction temperature was slowly raised to room temperature for 16 hours of reaction. Saturated solution of sodium bicarbonate was added to adjust the pH to 7. Ethyl acetate was used for extraciton, and the obtained extaract was dried with anhydrous sodium sulfate, concentrated and separated with silica gel column chromatography to obtain a compound G14.

The compound G14 and arylboronic acid compound (R³B(OH)₂) (1.5 equivalents) were dissolved in tetrahydrofuran-water, followed by adding catalyst Pd(dppf)Cl₂ (0.1 equivalent) and K₂CO₃ (3.0 equivalents) and heating to 90°C for reacting for 16 hours. After the reaction was completed, the organic phase was removed under reduced pressure, the residue was diluted in a large amount of ethyl acetate, and washed with saturated brine twice. The obtained organic phase was dried with anhydrous sodium sulfate, filtered, concentrated, and separated by silica gel column chromatography to obtain a compound G15.

Referring to the experimental operations for preparing the compound G11 from the compound G10, a compound G16 is prepared from the compound G15.

Referring to the hydrolysis experimental operations for preparing the compound G12 from the compound G11, a compound G17 is prepared from the compound G16.

Referring to the experimental operations for preparing the compound G13 from the compound G12, a compound G18 is prepared from the compound G17.

### General experimental operation 2:

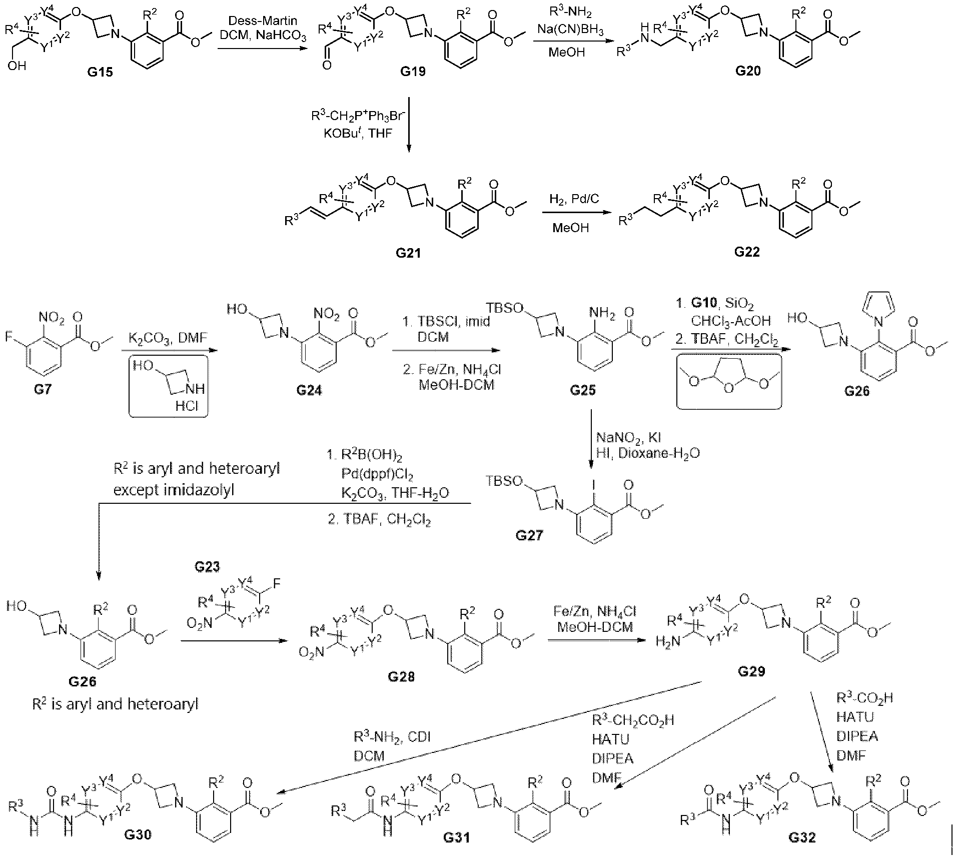

The compound G15 (1.0 equivalent) was dissolved in anhydrous dichloromethane, and then anhydrous sodium bicarbonate (5.0 equivalents) was added. Dess-Martin oxidant was added in batches under ice bath cooling, followed by gradually restoring to room temperature and stirring for more than 2 hours, and monitoring by thin-layer chromatography until the end of the reaction. The reaction solution was diluted by addition of a large amount of ethyl acetate, and then washed with saturated aqueous solution of sodium. The obtained organic phase was dried with anhydrous sodium sulfate, filtered, concentrated, and then separated by silica gel column chromatography to obtain a compound G19.

The compound G19 was dissolved in anhydrous methanol, and arylamine (R³-NH₂) (2.0 equivalents) was added, stirred overnight at room temperature, and cooled in an ice bath again. Sodium cyanoborohydride (2.0 equivalents) was added, followed by continuing stirring at room temperature for more than 2 hours, and monitoring thin-layer chromatography until the end of reaction. The reaction was quenched by adding an appropriate amount of saturated aqueous solution of ammonium chloride under an ice bath, the reaction solution was diluted and extracted with a large amount of ethyl acetate. The obtained organic phase was dried with anhydrous sodium sulfate, filtered and concentrated, and then separated by silica gel column chromatography to obtain a compound G20.

The compound G19 (1.0 equivalent) was dissolved in anhydrous tetrahydrofuran, followed by adding potassium tert-butoxide (1.5 equivalents), lowering the temperature to -78°C. THF solution of aryl Wittig reagent (1.2 equivalents) was then added dropwise, and the temperature was kept at -78 °C while continuing stirring for 2 hours. The reaction bottle was transferred to an ice bath, gradually restored to 0°C and stirred for 2 hours. Saturated aqueous solution of ammonium chloride was added to quench the reaction, and the reaction solution was diluted and extracted with a large amount of ethyl acetate. The obtained organic phase was dried with anhydrous sodium sulfate, filtered and concentrated, and then separated by silica gel column chromatography to obtain a compound G21.

The compound G21 was dissolved in methanol, and then an appropriate amount of palladium carbon (0.2 equivalent) was added. After full replacement of hydrogen atmosphere, a stirring operation was performed at room temperature for more than 2 hours. Thin-layer chromatography was used for monitoring until the end of the reaction, and the insoluble substance was removed by filtration. The filtrate was concentrated and then further purified by silica gel column chromatography to obtain a compound G22.

3-hydroxyazetidine hydrochloride (1.0 equivalent), methyl 3-fluoro-2-nitrobenzoate G7 (1.0 equivalent), and cesium carbonate (2.0 equivalents) were dissolved in N,N-dimethylformamide, and heated to 50°C for 2 hours of reaction under nitrogen protection. The reaction solution was diluted with a large amount of ethyl acetate after it was cooled to room temperature, and washed with saturated aqueous solution of ammonium chloride 3 times. The obtained organic phase was dried with anhydrous sodium sulfate, filtered and concentrated, and then separated by silica gel column chromatography to obtain a compound G24. The compound G24 (1.0 equivalent) was dissolved in dichloromethane, followed by adding imidazole (2.0 equivalents), cooling in an ice bath, adding tert-butyl dimethylchlorosilane (1.2 equivalents), restoring to room temperature and stirring for 14 hours. Saturated aqueous solution of ammonium chloride was added to quench the reaction, and the reaction solution was diluted and extracted with a large amount of ethyl acetate. The obtained organic phase was dried with anhydrous sodium sulfate, filtered and concentrated to obtain a crude product, and the crude product was further processed to obtain a compound G25 by referring to the nitro reduction experimental operations of prepareing the compound G9 from the compound G8.

Referring to the experimental operation for preparing the compound G10 from the compound G9, the tert-butyldimethylsilyl ether intermediate of compound G26 was prepared from the compound G25. The tert-butyldimethylsilyl ether intermediate (1.0 equivalent) of compound G26 was dissolved in dichloromethane, followed by adding tetrabutylammonium fluoride in tetrahydrofuran solution (1.0 mole per liter, 1.5 equivalents) and reacting at room temperature for more than 1 hour, and monitoring by thin-layer chromatography until the end of the reaction. The solvent was removed under reduced pressure, the reaction solution was diluted with a large amount of ethyl acetate, and washed with saturated aqueous solution of ammonium chloride, and the aqueous phase was extracted twice with ethyl acetate. The obtained organic phases were combined, dried with anhydrous sodium sulfate, filtered, concentrated, and separated by silica gel column chromatography to obtain a compound G26 (when R² is a pyrrole ring).

Referring to the experimental operation for preparing the compound G14 from the compound G9, compound G27 was prepared from the compound G25.

Referring to the experimental operation for preparing the compound G15 from the compound G14, the tert-butyldimethylsilyl ether intermediate of compound G26 was prepared from the compound G27, and the compound G26 was obtained from the intermediate by removing the tert-butyl dimethyl ether protective group with tetrabutylammonium fluoride.

Referring to the experimental operation for preparing the compound G24 from the compound G7, compound G28 was prepared from the compound G26 and the compound G23.

Referring to the nitro reduction experimental operation for preparing the compound G9 from the compound G8, compound G29 was prepared from the compound G28.

Under ice bath cooling, N,N'-carbonyldiimidazole (CDI) (3.0 equivalents) was added to the solvent of arylamine (R³-NH₂) in anhydrous THF (3.0 equivalents), followed by restoring to room temperature and stirring for more than 2 hours under nitrogen protection. After the compound G29 (1.0 equivalent) was added, the reaction was subjected to refluxing for 14 hours in the reaction system, and cooled to room temperature. Saturated aqueous solution of ammonium chloride was added to quench the reaction, the organic phase was removed under reduced pressure, and the reaction solution was diluted and extracted with a large amount of ethyl acetate. The obtained organic phase was dried with anhydrous sodium sulfate, filtered and concentrated, and then separated by silica gel column chromatography to obtain a compound G30.

Arylmethyl carboxylic acid (R³-CH₂CO₂H) (2.0 equivalents) was dissolved in an appropriate amount of anhydrous N,N-dimethylformamide, and then N,N-diisopropylethylamine (3.0 equivalents), the compound G29 (1.0 equivalent) and HATU (1.5 equivalents) were respectively under ice bath cooling. The reaction was restored to room temperature and stirred for more than 4 hours under nitrogen protection, and monitored by thin-layer chromatography until the end of the reaction. The reaction solution was diluted with a large amount of ethyl acetate, and washed with saturated aqueous solution of ammonium chloride, and the aqueous phase was extracted twice with ethyl acetate. The obtained organic phases were combined, dried with anhydrous sodium sulfate, filtered, concentrated, and separated by silica gel column chromatography to obtain a compound G31.

Referring to the experimental operation for preparing the compound G31 from the compound G29, compound G32 was prepared from the compound G29.

### General experimental operation 3:

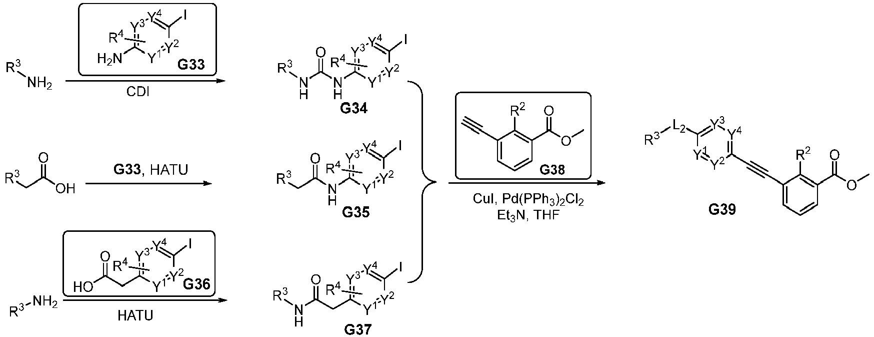

Referring to the experimental operation for preparing the compound G30 from the compound G29, compound G34 was prepared from arylamine (R³-NH₂) and the compound G33.

Referring to the experimental operation for preparing the compound G31 from the compound G29, compound G35 was prepared from arylmethyl carboxylic acid (R³-CH₂CO₂H) and the compound G33.

Referring to the experimental operation for preparing the compound G31 from the compound G29, compound G37 was prepared from arylamine (R³-NH₂) and the compound G36.

The compound G34 (1.0 equivalent) and the compound G38 (1.2 equivalents) were dissolved in anhydrous THF, and then triethylamine (2.0 equivalents) was added. Cuprous iodide (0.025 equivalents) and Pd(PPh₃)₂Cl₂ (0.05 equivalent) were pre-mixed and then added into the reaction system together. The reaction system was stirred at room temperature for 14 hours under nitrogen protection, the reaction solution was diluted with a large amount of ethyl acetate and washed with saturated aqueous solution of ammonium chloride, and the aqueous phase was extracted twice with ethyl acetate. The obtained organic phases were combined, dried with anhydrous sodium sulfate, filtered, concentrated, and separated by silica gel column chromatography to obtain a compound G39.

### Example 1:

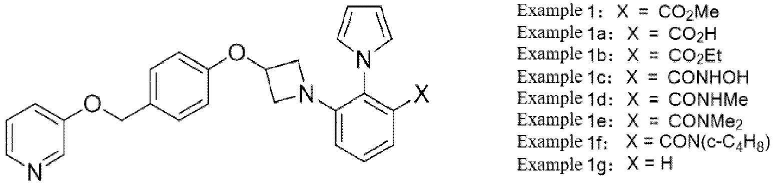

Compound 1 (9 mg, yield 50%) was synthesized according to the general experimental operation 1. ¹H NMR (300 MHz, CDCl₃) *δ* 8.37 (dd, *J=* 2.7, 1.0 Hz, 1H), 8.23 (dd, *J=* 4.1, 1.9 Hz, 1H), 7.41-7.09 (m, 6H), 6.83-6.55 (m, 5H), 6.25 (t, *J=* 2.1 Hz, 2H), 5.02 (s, 2H), 4.93-4.78 (m, 1H), 3.91-3.73 (m, 2H), 3.71-3.31 (m, 5H). Mass spectrum (mass-to-charge ratio): C₂₇H₂₅N₃NaO₄⁺ [M+Na]⁺; theoretical value: 478.1737; measured value: 478.1738.

### Example 1a:

Compound 1a (3.8 mg, yield 80%) was synthesized according to the general experimental operation 1. ¹H NMR (400 MHz, CDCl₃) *δ* 8.41-8.30 (m, 1H), 8.28-8.15 (m, 1H), 7.42-7.19 (m, 6H), 6.86-6.63 (m, 5H), 6.37-6.05 (m, 2H), 5.02 (s, 2H), 4.89-4.79 (m, 1H), 3.85-3.75 (m, 2H), 3.69-3.60 (m, 2H). Mass spectrum (mass-to-charge ratio): C₂₆H₂₃N₃NaO₄⁺ [M+Na]⁺; theoretical value: 464.1581; measured value: 464.1582.

### Example 1b:

Compound 1b (10.5 mg, yield 98%) was synthesized according to the general experimental operation 1. ¹H NMR (400 MHz, CDCl₃) *δ* 8.58 - 8.11 (m, 2H), 7.44 - 7.05 (m, 5H), 6.78 - 6.58 (m, 5H), 6.24 (t, *J=* 2.1 Hz, 2H), 5.02 (s, 2H), 4.90 - 4.77 (m, 1H), 4.06 (q, *J* = 7.1 Hz, 2H), 3.85 - 3.71 (m, 2H), 3.71 - 3.57 (m, 2H), 1.09 (t, *J* = 7.1 Hz, 3H). Mass spectrum (mass-to-charge ratio): C₂₈H₂₈N₃O₄⁺ [M+H]⁺; theoretical value: 470.2074; measured value: 470.2080.

### Example 1c:

Compound 1c (6.5 mg, yield 96%) was synthesized according to the general experimental operation 1. ¹H NMR (500 MHz, CDCl₃) *δ* 8.44 - 8.13 (m, 2H), 7.40 - 7.27 (m, 3H), 7.27 - 7.15 (m, 3H), 6.81 - 6.63 (m, 5H), 6.43 - 6.29 (m, 2H), 5.01 (s, 2H), 4.89 - 4.75 (m, 1H), 3.88 - 3.75 (m, 2H), 3.69 - 3.61 (m, 2H). Mass spectrum (mass-to-charge ratio): C₂₆H₂₅N₄O₄⁺ [M+H]⁺; theoretical value: 457.1870; measured value: 457.1870.

### Example 1d:

Compound 1d (6.6 mg, yield 64%) was synthesized according to the general experimental operation 1. ¹H NMR (500 MHz, CDCl₃) *δ* 8.47 - 8.08 (m, 2H), 7.42 - 7.14 (m, 6H), 6.75 (t, *J* = 2.1 Hz, 2H), 6.72 - 6.66 (m, 2H), 6.66 - 6.58 (m, 1H), 6.31 (t, *J=* 2.1 Hz, 2H), 5.12 - 5.03 (m, 1H), 5.01 (s, 2H), 4.89 - 4.76 (m, 1H), 2.61 (d, *J=* 4.9 Hz, 3H). 13C NMR (126 MHz, CDCl₃) *δ* 167.44, 156.83, 154.93, 147.05, 142.30, 138.27, 135.76, 129.49, 129.03, 128.93, 124.17, 123.94, 123.71, 121.56, 119.36, 115.32, 114.78, 110.27, 77.49, 77.23, 76.98, 69.95, 66.54, 59.44, 26.87. Mass spectrum (mass-to-charge ratio): C₂₇H₂₇N₄O₃⁺ [M+H]⁺; theoretical value: 455.2078; measured value: 455.2087.

### Example Ie:

Compound 1e (13.8 mg, yield 33%) was synthesized according to the general experimental operation one. ¹H NMR (300 MHz, CDCl₃) *δ* 8.54 - 8.03 (m, 2H), 7.41 - 7.07 (m, 5H), 6.97 - 6.64 (m, 5H), 6.64 - 6.53 (m, 1H), 6.21 (t, *J=* 2.0 Hz, 2H), 5.01 (s, 2H), 4.89 - 4.76 (m, 1H), 4.07 - 3.86 (m, 1H), 3.71 - 3.44 (m, 3H), 2.82 (s, 3H), 2.63 (s, 3H). Mass spectrum (mass-to-charge ratio): C₂₈H₂₉N₄O₃⁺ [M+H]⁺; theoretical value: 469.2234; measured value: 469.2229.

### Example 1f:

Compound 1f (10.4 mg, yield 92%) was synthesized according to the general experimental operation 1. ¹H NMR (500 MHz, CDCl₃) *δ* 8.29 (d, *J =* 69.3 Hz, 2H), 7.37 - 7.13 (m, 5H), 6.92 - 6.73 (m, 3H), 6.73 - 6.64 (m, 2H), 6.64 - 6.51 (m, 1H), 6.20 (t, *J=* 2.1 Hz, 2H), 5.00 (s, 2H), 4.87 - 4.77 (m, 1H), 3.79 (s, 2H), 3.64 - 3.53 (m, 2H), 3.43 - 3.12 (m, 2H), 3.08 - 2.91 (m, 2H), 1.77 - 1.57 (m, 4H). Mass spectrum (mass-to-charge ratio): C₃₀H₃₁N₄O₃⁺ [M+H]⁺; theoretical value: 495.2391; measured value: 495.2394.

### Example 1g:

Compound 1g (4.3 mg, yield 48%) was synthesized according to the general experimental operation 1. ¹H NMR (400 MHz, CDCl₃) *δ* 8.47 - 8.13 (m, 2H), 7.38 - 7.19 (m, 5H), 7.19 - 7.08 (m, 1H), 6.89 - 6.79 (m, 3H), 6.76 - 6.69 (m, 2H), 6.65 - 6.55 (m, 1H), 6.28 (t, *J=* 2.1 Hz, 2H), 5.02 (s, 2H), 4.91 - 4.81 (m, 1H), 3.89 - 3.76 (m, 2H), 3.69 - 3.57 (m, 2H). Mass spectrum (mass-to-charge ratio): C₂₅H₂₄N₃O₂⁺ [M+H]⁺; theoretical value: 398.1863; measured value: 398.1863.

### Example 2:

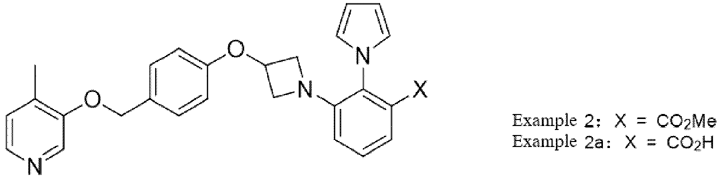

Compound 2 (6 mg, yield 25%) was synthesized according to the general experimental operation 1. ¹H NMR (500 MHz, CDCl₃) *δ* 8.19 (d, *J =* 45.1 Hz, 2H), 7.38 - 7.27 (m, 3H), 7.15 (dd, *J=* 7.6, 1.3 Hz, 1H), 7.11 (s, 1H), 6.77 - 6.63 (m, 5H), 6.26 (t, J= 2.1 Hz, 2H), 5.08 (s, 2H), 4.88 - 4.80 (m, 1H), 3.86 - 3.77 (m, 2H), 3.70 - 3.64 (m, 2H), 3.62 (s, 3H), 2.26 (s, 3H). Mass spectrum (mass-to-charge ratio): C₂₈H₂₈N₃O₄⁺ [M+H]⁺; theoretical value: 470.2074; measured value: 470.2076.

### Example 2a:

Compound 2a (3 mg, yield 98%) was synthesized according to the general experimental operation 1. ¹H NMR (500 MHz, CDCl₃) *δ* 8.13 (d, *J =* 47.0 Hz, 2H), 7.38 - 7.21 (m, 4H), 7.15 (s, 1H), 6.81 - 6.73 (m, 2H), 6.73 - 6.64 (m, 3H), 6.24 (t, *J=* 1.9 Hz, 2H), 5.07 (s, 2H), 4.89 - 4.78 (m, 1H), 3.85 - 3.75 (m, 2H), 3.73 - 3.59 (m, 2H). Mass spectrum (mass-to-charge ratio): C₂₇H₂₆N₃O₄⁺ [M+H]⁺; theoretical value: 456.1918; measured value: 456.1920.

### Example 3:

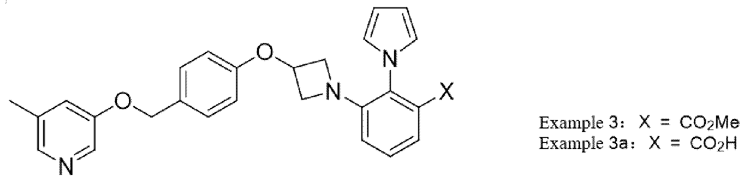

Compound 3 (7 mg, yield 28%) was synthesized according to the general experimental operation 1. ¹H NMR (400 MHz, CDCl₃) *δ* 8.14 (d, *J =* 40.5 Hz, 2H), 7.38 - 7.22 (m, 3H), 7.21 - 7.11 (m, 1H), 7.08 (s, 1H), 6.77 - 6.61 (m, 5H), 6.26 (t, *J=* 2.1 Hz, 2H), 5.00 (s, 2H), 4.89 - 4.78 (m, 1H), 3.85 - 3.75 (m, 2H), 3.70 - 3.63 (m, 2H), 3.62 (s, 3H), 2.32 (s, 3H). Mass spectrum (mass-to-charge ratio): C₂₈H₂₈N₃O₄⁺ [M+H]⁺; theoretical value: 470.2074; measured value: 470.2084.

### Example 3a:

Compound 3a (4.3 mg, yield 44%) was synthesized according to the general experimental operation 1. ¹H NMR (400 MHz, CDCl₃) *δ* 8.26 - 7.91 (m, 2H), 7.39 - 7.19 (m, 4H), 7.13 (s, 1H), 6.84 - 6.62 (m, 5H), 6.34 - 6.16 (m, 2H), 5.00 (s, 2H), 4.88 - 4.78 (m, 1H), 3.85 - 3.69 (m, 2H), 3.65 (dd, *J* = 8.7, 4.8 Hz, 2H), 2.32 (s, 3H). Mass spectrum (mass-to-charge ratio): C₂₇H₂₆N₃O₄⁺ [M+H]⁺; theoretical value: 456.1918; measured value: 456.1920.

### Example 4:

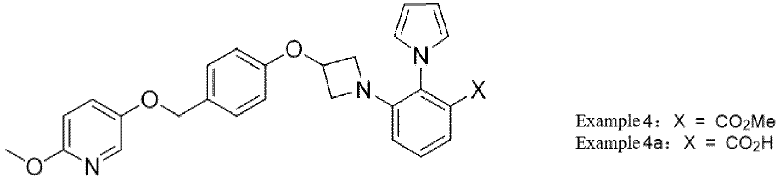

Compound 4 (11.4 mg, yield 44%) was synthesized according to the general experimental operation 1. ¹H NMR (400 MHz, CDCl₃) *δ* 7.90 - 7.78 (m, 1H), 7.39 - 7.07 (m, 5H), 6.76 - 6.60 (m, 6H), 6.26 (t, *J=* 2.1 Hz, 2H), 4.95 (s, 2H), 4.89 - 4.78 (m, 1H), 3.89 (s, 3H), 3.86 - 3.76 (m, 2H), 3.69 - 3.63 (m, 2H), 3.62 (s, 3H). Mass spectrum (mass-to-charge ratio): C₂₈H₂₈N₃O₅⁺ [M+H]⁺; theoretical value: 486.2023; measured value: 486.2028.

### Example 4a:

Compound 4a (1.3 mg, yield 27%) was synthesized according to the general experimental operation 1. ¹H NMR (500 MHz, CDCl₃) *δ* 8.11 (s, 1H), 7.87 - 7.81 (m, 1H), 7.37 - 7.18 (m, 5H), 6.78 - 6.64 (m, 5H), 6.30 - 6.22 (m, 2H), 4.95 (s, 2H), 4.86 - 4.80 (m, 1H), 3.89 (s, 3H), 3.82 - 3.73 (m, 2H), 3.69 - 3.61 (m, 2H). Mass spectrum (mass-to-charge ratio): C₂₇H₂₆N₃O₅⁺ [M+H]⁺; theoretical value: 472.1867; measured value: 472.1869.

### Example 5:

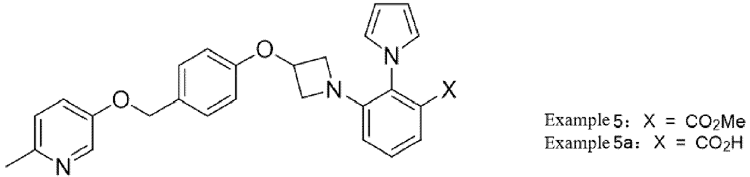

Compound 5 (16 mg, yield 42%) was synthesized according to the general experimental operation 1. ¹H NMR (300 MHz, CDCl₃) *δ* 8.24 (d, *J=* 2.3 Hz, 1H), 7.40 - 6.97 (m, 6H), 6.76 - 6.60 (m, 5H), 6.25 (t, *J=* 2.1 Hz, 2H), 4.98 (s, 2H), 4.89 - 4.77 (m, 1H), 3.86 - 3.73 (m, 2H), 3.69 - 3.57 (m, 5H), 2.49 (s, 3H). Mass spectrum (mass-to-charge ratio): C₂₈H₂₈N₃O₄⁺ [M+H]⁺; theoretical value: 470.2074; measured value: 470.2076.

### Example 5a:

Compound 5a (8.1 mg, yield 83%) was synthesized according to the general experimental operation 1. ¹H NMR (400 MHz, CDCl₃) *δ* 8.33 - 8.09 (m, 1H), 7.38 - 7.16 (m, 5H), 7.16 - 6.98 (m, 1H), 6.85 - 6.59 (m, 5H), 6.34 - 6.11 (m, 2H), 4.99 (s, 2H), 4.88 - 4.76 (m, 1H), 3.85 - 3.73 (m, 2H), 3.73 - 3.56 (m, 2H), 2.47 (s, 2H). Mass spectrum (mass-to-charge ratio): C₂₇H₂₆N₃O₄⁺ [M+H]⁺; theoretical value: 456.1918; measured value: 456.1917.

### Example 6:

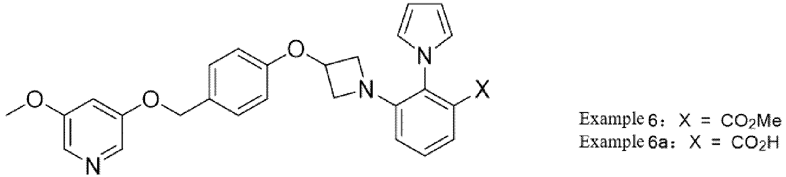

Compound 6 (17 mg, yield 44%) was synthesized according to the general experimental operation 1. ¹H NMR (300 MHz, CDCl₃) *δ* 8.12 - 7.85 (m, 2H), 7.37 - 7.22 (m, 3H), 7.20 - 7.10 (m, 1H), 6.84 - 6.64 (m, 6H), 6.25 (t, *J=* 2.1 Hz, 2H), 5.00 (s, 2H), 4.89 - 4.79 (m, 1H), 3.88 - 3.74 (m, 5H), 3.71 - 3.57 (m, 5H). Mass spectrum (mass-to-charge ratio): C₂₈H₂₇N₃NaO₅⁺ [M+Na]⁺; theoretical value: 508.1843; measured value: 508.1845.

### Example 6a:

Compound 6a (3.6 mg, yield 52%) was synthesized according to the general experimental operation 1. ¹H NMR (400 MHz, CDCl₃) *δ* 7.97 (s, 2H), 7.38 - 7.19 (m, 5H), 6.88 - 6.64 (m, 5H), 6.26 (t, *J=* 2.0 Hz, 2H), 5.00 (s, 2H), 4.90 - 4.77 (m, 1H), 3.88 - 3.72 (m, 5H), 3.71 - 3.60 (m, 2H). Mass spectrum (mass-to-charge ratio): C₂₇H₂₆N₃O₅⁺ [M+H]⁺; theoretical value: 472.1867; measured value: 472.1873.

### Example 7:

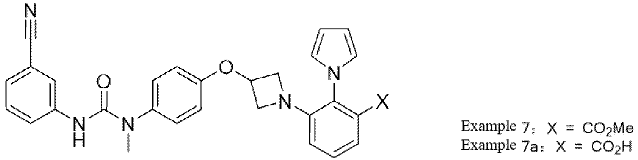

Compound 7 (19 mg, yield 68%) was synthesized according to the general experimental operation 1. ¹H NMR (300 MHz, CDCl₃) *δ* 7.66 (t, *J=* 1.9 Hz, 1H), 7.48 (dt, *J=* 8.1, 1.8 Hz, 1H), 7.30 *(t, J=* 8.1 Hz, 2H), 7.24-7.15 (m, 3H), 6.83-6.74 (m, 2H), 6.74-6.66 (m, 3H), 6.31-6.21 (m, 3H), 4.92-4.79 (m, 1H), 3.83 (dd, *J=* 8.7, 6.2 Hz, 2H), 3.67 (dd, *J=* 8.8, 4.8 Hz, 2H), 3.61 (s, 3H), 3.28 (s, 3H). High-resolution mass spectrum (mass-to-charge ratio): C₃₀H₂₇N₅O₄Na⁺ [M+Na]⁺; theoretical value: 544.1961; measured value: 544.1956.

### Example 7a:

Compound 7a (2 mg, yield 30%) was synthesized according to the general experimental operation 1. ¹H NMR (300 MHz, MeOH-*d*₄) *δ* 7.82-7.77 (m, 1H), 7.62-7.56 (m, 1H), 7.40 (d, *J=* 7.7 Hz, 1H), 7.35-7.30 (m, 1H), 7.29 (s, 1H), 7.29-7.21 (m, 2H), 7.03-6.96 (m, 1H), 6.84 (d, *J* = 9.0 Hz, 2H), 6.78-6.66 (m, 3H), 6.16 (s, 2H), 4.81-4.75 (m, 1H), 3.88-3.79 (m, 2H), 3.56-3.48 (m, 3H), 3.26 (s, 3H). Mass spectrum (mass-to-charge ratio): C₂₉H₂₅N₅O₄Na⁺ [M+Na]⁺; theoretical value: 530.1804; measured value: 530.1802.

### Example 8:

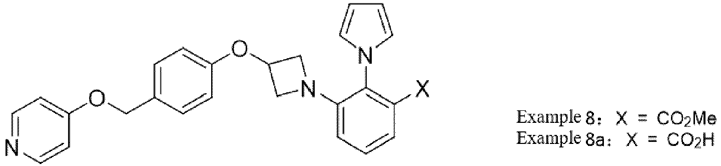

Compound 8 (4 mg, yield 16%) was synthesized according to the general experimental operation 1. ¹H NMR (400 MHz, CDCl₃) *δ* 8.76 - 8.22 (m, 2H), 7.36 - 7.22 (m, 4H), 7.16 (dd, J= 7.7, 1.4 Hz, 1H), 6.90 (s, 1H), 6.76 - 6.63 (m, 5H), 6.26 (t, *J=* 2.1 Hz, 2H), 5.02 (s, 2H), 4.90 - 4.79 (m, 2H), 3.81 (dd, *J=* 9.1, 6.2 Hz, 2H), 3.70 - 3.55 (m, 5H). Mass spectrum (mass-to-charge ratio): C₂₇H₂₆N₃O₄⁺ [M+H]⁺; theoretical value: 456.1918; measured value: 456.1918.

### Example 8a:

Compound 8a (3.2 mg, yield 94%) was synthesized according to the general experimental operation 1. ¹H NMR (500 MHz, CDCl₃) *δ* 8.59 - 8.15 (m, 2H), 7.42 - 7.11 (m, 5H), 6.89 (s, 1H), 6.79 - 6.55 (m, 5H), 6.29 - 6.06 (m, 2H), 5.02 (s, 2H), 4.87 - 4.75 (m, 1H), 3.84 - 3.69 (m, 2H), 3.68 - 3.52 (m, 2H). Mass spectrum (mass-to-charge ratio): C₂₆H₂₄N₃O₄⁺ [M+H]⁺; theoretical value: 442.1761; measured value: 442.1760.

### Example 9:

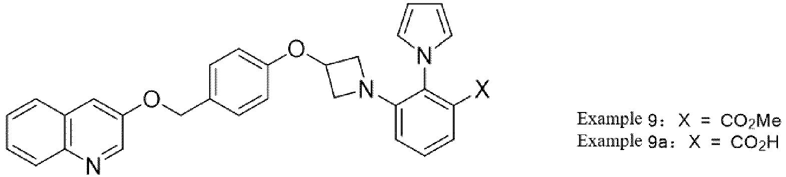

Compound 9 (18 mg, yield 27%) was synthesized according to the general experimental operation 1. ¹H NMR (400 MHz, CDCl₃) *δ* 8.80 - 8.64 (m, 1H), 8.11 - 7.97 (m, 1H), 7.77 - 7.64 (m, 1H), 7.62 - 7.43 (m, 3H), 7.39 (d, *J=* 8.6 Hz, 2H), 7.35 - 7.22 (m, 1H), 7.22 - 7.07 (m, 1H), 6.81 - 6.61 (m, 5H), 6.26 (t, *J=* 2.1 Hz, 2H), 5.11 (s, 2H), 4.90 - 4.80 (m, 1H), 3.86 - 3.77 (m, 2H), 3.71 - 3.64 (m, 2H), 3.62 (s, 3H). Mass spectrum (mass-to-charge ratio): C₃₁H₂₈N₃O₄⁺ [M+H]⁺; theoretical value: 506.2074; measured value: 506.2078.

### Example 9a:

Compound 9a (8.1 mg, yield 84%) was synthesized according to the general experimental operation 1. ¹H NMR (300 MHz, CDCl₃) *δ* 8.88 - 8.42 (m, 1H), 8.13 - 7.89 (m, 1H), 7.88 - 7.65 (m, 1H), 7.65 - 7.45 (m, 3H), 7.45 - 7.24 (m, 3H), 7.24 - 7.07 (m, 1H), 6.87 - 6.53 (m, 5H), 6.35 - 6.10 (m, 2H), 5.12 (s, 2H), 4.85 (dd, *J=* 10.6, 5.7 Hz, 1H), 3.84 - 3.69 (m, 2H), 3.71 - 3.52 (m, 2H). Mass spectrum (mass-to-charge ratio): C₃₀H₂₆N₃O₄⁺ [M+H]⁺; theoretical value: 492.1918; measured value: 492.1919.

### Example 10:

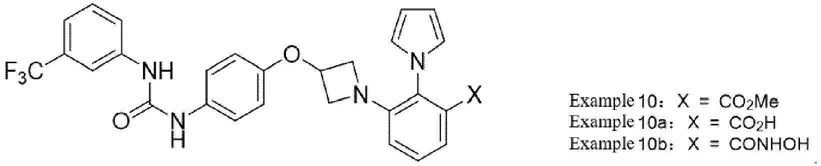

Compound 10 (11 mg, yield 44%) was synthesized according to general experimental operation 2. ¹H NMR (400 MHz, CDCl₃) *δ* 7.54-7.44 (m, 2H), 7.32 (d, *J* = 5.5 Hz, 1H), 7.23 (d, *J=* 7.7 Hz, 1H), 7.17-7.06 (m, 4H), 6.69 (q, *J=* 2.2 Hz, 2H), 6.65 (dd, *J* = 8.3, 1.4 Hz, 1H), 6.60-6.54 (m, 2H), 6.23 (t, *J* = 2.1 Hz, 2H), 4.70 (p, *J=* 5.3 Hz, 1H), 3.75 (dd, *J=* 8.6, 6.1 Hz, 2H), 3.58 (dd, *J=* 8.9, 4.7 Hz, 5H). Mass spectrum (mass-to-charge ratio): C₂₉H₂₅F₃N₄O₄Na⁺ [M+Na]⁺; theoretical value: 573.1726; Found value: 573.1722.

### Example 10a:

Compound 10a (4.6 mg, yield 72%) was synthesized according to general experimental operation 2. ¹H NMR (400 MHz, MeOH-*d*₄) *δ* 7.87 (d, *J=* 2.0 Hz, 1H), 7.57 (dd, *J =* 8.2, 2.1 Hz, 1H), 7.44 (t, *J=* 8.0 Hz, 1H), 7.33-7.25 (m, 4H), 7.03 (dd, *J=* 7.6, 1.3 Hz, 1H), 6.73-6.66 (m, 5H), 6.18 (t, *J=* 2.1 Hz, 2H), 4.82-4.79 (m, 1H), 3.80 (dd, *J=* 8.7, 6.0 Hz, 2H), 3.51 (dd, *J* = 8.9, 4.3 Hz, 2H). Mass spectrum (mass-to-charge ratio): C₂₈H₂₃F₃N₄O₄Na⁺ [M+Na]⁺; theoretical value: 559.1569; Found value: 559.1565.

### Example 10b:

Compound 10b (2.5 mg, yield 14.4%) was synthesized according to general experimental operation 2. ¹H NMR (400 MHz, MeOH-*d*₄) *δ* 7.98 - 7.79 (m, 1H), 7.59 (dd, *J=* 8.3, 2.1 Hz, 1H), 7.46 (t, *J=* 8.0 Hz, 1H), 7.38 - 7.24 (m, 4H), 6.90 - 6.62 (m, 6H), 6.21 (t, *J* = 2.2 Hz, 2H), 3.83 (dd, *J =* 8.7, 6.1 Hz, 2H), 3.51 (td, *J=* 10.5, 9.6, 4.0 Hz, 2H). Mass spectrum (mass-to-charge ratio): C₂₈H₂₄F₃N₅NaO₄⁺ [M+Na]⁺; theoretical value: 574.16726 measured value: 574.16748.

### Example 11:

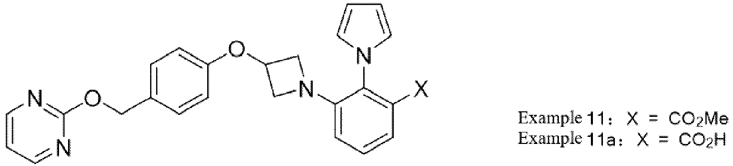

Compound 11 (8.7 mg, yield 30%) was synthesized according to the general experimental operation 1. ¹H NMR (300 MHz, CDCl₃) *δ* 8.62 - 8.42 (m, 2H), 7.39 (d, *J=* 8.6 Hz, 2H), 7.33 - 7.26 (m, 1H), 7.19 - 7.07 (m, 1H), 6.94 (t, *J=* 4.7 Hz, 1H), 6.75 - 6.57 (m, 5H), 6.31 - 6.14 (m, 2H), 5.36 (s, 2H), 4.88 - 4.76 (m, 1H), 3.84 - 3.73 (m, 2H), 3.67 - 3.57 (m, 5H). Mass spectrum (mass-to-charge ratio): C₂₆H₂₅N₄O₄⁺ [M+H]⁺; theoretical value: 457.1870; measured value: 457.1877.

### Example 11a:

Compound 11a (5.4 mg, yield 93%) was synthesized according to the general experimental operation 1. ¹H NMR (300 MHz, CDCl₃) *δ* 8.52 (d, *J=* 4.7 Hz, 2H), 7.39 (d, *J=* 8.6 Hz, 2H), 7.32 - 7.18 (m, 1H), 7.08 - 6.99 (m, 1H), 6.94 (t, *J=* 4.8 Hz, 1H), 6.76 - 6.54 (m, 5H), 6.23 (t, *J=* 2.1 Hz, 2H), 5.36 (s, 2H), 4.87 - 4.77 (m, 1H), 3.82 - 3.68 (m, 2H), 3.68 - 3.55 (m, 2H). Mass spectrum (mass-to-charge ratio): C₂₅H₂₃N₄O₄⁺ [M+H]⁺; theoretical value: 443.1714; measured value: 443.1714.

### Example 12:

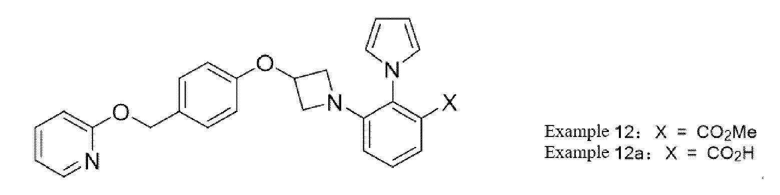

Compound 12 (5.8 mg, yield 83%) was synthesized according to the general experimental operation 1. ¹H NMR (300 MHz, CDCl₃) *δ* 8.25 - 8.09 (m, 1H), 7.65 - 7.51 (m, 1H), 7.43 - 7.22 (m, 3H), 7.21 - 7.07 (m, 1H), 6.94 - 6.83 (m, 1H), 6.83 - 6.61 (m, 6H), 6.25 (t, *J=* 2.1 Hz, 2H), 5.29 (s, 2H), 4.89 - 4.78 (m, 1H), 3.87 - 3.73 (m, 2H), 3.70 - 3.57 (m, 5H). Mass spectrum (mass-to-charge ratio): C₂₇H₂₆N₃O₄⁺ [M+H]⁺; theoretical value: 456.1918; measured value: 456.1917.

### Example 12a:

Compound 12a (19.7 mg, yield 24%) was synthesized according to the general experimental operation 1. ¹H NMR (500 MHz, CDCl₃) *δ* 8.23 - 8.09 (m, 1H), 7.63 - 7.51 (m, 1H), 7.40 - 7.19 (m, 4H), 6.93 - 6.83 (m, 1H), 6.77 (d, *J=* 8.4 Hz, 1H), 6.74 - 6.62 (m, 5H), 6.21 (t, *J=* 2.1 Hz, 2H), 5.24 (s, 2H), 4.86 - 4.77 (m, 1H), 3.82 - 3.72 (m, 2H), 3.68 - 3.58 (m, 2H). Mass spectrum (mass-to-charge ratio): C₂₆H₂₄N₃O₄⁺ [M+H]⁺; theoretical value: 442.1761; measured value: 442.1766.

### Example 13:

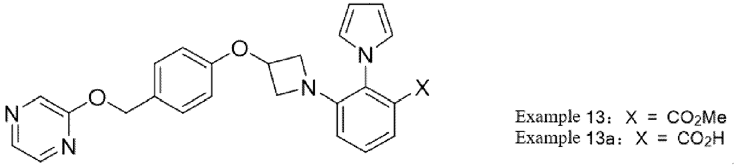

Compound 13 (6 mg, yield 33%) was synthesized according to the general experimental operation 1. ¹H NMR (400 MHz, CDCl₃) *δ* 8.25 (s, 1H), 8.20 - 8.06 (m, 2H), 7.40 - 7.33 (m, 2H), 7.33 - 7.27 (m, 1H), 7.19 - 7.08 (m, 1H), 6.76 - 6.63 (m, 5H), 6.25 (t, *J* = 2.1 Hz, 2H), 5.31 (s, 2H), 4.88 - 4.81 (m, 1H), 3.86 - 3.75 (m, 2H), 3.69 - 3.63 (m, 2H), 3.62 (s, 3H). Mass spectrum (mass-to-charge ratio): C₂₆H₂₅N₄O₄⁺ [M+H]⁺; theoretical value: 457.1870; measured value: 457.1872.

### Example 13a:

Compound 13a (4 mg, yield 9%) was synthesized according to general experimental operation 1. ¹H NMR (500 MHz, CDCl₃) *δ* 8.23 (s, 1H), 8.16 - 8.03 (m, 2H), 7.40 - 7.26 (m, 4H), 6.75 - 6.64 (m, 5H), 6.25 (t, *J* = 2.1 Hz, 2H), 5.29 (s, 2H), 4.86 - 4.78 (m, 1H), 3.80 - 3.74 (m, 2H), 3.68 - 3.61 (m, 2H). Mass spectrum (mass-to-charge ratio): C₂₅H₂₃N₄O₄⁺ [M+H]⁺; theoretical value: 443.1714; measured value: 443.1708.

### Example 14:

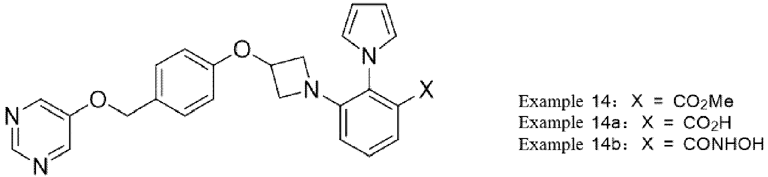

Compound 14 (3 mg, yield 17%) was synthesized according to the general experimental operation 1. ¹H NMR (500 MHz, CDCl₃) *δ* 8.86 (s, 1H), 8.45 (s, 2H), 7.32 (t, *J* = 8.3 Hz, 3H), 7.16 (dd, *J=* 7.7, 1.3 Hz, 1H), 6.76-6.66 (m, 5H), 6.26 (t, *J=* 2.1 Hz, 2H), 5.08 (s, 2H), 4.88-4.81 (m, 1H), 3.86-3.78 (m, 2H), 3.69-3.63 (m, 2H), 3.62 (s, 3H). High-resolution mass spectrum (mass-to-charge ratio): C₂₆H₂₅N₄O₄⁺ [M+H]⁺; theoretical value: 457.1870; measured value: 457.1873.

### Example 14a:

Compound 14a (1.4 mg, yield 48%) was synthesized according to general experimental operation 1. ¹H NMR (400 MHz, CDCl₃) *δ* 8.84 (s, 1H), 8.46 (s, 2H), 7.42-7.15 (m, 4H), 6.86-6.59 (m, 5H), 6.24 (s, 2H), 5.08 (s, 2H), 4.89-4.78 (m, 1H), 3.83-3.72 (m, 2H), 3.69-3.57 (m, 2H). High resolution mass spectrum (mass-to-charge ratio): C₂₅H₂₃N₄O₄⁺ [M+H]⁺; theoretical value: 443.1714; measured value: 443.1716.

### Example 14b:

Compound 14b (1.7 mg, yield 20%) was synthesized according to the general experimental operation 1. ¹H NMR (300 MHz, CDCl₃) *δ* 8.85 (s, 1H), 8.45 (s, 2H), 7.45 - 7.28 (m, 4H), 6.87 - 6.58 (m, 5H), 6.41 (t, *J=* 1.9 Hz, 2H), 5.08 (s, 2H), 4.93 - 4.78 (m, 1H), 3.89 - 3.75 (m, 2H), 3.75 - 3.59 (m, 2H). Mass spectrum (mass-to-charge ratio): C₂₅H₂₃N₅NaO₄⁺ [M+Na]⁺; theoretical value: 480.16423; measured value: 480.16483.

### Example 15:

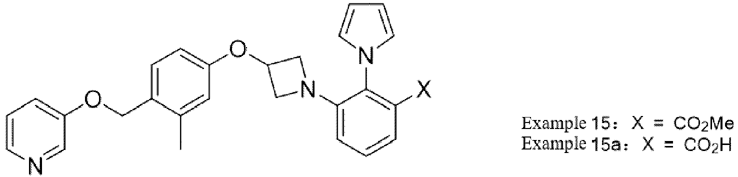

Compound 15 (5 mg, yield 10.5%) was synthesized according to the general experimental operation 1. ¹H NMR (500 MHz, CDCl₃) *δ* 8.49 - 8.15 (m, 2H), 7.31 - 7.24 (m, 4H), 7.15 (dd, *J=* 7.7, 1.3 Hz, 1H), 6.71 (t, *J=* 2.1 Hz, 2H), 6.68 (dd, *J=* 8.2, 1.3 Hz, 1H), 6.61 (d, *J=* 2.4 Hz, 1H), 6.51 (dd, *J=* 8.3, 2.6 Hz, 1H), 6.25 (t, *J* = 2.1 Hz, 2H), 5.00 (s, 2H), 4.88 - 4.78 (m, 1H), 3.85 - 3.75 (m, 2H), 3.67 - 3.63 (m, 2H), 3.61 (s, 3H), 2.33 (s, 3H). Mass spectrum (mass-to-charge ratio): C₂₈H₂₈N₃O₄⁺ [M+H]⁺; theoretical value: 470.20743; measured value: 470.20764.

### Example 15a:

Compound 15a (2 mg, yield 34.36%) was synthesized according to general experimental operation 1. ¹H NMR (500 MHz, CDCl₃) *δ* 8.37 (s, 1H), 8.24 (s, 1H), 7.34 - 7.24 (m, 5H), 6.75 (t, *J=* 2.1 Hz, 2H), 6.71 (dd, *J=* 8.0, 1.4 Hz, 1H), 6.61 (d, *J=* 2.4 Hz, 1H), 6.50 (dd, *J=* 8.3, 2.5 Hz, 1H), 6.27 (t, *J=* 2.0 Hz, 2H), 5.00 (s, 2H), 4.88 - 4.78 (m, 1H), 3.86 - 3.74 (m, 2H), 3.68 - 3.57 (m, 2H), 2.33 (s, 3H). Mass spectrum (mass-to-charge ratio): C₂₇H₂₆N₃O₄⁺ [M+H]⁺; theoretical value: 456.19178; found value: 456.19189.

### Example 16:

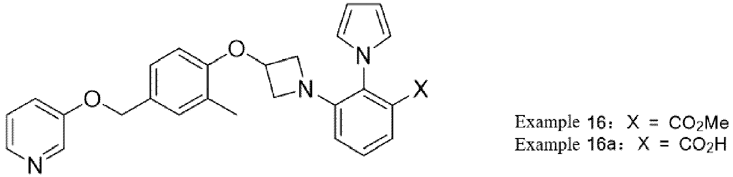

Compound 16 (5 mg, yield 6.43%) was synthesized according to the general experimental operation 1. ¹H NMR (500 MHz, CDCl₃) *δ* 8.38 (s, 1H), 8.24 (s, 1H), 7.32 - 7.28 (m, 1H), 7.28 - 7.21 (m, 2H), 7.21 - 7.19 (m, 1H), 7.17 - 7.13 (m, 2H), 6.71 (t, *J=* 2.1 Hz, 2H), 6.69 (dd, *J* = 8.2, 1.3 Hz, 1H), 6.44 (d, *J* = 8.3 Hz, 1H), 6.26 (t, *J=* 2.1 Hz, 2H), 4.99 (s, 2H), 4.89 - 4.79 (m, 1H), 3.85 - 3.77 (m, 2H), 3.68 - 3.64 (m, 2H), 3.62 (s, 3H), 2.19 (s, 3H). Mass spectrum (mass-to-charge ratio): C₂₈H₂₈N₃O₄⁺ [M+H]⁺; theoretical value: 470.20743; measured value: 470.2076.

### Example 16a:

Compound 16a (4 mg, yield 58.90%) was synthesized according to the general experimental operation 1. ¹H NMR (500 MHz, CDCl₃) *δ* 8.34 (s, 1H), 8.21 (s, 1H), 7.32 - 7.27 (m, 2H), 7.25 - 7.21 (m, 2H), 7.20 - 7.18 (m, 1H), 7.17 - 7.12 (m, 1H), 6.76 - 6.71 (m, 2H), 6.69 (d, *J=* 7.8 Hz, 1H), 6.43 (d, *J=* 8.3 Hz, 1H), 6.27 - 6.22 (m, 2H), 4.98 (s, 2H), 4.87 - 4.80 (m, 1H), 3.81 - 3.74 (m, 2H), 3.67 - 3.63 (m, 2H), 2.19 (s, 3H). Mass spectrum (mass-to-charge ratio): C₂₇H₂₆N₃O₄⁺ [M+H]⁺; theoretical value: 456.19178; measured value: 456.19193.

### Example 17:

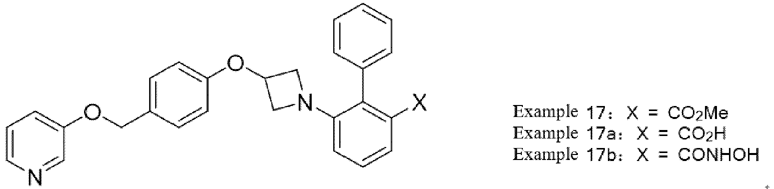

Compound 17 (31 mg, yield 35.2%) was synthesized according to the general experimental operation 1. ¹H NMR (300 MHz, CDCl₃) *δ* 8.37 (s, 1H), 8.25 (s, 1H), 7.46 - 7.13 (m, 11H), 6.74 - 6.59 (m, 3H), 5.02 (s, 2H), 4.74 (ddd, *J=* 6.2, 4.8, 1.4 Hz, 1H), 3.77 - 3.63 (m, 2H), 3.57 - 3.42 (m, 5H). Mass spectrum (mass-to-charge ratio): C₂₉H₂₇N₂O₄⁺ [M+H]⁺ ; theoretical value: 467.19653; measured value: 467.19669.

### Example 17a:

Compound 17a (25 mg, yield 85%) was synthesized according to the general experimental operation 1. ¹H NMR (500 MHz, MeOH-*d*₄) *δ* 8.31 - 8.15 (m, 1H), 8.15 - 8.00 (m, 1H), 7.52 - 7.39 (m, 1H), 7.39 - 7.17 (m, 9H), 7.17 - 7.00 (m, 1H), 6.78 - 6.61 (m, 3H), 5.05 (s, 2H), 4.79 - 4.68 (m, 1H), 3.78 - 3.63 (m, 2H), 3.41 - 3.32 (m, 2H). Mass spectrum (mass-to-charge ratio): C₂₈H₂₅N₂O₄⁺ [M+H]⁺; theoretical value: 453.2; measured value: 453.2.

### Example 17b:

Compound 17b (6.7 mg, yield 79%) was synthesized according to the general experimental operation 1. ¹H NMR (500 MHz, MeOH-*d*₄) *δ* 8.28 - 8.16 (m, 1H), 8.12 - 8.05 (m, 1H), 7.49 - 7.39 (m, 1H), 7.38 - 7.23 (m, 9H), 6.85 (d, *J* = 7.4 Hz, 1H), 6.74 - 6.63 (m, 3H), 5.05 (s, 2H), 4.78 - 4.72 (m, 1H), 4.58 (s, 1H), 3.78 - 3.64 (m, 2H), 3.41 - 3.35 (m, 2H). Mass spectrum (mass-to-charge ratio): C₂₈H₂₆N₃O₄⁺ [M+H]⁺; theoretical value: 468.2; measured value: 468.2.

### Example 18:

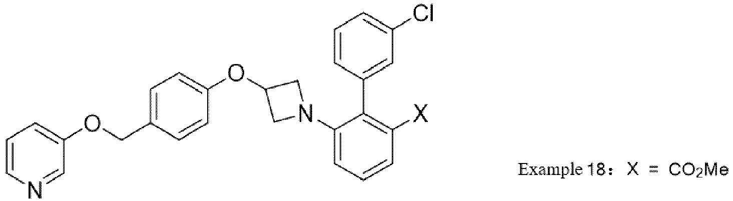

Compound 18 (15 mg, yield 19.0%) was synthesized according to the general experimental operation 1. ¹H NMR (400 MHz, CDCl₃) *δ* 8.39 (s, 1H), 8.25 (d, *J=* 4.5 Hz, 1H), 7.44 - 7.12 (m, 11H), 6.76 - 6.65 (m, 3H), 5.04 (s, 2H), 4.79 (p, *J=* 5.4 Hz, 1H), 3.81 (s, 1H), 3.71 (s, 2H), 3.57 (s, 3H), 3.50 (s, 1H). Mass spectrum (mass-to-charge ratio): C₂₉H₂₆ClN₂O₄⁺ [M+H]⁺; theoretical value: 501.15756; measured value: 501.15768.

### Example 19:

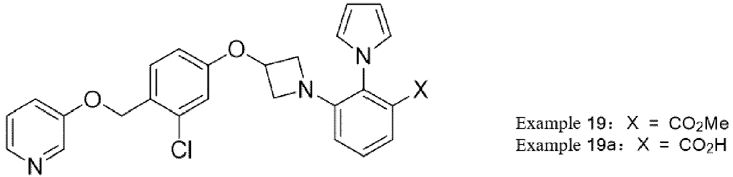

Compound 19 (5 mg, yield 4.21%) was synthesized according to the general experimental operation 1. ¹H NMR (500 MHz, CDCl₃) *δ* 8.39 (s, 1H), 8.26 (s, 1H), 7.39 (d, J = 8.5 Hz, 1H), 7.32 - 7.28 (m, 1H), 7.26 - 7.22 (m, 2H), 7.16 (dd, *J* = 7.6, 1.2 Hz, 1H), 6.78 (d, *J=* 2.5 Hz, 1H), 6.71 (t, *J=* 2.1 Hz, 2H), 6.68 (dd, *J=* 8.2, 1.2 Hz, 1H), 6.62 (dd, *J=* 8.5, 2.5 Hz, 1H), 6.26 (t, *J* = 2.1 Hz, 2H), 5.12 (s, 2H), 4.85 - 4.77 (m, 1H), 3.81 (dd, J= 8.9, 6.3 Hz, 2H), 3.64 (dd, *J* = 9.1, 4.8 Hz, 2H), 3.62 (s, 3H). Mass spectrum (mass-to-charge ratio): C₂₇H₂₅ClN₃O₄⁺ [M+H]⁺; theoretical value: 490.15281; measured value: 490.15295.

### Example 19a:

Compound 19a (3 mg, yield 44.12%) was synthesized according to general experimental operation 1. ¹H NMR (300 MHz, CDCl₃) *δ* 8.35 (s, 1H), 8.23 (s, 1H), 7.39 (d, *J* = 8.5 Hz, 1H), 7.33 - 7.20 (m, 4H), 6.80 - 6.58 (m, 5H), 6.25 (s, 2H), 5.11 (s, 2H), 4.88 - 4.74 (m, 1H), 3.83 - 3.72 (m, 2H), 3.65 - 3.58 (m, 2H). Mass spectrum (mass-to-charge ratio): C₂₆H₂₃ClN₃O₄⁺ [M+H]⁺; theoretical value: 476.13716; measured value: 476.13736.

### Example 20:

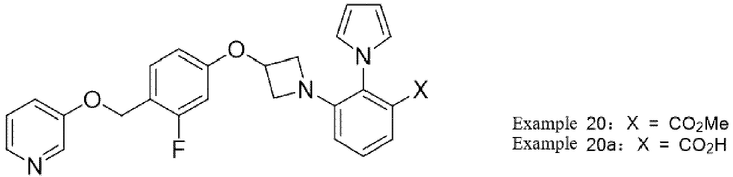

Compound 20 (5 mg, yield 4.92%) was synthesized according to the general experimental operation 1. ¹H NMR (400 MHz, CDCl₃) *δ* 8.43 (s, 1H), 8.30 (s, 1H), 7.39 - 7.28 (m, 4H), 7.18 (dd, *J=* 7.6, 1.1 Hz, 1H), 6.74 - 6.67 (m, 3H), 6.56 - 6.45 (m, 2H), 6.28 (t, *J=* 2.0 Hz, 2H), 5.09 (s, 2H), 4.88 - 4.80 (m, 1H), 3.83 (dd, *J=* 8.9, 6.3 Hz, 2H), 3.67 (dd, *J* = 9.0, 4.8 Hz, 2H), 3.64 (s, 3H). Mass spectrum (mass-to-charge ratio): C₂₇H₂₅FN₃O₄⁺ [M+H]⁺; theoretical value: 474.18236; measured value: 474.18250.

### Example 20a:

Compound 20a (1.5 mg, yield 30.92%) was synthesized according to general experimental operation 1. ¹H NMR (500 MHz, CDCl₃) *δ* 8.65 - 7.97 (m, 2H), 7.37 - 7.27 (m, 4H), 7.23 - 7.13 (m, 1H), 6.72 (s, 2H), 6.66 - 6.59 (m, 1H), 6.51 - 6.41 (m, 2H), 6.25 - 6.13 (m, 2H), 5.06 (s, 2H), 4.83 - 4.73 (m, 1H), 3.79 - 3.72 (m, 2H), 3.65 - 3.57 (m, 2H). Mass spectrum (mass-to-charge ratio): C₂₆H₂₃FN₃O₄⁺ [M+H]⁺; theoretical value: 460.16671 Measured value: 460.16672.

### Example 21:

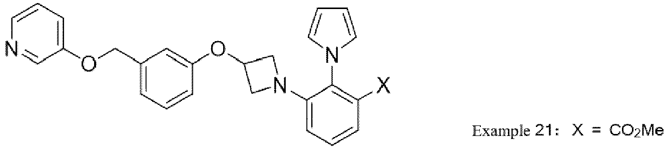

Compound 21 (3 mg, yield 9.59%) was synthesized according to the general experimental operation 1. ¹H NMR (500 MHz, CDCl₃) *δ* 8.52 - 8.33 (m, 1H), 8.33 - 8.18 (m, 1H), 7.32 - 7.27 (m, 2H), 7.26 - 7.22 (m, 2H), 7.15 (dd, *J* = 7.7, 1.3 Hz, 1H), 7.01 (d, *J* = 7.6 Hz, 1H), 6.79 - 6.77 (m, 1H), 6.70 (t, *J* = 2.1 Hz, 2H), 6.68 (dd, *J* = 8.2, 1.3 Hz, 1H), 6.64 (dd, *J* = 8.1, 2.3 Hz, 1H), 6.25 (t, *J* = 2.1 Hz, 2H), 5.06 (s, 2H), 4.88 - 4.81 (m, 1H), 3.80 (dd, *J* = 9.1, 6.2 Hz, 2H), 3.65 (dd, *J* = 9.1, 4.8 Hz, 2H), 3.61 (s, 3H). Mass spectrum (mass-to-charge ratio): C₂₇H₂₆N₃O₄⁺ [M+H]⁺; theoretical value: 456.19178; measured value: 456.19480.

### Example 22:

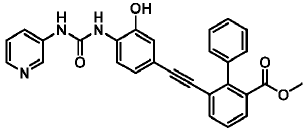

Compound 22 (30 mg, yield 43.05%) was synthesized according to general experimental operation 3. ¹H NMR (500 MHz, DMSO) δ 10.31 (s, 1H), 9.55 (s, 1H), 8.55 (s, 1H), 8.41 (s, 1H), 8.18 (d, J = 4.3 Hz, 1H), 8.03 (d, J = 8.4 Hz, 1H), 7.92 (d, J = 7.5 Hz, 1H), 7.78 (d, J = 7.8 Hz, 1H), 7.72 (d, J = 7.8 Hz, 1H), 7.51 (t, J = 7.8 Hz, 1H), 7.45 (dt, J = 14.2, 6.9 Hz, 3H), 7.30 (d, J = 6.7 Hz, 3H), 6.66 (d, J = 1.6 Hz, 1H), 6.62 (d, J = 8.4 Hz, 1H), 3.51 (s, 3H) ppm. Mass spectrum (mass-to-charge ratio): C₂₈H₂₁O₄N₃⁺ [M+H]⁺; theoretical value: 464.2; measured value: 464.2.

### Example 23:

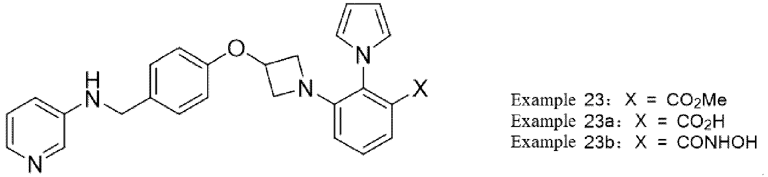

Compound 23 (39 mg, yield 31%) was synthesized according to the general experimental operation 1. ¹H NMR (400 MHz, MeOH-*d*₄) *δ* 7.93 - 7.85 (m, 1H), 7.76 - 7.67 (m, 1H), 7.36 - 7.23 (m, 3H), 7.13 - 6.94 (m, 3H), 6.82 - 6.66 (m, 5H), 6.20 (t, *J* = 2.1 Hz, 2H), 4.87 - 4.79 (m, 1H), 4.26 (s, 2H), 3.89 - 3.80 (m, 2H), 3.58 - 3.46 (m, 5H). Mass spectrum (mass-to-charge ratio): C₂₇H₂₇N₄O₃⁺ [M+H]⁺; theoretical value: 455.20777; measured value: 455.20776.

### Example 23a:

Compound 23a (4.4 mg, yield 80%) was synthesized according to general experimental operation 1. ¹H NMR (400 MHz, MeOH-*d*₄) *δ* 8.01 - 7.62 (m, 2H), 7.31 - 6.91 (m, 5H), 6.89 - 6.63 (m, 5H), 6.54 (dd, *J* = 8.2, 1.2 Hz, 1H), 6.12 (t, *J* = 2.1 Hz, 2H), 4.84 - 4.73 (m, 1H), 4.25 (s, 2H), 3.81 - 3.65 (m, 2H), 3.51 - 3.35 (m, 2H). Mass spectrum (mass-to-charge ratio): C₂₆H₂₅N₄O₃⁺ [M+H]⁺; theoretical value: 441.19212; measured value: 441.19196.

### Example 23b:

Compound 23b (3 mg, yield 27%) was synthesized according to the general experimental operation 1. ¹H NMR (400 MHz, CDCl₃) *δ* 8.12 - 7.85 (m, 2H), 7.43 - 7.30 (m, 2H), 7.27 - 7.17 (m, 2H), 7.12 - 7.00 (m, 1H), 6.93 - 6.80 (m, 1H), 6.80 - 6.57 (m, 5H), 6.44 - 6.31 (m, 2H), 4.88 - 4.77 (m, 1H), 4.27 (s, 2H), 3.85 - 3.74 (m, 2H), 3.70 - 3.61 (m, 2H). Mass spectrum (mass-to-charge ratio): C₂₆H₂₆N₅O₃⁺ [M+H]⁺; theoretical value: 456.20302; measured value: 456.20313.

### Example 24:

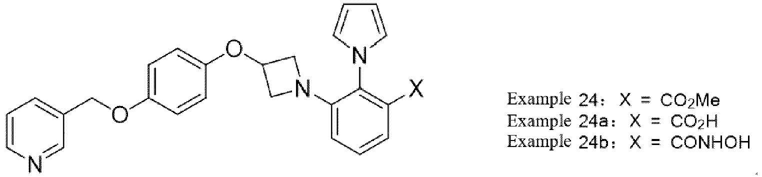

Compound 24 (40 mg, yield 39.85%) was synthesized according to the general experimental operation 1. ¹H NMR (500 MHz, CDCl₃) *δ* 8.66 (d, *J* = 1.8 Hz, 1H), 8.58 (dd, *J* = 4.8, 1.3 Hz, 1H), 7.78 - 7.72 (m, 1H), 7.34 - 7.27 (m, 2H), 7.15 (dd, *J* = 7.6, 1.1 Hz, 1H), 6.89 - 6.84 (m, 2H), 6.70 (t, *J* = 2.1 Hz, 2H), 6.69 - 6.66 (m, 1H), 6.65 - 6.62 (m, 2H), 6.25 (t, *J* = 2.1 Hz, 2H), 5.02 (s, 2H), 4.80 - 4.74 (m, 1H), 3.81 - 3.74 (m, 2H), 3.66 - 3.62 (m, 2H), 3.61 (s, 3H). Mass spectrum (mass-to-charge ratio): C₂₇H₂₆N₃O₄⁺ [M+H]⁺; theoretical value: 456.19178; measured value: 456.19189.

### Example 24a:

Compound 24a (6 mg, yield 51.59%) was synthesized according to general experimental operation 1. ¹H NMR (500 MHz, CDCl₃) *δ* 8.62 (s, 1H), 8.59 - 8.49 (m, 1H), 7.81 (d, *J* = 7.8 Hz, 1H), 7.35 (dd, *J* = 7.7, 5.0 Hz, 1H), 7.33 - 7.27 (m, 1H), 7.26 - 7.20 (m, 1H), 6.88 - 6.81 (m, 2H), 6.74 (s, 2H), 6.68 (d, *J* = 7.1 Hz, 1H), 6.66 - 6.60 (m, 2H), 6.26 - 6.20 (m, 2H), 5.01 (s, 2H), 4.79 - 4.73 (m, 1H), 3.81 - 3.71 (m, 2H), 3.66 - 3.59 (m, 2H). Mass spectrum (mass-to-charge ratio): C₂₆H₂₄N₃O₄⁺ [M+H]⁺; theoretical value: 442.17613; measured value: 442.17578.

### Example 24b:

Compound 24b (10 mg, yield 53.83%) was synthesized according to general experimental operation 1. ¹H NMR (500 MHz, MeOH-*d*₄/CDCl₃) *δ* 8.58 (d, *J* = 1.2 Hz, 1H), 8.47 (dd, *J* = 4.8, 1.2 Hz, 1H), 7.94 - 7.85 (m, 1H), 7.43 (dd, *J* = 7.8, 5.0 Hz, 1H), 7.29 (t, *J* = 7.9 Hz, 1H), 6.94 - 6.87 (m, 2H), 6.86 - 6.82 (m, 1H), 6.75 (t, *J* = 2.1 Hz, 2H), 6.69 (dd, *J* = 8.2, 0.9 Hz, 1H), 6.67 - 6.62 (m, 2H), 6.19 (t, *J* = 2.1 Hz, 2H), 5.06 (s, 2H), 4.79 - 4.75 (m, 1H), 3.82 - 3.74 (m, 2H), 3.60 - 3.50 (m, 2H). Mass spectrum (mass-to-charge ratio): C₂₆H₂₅N₄O₄⁺ [M+H]⁺; theoretical value: 457.18703; measured value: 457.18719.

### Example 25:

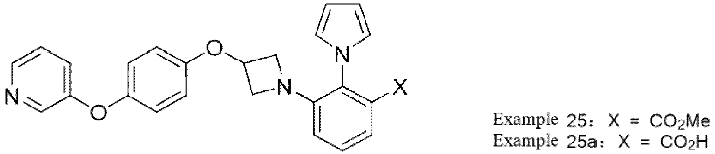

Compound 25 (8 mg, yield 52%) was synthesized according to the general experimental operation 1. ¹H NMR (400 MHz, CDCl₃) *δ* 8.39 - 8.36 (m, 1H), 8.36 - 8.33 (m, 1H), 7.32 (t, *J* = 7.9 Hz, 1H), 7.27 - 7.21 (m, 2H), 7.18 (dd, *J* = 7.7, 1.3 Hz, 1H), 7.00 - 6.96 (m, 2H), 6.77 - 6.68 (m, 5H), 6.28 (t, *J* = 2.1 Hz, 2H), 4.88 - 4.80 (m, 1H), 3.82 (dd, *J* = 9.0, 6.3 Hz, 2H), 3.68 (dd, *J* = 9.1, 4.8 Hz, 2H), 3.64 (s, 3H). Mass spectrum (mass-to-charge ratio): C₂₆H₂₄N₃O₄⁺ [M+H]⁺; theoretical value: 442.17613; measured value: 442.17535.

### Example 25a:

Compound 25a (6 mg, yield 51.64%) was synthesized according to general experimental operation 1. ¹H NMR (400 MHz, CDCl₃) *δ* 8.31 - 8.28 (m, 1H), 8.28 - 8.24 (m, 1H), 7.32 - 7.28 (m, 1H), 7.27 - 7.23 (m, 2H), 7.22 - 7.16 (m, 1H), 6.98 - 6.91 (m, 2H), 6.74 (t, *J* = 2.0 Hz, 2H), 6.71 - 6.65 (m, 3H), 6.23 (t, *J* = 2.0 Hz, 2H), 4.84 - 4.75 (m, 1H), 3.81 - 3.72 (m, 2H), 3.66 - 3.61 (m, 2H). Mass spectrum (mass-to-charge ratio): C₂₅H₂₂N₃O₄⁺ [M+H]⁺; theoretical value: 428.16048; measured value: 428.1606.

### Example 26:

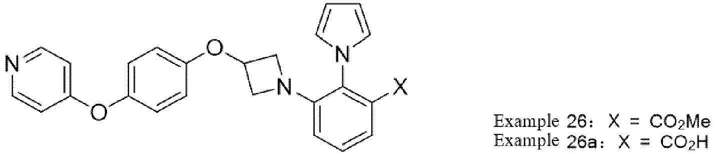

Compound 26 (7 mg, yield 48%) was synthesized according to the general experimental operation 1. ¹H NMR (400 MHz, CDCl₃) *δ* 8.50 - 8.45 (m, 2H), 7.33 (t, *J* = 7.9 Hz, 1H), 7.20 - 7.17 (m, 1H), 7.03 - 7.00 (m, 2H), 6.84 - 6.80 (m, 2H), 6.77 - 6.71 (m, 5H), 6.28 (t, *J* = 2.1 Hz, 2H), 4.89 - 4.81 (m, 1H), 3.87 - 3.81 (m, 2H), 3.72 - 3.67 (m, 2H), 3.64 (s, 3H). Mass spectrum (mass-to-charge ratio): C₂₆H₂₄N₃O₄⁺ [M+H]⁺; theoretical value: 442.17613; measured value: 442.17609.

### Example 26a:

Compound 26a (7 mg, yield 72.30%) was synthesized according to the general experimental operation 1. ¹H NMR (400 MHz, MeOH-*d*₄-CDCl₃) *δ* 8.33 (d, *J* = 6.2 Hz, 2H), 7.37 - 7.34 (m, 1H), 7.28 (t, *J* = 7.9 Hz, 1H), 7.16 (d, *J* = 7.6 Hz, 1H), 7.00 - 6.95 (m, 2H), 6.83 - 6.79 (m, 2H), 6.74 - 6.71 (m, 3H), 6.69 - 6.65 (m, 1H), 6.20 (t, *J* = 1.9 Hz, 2H), 4.87 - 4.75 (m, 1H), 3.81 - 3.71 (m, 2H), 3.65 - 3.58 (m, 2H). Mass spectrum (mass-to-charge ratio): C₂₅H₂₂N₃O₄⁺ [M+H]⁺; theoretical value: 428.16048; measured value: 428.16049.

### Example 27:

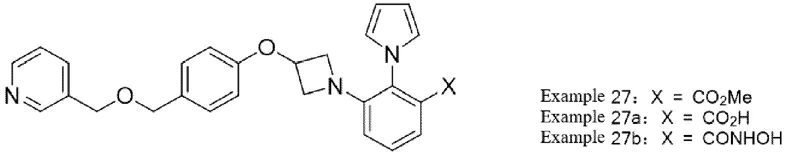

Compound 27 (11.4 mg, yield 18.3%) was synthesized according to the general experimental operation 1. ¹H NMR (400 MHz, CDCl₃) *δ* 8.71 - 8.37 (m, 2H), 7.73 (d, *J* = 7.8 Hz, 1H), 7.40 - 7.22 (m, 4H), 7.16 (d, *J* = 7.6 Hz, 1H), 6.77 - 6.60 (m, 5H), 6.26 (t, *J* = 2.2 Hz, 2H), 4.84 (q, *J* = 5.5 Hz, 1H), 4.55 (s, 2H), 4.50 (s, 2H), 3.81 (dd, *J* = 8.6, 6.2 Hz, 2H), 3.64 (d, *J* = 17.0 Hz, 5H). Mass spectrum (mass-to-charge ratio): C₂₈H₂₈N₃O₄⁺ [M+H]⁺; theoretical value: 470.20743; measured value: 470.20859.

### Example 27a:

Compound 27a (10.3 mg, yield 17.11%) was synthesized according to general experimental operation 1. ¹H NMR (400 MHz, CDCl₃) *δ* 8.64 - 8.44 (m, 2H), 7.78 (d, *J* = 8.0 Hz, 1H), 7.41 - 7.19 (m, 4H), 6.76 (t, *J* = 2.2 Hz, 2H), 6.72 - 6.64 (m, 3H), 6.25 (t, *J* = 2.2 Hz, 2H), 4.84 (p, *J* = 5.5 Hz, 1H), 4.54 (s, 2H), 4.50 (s, 2H), 3.79 (dd, *J* = 8.6, 6.2 Hz, 2H), 3.65 (dd, *J* = 8.7, 4.9 Hz, 2H). Mass spectrum (mass-to-charge ratio): C₂₇H₂₆N₃O₄⁺ [M+H]⁺; theoretical value: 456.19178; measured value: 456.19113.

### Example 27b:

Compound 27b (28 mg, yield 64.72%) was synthesized according to the general experimental operation 1. ¹H NMR (300 MHz, CDCl₃) *δ* 8.64 - 8.33 (m, 2H), 7.71 (d, *J* = 7.6 Hz, 1H), 7.27 (t, *J* = 9.7 Hz, 4H), 6.82 - 6.54 (m, 5H), 6.33 (s, 2H), 5.30 (s, 2H), 4.90 - 4.72 (m, 1H), 4.53 (s, 2H), 4.49 (s, 2H), 3.78 (q, *J* = 7.2, 6.7 Hz, 2H), 3.64 (dd, *J* = 8.5, 4.6 Hz, 2H). Mass spectrum (mass-to-charge ratio): C₂₇H₂₆N₄NaO₄⁺ [M+Na]⁺; theoretical value: 493.18463; measured value: 493.18463.

### Example 28:

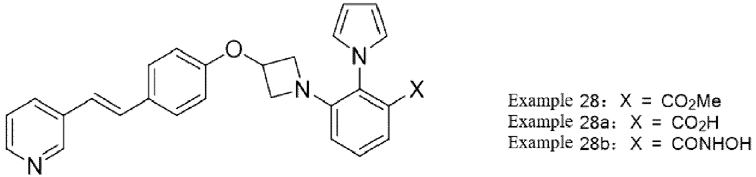

Compound 28 (12 mg, yield 56.78%) was synthesized according to general experimental operation 2. ¹H NMR (500 MHz, CDCl₃) *δ* 8.72 (d, *J* = 2.2 Hz, 1H), 8.49 (dd, *J* = 4.8, 1.6 Hz, 1H), 7.82 (dt, *J* = 8.0, 1.8 Hz, 1H), 7.48 - 7.43 (m, 2H), 7.35 - 7.29 (m, 2H), 7.18 (dd, *J* = 7.7, 1.4 Hz, 1H), 7.12 (d, *J* = 16.4 Hz, 1H), 6.95 (d, *J* = 16.4 Hz, 1H), 6.75 - 6.70 (m, 5H), 6.28 (t, *J* = 2.1 Hz, 2H), 4.93 - 4.85 (m, 1H), 3.84 (dd, *J* = 9.2, 6.2 Hz, 2H), 3.69 (dd, *J* = 9.2, 4.8 Hz, 2H), 3.64 (s, 3H). Mass spectrum (mass-to-charge ratio): C₂₈H₂₆N₃O₃⁺ [M+H]⁺; theoretical value: 452.19687; measured value: 452.19684.

### Example 28a:

Compound 28a (5 mg, yield 57.34%) was synthesized according to general experimental operation 2. ¹H NMR (300 MHz, CDCl₃) *δ* 8.61 (s, 1H), 8.42 (d, *J* = 4.1 Hz, 1H), 7.87 - 7.78 (m, 1H), 7.44 (d, *J* = 8.7 Hz, 2H), 7.35 - 7.27 (m, 2H), 7.26 - 7.20 (m, 1H), 7.11 (d, *J* = 16.4 Hz, 1H), 6.91 (d, *J* = 16.4 Hz, 1H), 6.79 - 6.74 (m, 2H), 6.73 - 6.64 (m, 3H), 6.32 - 6.21 (m, 2H), 4.90 - 4.81 (m, 1H), 3.86 - 3.73 (m, 2H), 3.71 - 3.60 (m, 2H). Mass spectrum (mass-to-charge ratio): C₂₇H₂₄N₃O₃⁺ [M+H]⁺; theoretical value: 438.18122; measured value: 438.18134.

### Example 28b:

Compound 28b (7 mg, yield 57.34%) was synthesized according to general experimental operation 2. ¹H NMR (500 MHz, MeOH-*d*₄/CDCl₃) *δ* 8.63 (d, *J* = 1.6 Hz, 1H), 8.36 (dd, *J* = 4.8, 1.1 Hz, 1H), 8.03 (dt, *J* = 8.0, 1.7 Hz, 1H), 7.53 - 7.47 (m, 2H), 7.41 (dd, *J* = 8.0, 4.9 Hz, 1H), 7.34 - 7.29 (m, 1H), 7.24 (d, *J* = 16.4 Hz, 1H), 7.04 (d, *J* = 16.4 Hz, 1H), 6.85 (dd, *J* = 7.5, 1.2 Hz, 1H), 6.77 - 6.70 (m, 5H), 6.20 (t, *J* = 2.1 Hz, 2H), 4.91 - 4.88 (m, 1H), 3.84 (dd, *J* = 8.9, 6.2 Hz, 2H), 3.54 (dd, *J* = 9.0, 4.3 Hz, 2H). Mass spectrum (mass-to-charge ratio): C₂₇H₂₅N₄O₃⁺ [M+H]⁺; theoretical value: 453.19212; measured value: 453.19217.

### Example 29:

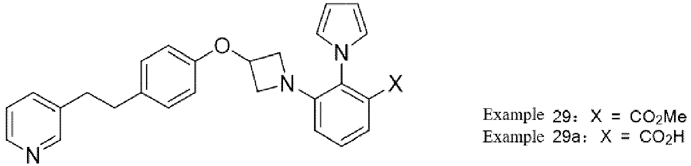

Compound 29 (2.5 mg, yield 49.78%) was synthesized according to general experimental operation 2. ¹H NMR (500 MHz, CDCl₃) *δ* 8.47 - 8.42 (m, 1H), 8.42 - 8.37 (m, 1H), 7.45 - 7.40 (m, 1H), 7.29 (t, *J* = 7.9 Hz, 1H), 7.20 (dd, *J* = 7.7, 4.8 Hz, 1H), 7.15 (dd, *J* = 7.6, 1.2 Hz, 1H), 7.04 - 6.98 (m, 2H), 6.71 (t, *J* = 2.1 Hz, 2H), 6.68 (dd, *J* = 8.2, 1.2 Hz, 1H), 6.60 (d, *J* = 8.6 Hz, 2H), 6.25 (t, *J* = 2.1 Hz, 2H), 4.84 - 4.76 (m, 1H), 3.79 (dd, *J* = 8.9, 6.3 Hz, 2H), 3.65 (dd, *J* = 9.0, 4.9 Hz, 2H), 3.61 (s, 3H), 2.91 - 2.81 (m, 4H). Mass spectrum (mass-to-charge ratio): C₂₈H₂₈N₃O₃⁺ [M+H]⁺; theoretical value: 454.21252; measured value: 454.21262.

### Example 29a:

Compound 28a (5.5 mg, yield 56.76%) was synthesized according to general experimental operation 2. ¹H NMR (500 MHz, MeOH-*d*₄) *δ* 8.30 (d, *J* = 4.0 Hz, 1H), 8.26 (s, 1H), 7.46 - 7.36 (m, 1H), 7.32 - 7.26 (m, 1H), 7.21 - 7.15 (m, 1H), 7.15 - 7.10 (m, 1H), 6.98 - 6.90 (m, 2H), 6.69 - 6.58 (m, 3H), 6.57 - 6.49 (m, 2H), 6.19 - 6.12 (m, 2H), 4.77 - 4.69 (m, 1H), 3.72 - 3.68 (m, 2H), 3.58 - 3.54 (m, 2H), 2.87 - 2.74 (m, 4H). Mass spectrum (mass-to-charge ratio): C₂₇H₂₆N₃O₃⁺ [M+H]⁺; theoretical value: 440.19687; measured value: 440.19690.

### Example 30:

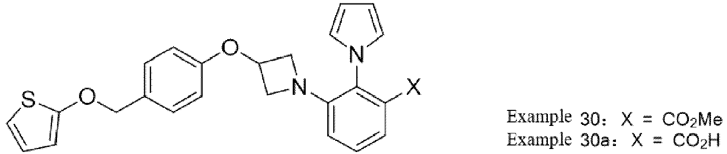

Compound 30 (7 mg, yield 6.37%) was synthesized according to the general experimental operation 1. ¹H NMR (500 MHz, CDCl₃) *δ* 7.33 - 7.27 (m, 3H), 7.15 (dd, *J* = 7.7, 1.3 Hz, 1H), 6.72 - 6.67 (m, 6H), 6.56 (dd, *J* = 5.8, 1.5 Hz, 1H), 6.27 - 6.24 (m, 3H), 4.98 (s, 2H), 4.88 - 4.81 (m, 1H), 3.82 - 3.79 (m, 2H), 3.67 - 3.63 (m, 2H), 3.62 (s, 3H). Mass spectrum (mass-to-charge ratio): C₂₆H₂₅N₂O₄S⁺ [M+H]⁺; theoretical value: 461.15295; measured value: 461.15305.

### Example 30a:

Compound 30a (6 mg, yield 61.89%) was synthesized according to general experimental operation 1. ¹H NMR (400 MHz, CDCl₃) *δ* 7.36 - 7.29 (m, 3H), 7.23 - 7.17 (m, 1H), 6.74 - 6.66 (m, 6H), 6.60 - 6.55 (m, 1H), 6.29 - 6.26 (m, 1H), 6.22 (s, 2H), 5.00 (s, 2H), 4.89 - 4.80 (m, 1H), 3.82 - 3.73 (m, 2H), 3.69 - 3.60 (m, 2H). Mass spectrum (mass-to-charge ratio): C₂₅H₂₃N₂O₄S⁺ [M+H]⁺; theoretical value: 447.13730; measured value: 447.13751.

### Example 31:

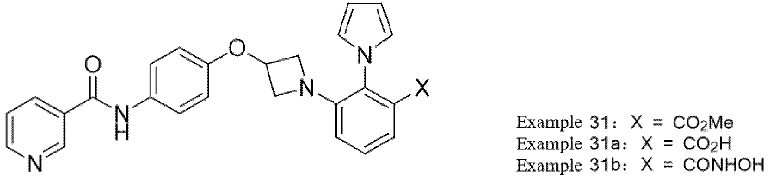

Compound 31 (103 mg, yield 79.90%) was synthesized according to general experimental operation 2. ¹H NMR (400 MHz, CDCl₃) *δ* 9.07 (s, 1H), 8.75 (d, *J* = 4.8 Hz, 1H), 8.19 (d, *J* = 7.9 Hz, 1H), 8.09 (s, 1H), 7.51 (d, *J* = 8.4 Hz, 2H), 7.42 (dd, *J* = 8.0, 4.9 Hz, 1H), 7.29 (dd, *J* = 14.2, 6.2 Hz, 1H), 7.15 (d, *J* = 7.6 Hz, 1H), 6.70 (d, *J* = 8.8 Hz, 5H), 6.25 (t, *J* = 2.2 Hz, 2H), 4.83 (p, *J* = 5.5 Hz, 1H), 3.81 (t, *J* = 7.4 Hz, 2H), 3.64 (d, *J* = 17.3 Hz, 5H). Mass spectrum (mass-to-charge ratio): C₂₇H₂₄N₄NaO₄⁺ [M+Na]⁺; theoretical value: 491.16898; measured value: 491.16910.

### Example 31a:

Compound 31a (81 mg, yield 92.78%) was synthesized according to general experimental operation 2. ¹H NMR (400 MHz, DMSO-*d*₆) *δ* 10.35 (s, 1H), 9.08 (s, 1H), 8.74 (d, *J* = 4.8 Hz, 1H), 8.27 (d, *J* = 8.0 Hz, 1H), 7.64 (d, *J* = 8.4 Hz, 2H), 7.55 (d, *J* = 6.9 Hz, 1H), 7.27 (d, *J* = 7.9 Hz, 1H), 6.93 (d, *J* = 7.4 Hz, 1H), 6.85 - 6.60 (m, 5H), 6.11 (s, 2H), 4.89 (s, 1H), 3.78 (t, *J* = 7.4 Hz, 2H), 3.42 (s, 2H). Mass spectrum (mass-to-charge ratio): C₂₆H₂₃N₄O₄⁺ [M+H]⁺ ; theoretical value 455.17138; measured value: 455.17120.

### Example 31b:

Compound 31b (3.4 mg, yield 17.69%) was synthesized according to general experimental operation 2. ¹H NMR (400 MHz, MeOH-*d*₄) *δ* 9.07 (d, *J* = 2.2 Hz, 1H), 8.72 (dd, *J* = 5.0, 1.6 Hz, 1H), 7.59 (dd, *J* = 8.0, 5.5 Hz, 3H), 7.40 - 7.25 (m, 1H), 6.85 (dd, *J* = 7.5, 1.3 Hz, 1H), 6.81 - 6.68 (m, 5H), 6.22 (t, *J* = 2.1 Hz, 2H), 3.85 (dd, *J* = 8.8, 6.1 Hz, 2H), 3.54 (dd, *J* = 8.9, 4.4 Hz, 2H). Mass spectrum (mass-to-charge ratio): C₂₆H₂₃N₅NaO₄⁺ [M+Na]⁺; theoretical value: 492.16423; measured value: 492.16479.

### Example 32:

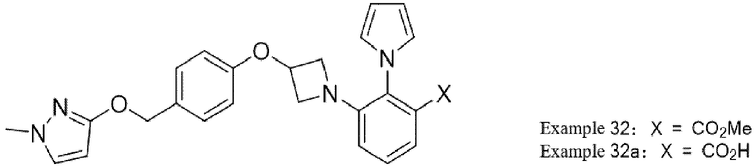

Compound 32 (11 mg, yield 23.99%) was synthesized according to the general experimental operation 1. ¹H NMR (400 MHz, CDCl₃) *δ* 7.39 - 7.35 (m, 2H), 7.34 - 7.29 (m, 1H), 7.19 - 7.13 (m, 2H), 6.75 - 6.66 (m, 5H), 6.27 (t, *J* = 2.1 Hz, 2H), 5.65 (d, *J* = 2.3 Hz, 1H), 5.11 (s, 2H), 4.90 - 4.82 (m, 1H), 3.82 (dd, *J* = 9.1, 6.2 Hz, 2H), 3.78 (s, 3H), 3.67 (dd, *J* = 9.2, 4.9 Hz, 2H), 3.64 (s, 3H). Mass spectrum (mass-to-charge ratio): C₂₆H₂₇N₄O₄⁺ [M+H]⁺; theoretical value: 459.20268; measured value: 459.20270.

### Example 32a:

Compound 32a (6 mg, yield 61.89%) was synthesized according to the general experimental operation 1. ¹H NMR (500 MHz, CDCl₃) *δ* 7.35 - 7.27 (m, 3H), 7.26 - 7.21 (m, 1H), 7.12 (d, *J* = 2.2 Hz, 1H), 6.73 - 6.65 (m, 5H), 6.22 (t, *J* = 2.0 Hz, 2H), 5.62 (d, *J* = 2.3 Hz, 1H), 5.07 (s, 2H), 4.87 - 4.78 (m, 1H), 3.80 - 3.75 (m, 2H), 3.74 (s, 3H), 3.65 - 3.61 (m, 2H). Mass spectrum (mass-to-charge ratio): C₂₅H₂₅N₄O₄⁺ [M+H]⁺; theoretical value: 445.18703; measured value: 445.18728.

### Example 33:

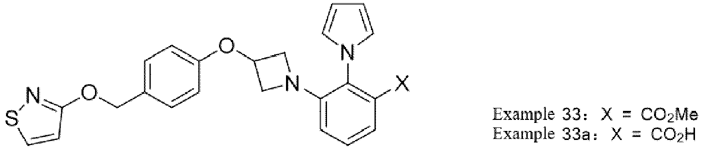

Compound 33 (23 mg, yield 27.14%) was synthesized according to the general experimental operation 1. ¹H NMR (500 MHz, CDCl₃): *δ* 8.44 (d, *J* = 4.7 Hz, 1H), 7.38 - 7.34 (m, 2H), 7.29 (t, *J* = 7.9 Hz, 1H), 7.15 (dd, *J* = 7.6, 1.3 Hz, 1H), 6.72 - 6.67 (m, 5H), 6.61 (d, *J* = 4.7 Hz, 1H), 6.25 (t, *J* = 2.1 Hz, 2H), 5.33 (s, 2H), 4.87 - 4.81 (m, 1H), 3.83 - 3.78 (m, 2H), 3.67 - 3.64 (m, 2H), 3.61 (s, 3H). Mass spectrum (mass-to-charge ratio): C₂₅H₂₄N₃O₄S⁺ [M+H]⁺; theoretical value: 462.14820; measured value: 462.14835.

### Example 33a:

Compound 33a (10 mg, yield 68.76%) was synthesized according to the general experimental operation 1. ¹H NMR (500 MHz, CDCl₃/MeOH-*d*₄) *δ* 8.46 - 8.37 (m, 1H), 7.35 - 7.29 (m, 2H), 7.25 - 7.19 (m, 1H), 7.16 - 7.05 (m, 1H), 6.74 - 6.63 (m, 4H), 6.63 - 6.52 (m, 2H), 6.23 - 6.12 (m, 2H), 5.26 (s, 2H), 4.83 - 4.73 (m, 1H), 3.77 - 3.67 (m, 2H), 3.62 - 3.54 (m, 2H). Mass spectrum (mass-to-charge ratio): C₂₄H₂₂N₃O₄S⁺ [M+H]⁺; theoretical value: 448.13255; measured value: 448.13272.

### Example 34:

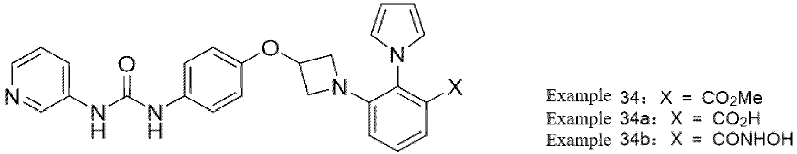

Compound 34 (40 mg, yield 75.16%) was synthesized according to general experimental operation 2. ¹H NMR (400 MHz, CDCl₃) *δ* 8.39 (s, 1H), 8.30 (d, *J* = 4.8 Hz, 1H), 8.06 (d, *J* = 8.4 Hz, 1H), 7.27 (s, 14H), 7.17 (dd, *J* = 7.7, 1.4 Hz, 1H), 6.88 (s, 1H), 6.77 - 6.64 (m, 8H), 6.26 (t, *J* = 2.1 Hz, 2H), 4.82 (t, *J* = 5.5 Hz, 1H), 3.86 - 3.78 (m, 2H), 3.68 - 3.59 (m, 6H). Mass spectrum (mass-to-charge ratio): C₂₇H₂₆N₅O₄⁺ [M+H]⁺; theoretical value: 484.19793; measured value: 484.19885.

### Example 34a:

Compound 34a (25 mg, yield 85.82%) was synthesized according to general experimental operation 2. ¹H NMR (500 MHz, MeOH-*d*₄) *δ* 10.15 (d, *J* = 2.6 Hz, 1H), 9.72 (dd, *J* = 4.8, 1.5 Hz, 1H), 9.55 (ddd, *J* = 8.4, 2.7, 1.4 Hz, 1H), 8.96 - 8.89 (m, 1H), 8.88 - 8.78 (m, 3H), 8.52 (dd, *J* = 7.6, 1.3 Hz, 1H), 8.30 (t, *J* = 2.1 Hz, 2H), 8.28 - 8.20 (m, 3H), 7.71 (t, *J* = 2.1 Hz, 2H), 6.39 - 6.27 (m, 1H), 5.42 - 5.26 (m, 2H), 5.05 (dd, *J=* 8.9, 4.4 Hz, 2H). Mass spectrum (mass-to-charge ratio): C₂₆H₂₄N₅O₄⁺ [M+H]⁺; theoretical value: 470.18228; measured value: 470.18253.

### Example 34b:

Compound 34b (20 mg, yield 76%) was synthesized according to general experimental operation 2. ¹H NMR (400 MHz, MeOH-*d*₄) *δ* 8.60 (d, *J* = 2.4 Hz, 1H), 8.16 (dd, *J* = 4.8, 1.3 Hz, 1H), 8.00 (d, *J* = 8.4 Hz, 1H), 7.48 - 7.22 (m, 4H), 6.87 (d, *J* = 7.5 Hz, 1H), 6.77 (t, *J* = 2.1 Hz, 2H), 6.76 - 6.59 (m, 3H), 6.18 (t, *J* = 2.2 Hz, 2H), 4.84 - 4.78 (m, 1H), 3.80 (dd, *J* = 8.7, 6.1 Hz, 2H), 3.50 (dd, *J* = 8.9, 4.4 Hz, 2H). Mass spectrum (mass-to-charge ratio): C₂₆H₂₅N₆O₄⁺ [M+H]⁺; theoretical value: 485.2; measured value: 485.2.

### Example 35:

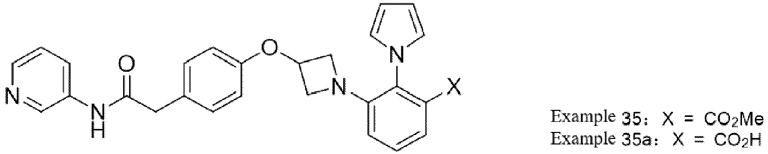

Compound 35 (25 mg, yield 18%) was synthesized according to general experimental operation 2. ¹H NMR (400 MHz, CDCl₃) *δ* 7.42 (d, *J* = 7.8 Hz, 2H), 7.35 - 7.03 (m, 7H), 6.77 - 6.60 (m, 5H), 6.26 (t, *J* = 2.1 Hz, 2H), 4.90 - 4.79 (m, 1H), 3.87 - 3.75 (m, 2H), 3.71 - 3.64 (m, 4H), 3.62 (s, 3H). Mass spectrum (mass-to-charge ratio): C₂₉H₂₈N₃O₄⁺ [M+H]⁺; theoretical value: 482.20; measured value: 482.20.

### Example 35a:

Compound 35a (3.5 mg, yield 25%) was synthesized according to general experimental operation 2. ¹H NMR (400 MHz, CDCl₃) *δ* 7.41 (d, *J* = 7.9 Hz, 2H), 7.35 - 7.04 (m, 7H), 6.81 - 6.61 (m, 5H), 6.30 - 6.17 (m, 2H), 4.87 - 4.78 (m, 1H), 3.84 - 3.74 (m, 2H), 3.70 - 3.60 (m, 4H). Mass spectrum (mass-to-charge ratio): C₂₈H₂₆N₃O₄⁺ [M+H]⁺; theoretical value: 468.19; measured value: 468.20.

### Example 36:

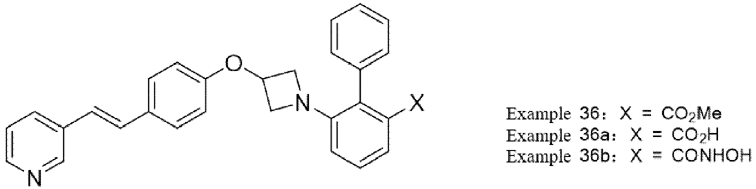

Compound 36 (28 mg, yield 66%) was synthesized according to general experimental operation 2. ¹H NMR (500 MHz, CDCl₃) *δ* 8.84 - 8.28 (m, 2H), 7.79 (d, *J* = 8.0 Hz, 1H), 7.44 - 7.23 (m, 9H), 7.22 - 7.16 (m, 1H), 7.07 (d, *J* = 16.4 Hz, 1H), 6.91 (d, *J* = 16.4 Hz, 1H), 6.72 - 6.60 (m, 3H), 4.80 - 4.69 (m, 1H), 3.75 - 3.67 (m, 2H), 3.55 - 3.46 (m, 5H). Mass spectrum (mass-to-charge ratio): C₃₀H₂₇N₂O₃⁺ [M+H]⁺; theoretical value: 463.20; measured value: 463.20.

### Example 36a:

Compound 36a (24 mg, yield 90%) was synthesized according to general experimental operation 2. ¹H NMR (400 MHz, MeOH-*d*₄) *δ* 8.89 - 8.40 (m, 2H), 8.40 - 8.28 (m, 1H), 7.74 - 7.61 (m, 1H), 7.58 - 7.48 (m, 2H), 7.46 - 7.25 (m, 7H), 7.12 (t, *J* = 12.2 Hz, 2H), 6.75 (t, *J* = 9.1 Hz, 3H), 4.82 - 4.76 (m, 1H), 3.81 - 3.71 (m, 2H), 3.45 - 3.36 (m, 2H). Mass spectrum (mass-to-charge ratio): C₂₉H₂₅N₂O₃⁺ [M+H]⁺; theoretical value: 449.18597; measured value: 449.20.

### Example 36b:

Compound 36b (10 mg, yield 59%) was synthesized according to general experimental operation 2. ¹H NMR (400 MHz, MeOH-*d*₄) *δ* 8.62 (s, 1H), 8.36 (d, *J* = 4.0 Hz, 1H), 8.02 (d, *J* = 7.9 Hz, 1H), 7.48 (d, *J* = 8.5 Hz, 2H), 7.43 - 7.17 (m, 8H), 7.03 (d, *J* = 16.4 Hz, 1H), 6.87 (d, *J* = 7.3 Hz, 1H), 6.76 - 6.64 (m, 3H), 4.83 - 4.73 (m, 1H), 3.79 - 3.68 (m, 2H), 3.43 - 3.36 (m, 2H). Mass spectrum (mass-to-charge ratio): C₂₉H₂₆N₃O₃⁺ [M+H]⁺; theoretical value: 464.19687; measured value: 464.20.

### Example 37:

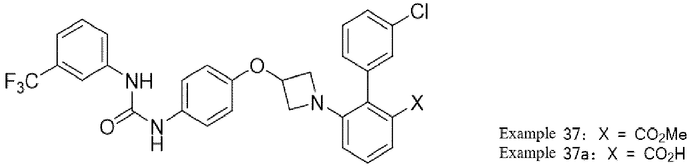

Compound 37 (27 mg, yield 56%) was synthesized according to general experimental operation 2. ¹H NMR (400 MHz, CDCl₃) *δ* 7.56 (d, *J* = 10.6 Hz, 2H), 7.37 (t, *J* = 7.8 Hz, 1H), 7.34-7.27 (m, 5H), 7.23 (dd, *J* = 7.7, 1.2 Hz, 1H), 7.21-7.15 (m, 3H), 6.84 (s, 1H), 6.70-6.62 (m, 3H), 6.58 (s, 1H), 4.73 (t, *J* = 5.6 Hz, 1H), 3.79-3.66 (m, 2H), 3.58-3.46 (m, 5H). Mass spectrum (mass-to-charge ratio): C₃₁H₂₄ClF₃N₃O₄⁻[M - H]⁻; theoretical: 594.1; measured value: 594.2.

### Example 37a:

Compound 37a (2.3 mg, yield 34%) was synthesized according to general experimental operation 2. ¹H NMR (500 MHz, MeOH-*d*₄) *δ* 7.88 (d, *J* = 2.2 Hz, 1H), 7.58 (dd, *J* = 8.1, 2.0 Hz, 1H), 7.45 (t, *J* = 7.9 Hz, 1H), 7.35-7.24 (m, 8H), 7.12 (d, *J* = 7.5 Hz, 1H), 6.74 (d, *J* = 8.6 Hz, 1H), 6.72-6.67 (m, 2H), 4.80-4.76 (m, 1H), 3.83-3.70 (m, 2H), 3.42 (dd, *J* = 14.5, 8.8 Hz, 2H). Mass spectrum (mass-to-charge ratio): C₃₀H₂₂ClF₃N₃O₄⁻[M-H]⁻; theoretical: 580.1; measured value: 580.2.

### Example 38:

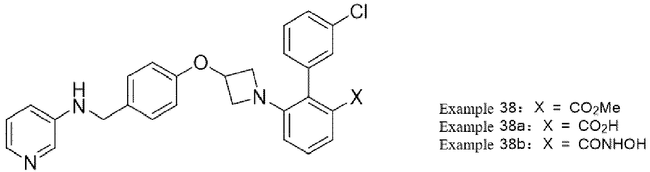

Compound 38 (23 mg, yield 55%) was synthesized according to general experimental operation 2. ¹H NMR (400 MHz, CDCl₃) *δ* 8.14 - 7.89 (m, 2H), 7.37 - 7.14 (m, 8H), 7.12 - 7.03 (m, 1H), 6.90 - 6.82 (m, 1H), 6.71 - 6.61 (m, 3H), 4.81 - 4.70 (m, 1H), 4.25 (s, 2H), 3.83 - 3.63 (m, 2H), 3.62 - 3.45 (m, 5H). Mass spectrum (mass-to-charge ratio): C₂₉H₂₇ClN₃O₃⁺ [M+H]⁺; theoretical value: 500.2; measured value: 500.2.

### Example 38a:

Compound 38a (16 mg, yield 96%) was synthesized according to general experimental operation 2. ¹H NMR (400 MHz, MeOH-*d*₄) *δ* 7.95 - 7.87 (m, 1H), 7.87 - 7.79 (m, 1H), 7.49 - 7.12 (m, 10H), 6.81 - 6.66 (m, 3H), 4.83 - 4.75 (m, 1H), 4.31 (s, 2H), 3.84 - 3.69 (m, 2H), 3.46 - 3.35 (m, 2H). Mass spectrum (mass-to-charge ratio): C₂₈H₂₅ClN₃O₃⁺ [M+H]⁺; theoretical value: 486.2; measured value: 486.1.

### Example 38b:

Compound 38b (7.3 mg, yield 85%) was synthesized according to general experimental operation 2. ¹H NMR (400 MHz, MeOH-*d*₄) *δ* 7.96 - 7.79 (m, 1H), 7.79 - 7.61 (m, 1H), 7.43 - 7.18 (m, 7H), 7.14 - 7.02 (m, 1H), 7.02 - 6.91 (m, 1H), 6.91 - 6.82 (m, 1H), 6.76 - 6.61 (m, 3H), 4.82 - 4.70 (m, 1H), 4.25 (s, 2H), 3.88 - 3.65 (m, 2H), 3.48 - 3.27 (m, 2H). Mass spectrum (mass-to-charge ratio): C₂₈H₂₆ClN₄O₃⁺ [M+H]⁺; theoretical value: 501.2; measured value: 501.1.

### Example 39:

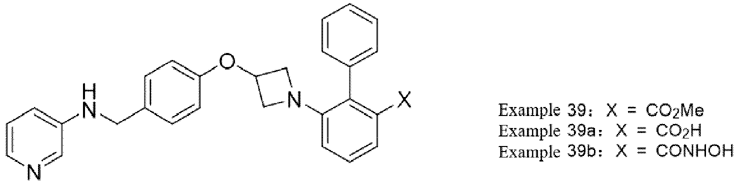

Compound 39 (23 mg, yield 63%) was synthesized according to general experimental operation 2. ¹H NMR (400 MHz, CDCl₃) *δ* 8.08 - 8.01 (m, 1H), 8.01 - 7.92 (m, 1H), 7.41 - 7.15 (m, 11H), 7.10 - 7.02 (m, 1H), 6.90 - 6.81 (m, 1H), 6.71 - 6.58 (m, 2H), 4.77 - 4.68 (m, 1H), 4.25 (s, 2H), 3.73 - 3.66 (m, 2H), 3.55 - 3.42 (m, 5H). Mass spectrum (mass-to-charge ratio): C₂₉H₂₈N₃O₃⁺ [M+H]⁺; theoretical value: 466.2; measured value: 466.2.

### Example 39a:

Compound 39a (1.6 mg, yield 32%) was synthesized according to general experimental operation 2. ¹H NMR (400 MHz, MeOH-*d*₄) *δ* 7.92 - 7.81 (m, 1H), 7.79 - 7.68 (m, 1H), 7.38 - 7.14 (m, 9H), 7.12 - 7.01 (m, 2H), 6.73 - 6.60 (m, 3H), 4.76 - 4.66 (m, 1H), 4.25 (s, 2H), 3.73 - 3.66 (m, 2H), 3.38 - 3.33 (m, 2H). Mass spectrum (mass-to-charge ratio): C₂₈H₂₆N₃O₃⁺ [M+H]⁺; theoretical value: 452.2; measured value: 452.2.

### Example 39b:

Compound 39b (2.2 mg, yield 37%) was synthesized according to general experimental operation 2. ¹H NMR (400 MHz, MeOH-*d*₄) *δ* 7.92 - 7.82 (m, 1H), 7.77 - 7.66 (m, 1H), 7.41 - 7.18 (m, 8H), 7.16 - 7.09 (m, 1H), 7.03 - 6.95 (m, 1H), 6.89 - 6.82 (m, 1H), 6.74 - 6.60 (m, 3H), 4.77 - 4.70 (m, 1H), 4.25 (s, 2H), 3.76 - 3.67 (m, 2H), 3.40 - 3.34 (m, 2H). Mass spectrum (mass-to-charge ratio): C₂₈H₂₇N₄O₃⁺ [M+H]⁺; theoretical value: 467.2; measured value: 467.2.

### Example 40:

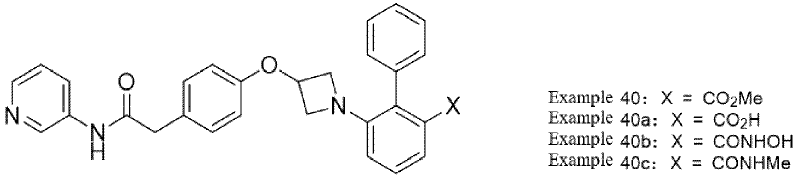

Compound 40 (65 mg, yield 59%) was synthesized according to general experimental operation 2. ¹H NMR (400 MHz, CDCl₃) *δ* 8.46 - 8.37 (m, 1H), 8.37 - 8.29 (m, 1H), 8.17 - 8.06 (m, 1H), 7.44 - 7.06 (m, 10H), 6.75 - 6.62 (m, 3H), 4.80 - 4.68 (m, 1H), 3.77 - 3.61 (m, 4H), 3.55 - 3.45 (m, 5H). Mass spectrum (mass-to-charge ratio): C₃₀H₂₈N₃O₄⁺ [M+H]⁺; theoretical value: 494.2; measured value: 494.2.

### Example 40a:

Compound 40a (5 mg, yield 51%) was synthesized according to general experimental operation 2. ¹H NMR (500 MHz, MeOH-*d*₄) *δ* 8.76 - 8.63 (m, 1H), 8.32 - 8.15 (m, 1H), 8.14 - 8.02 (m, 1H), 7.44 - 7.18 (m, 9H), 7.15 - 7.07 (m, 1H), 6.76 - 6.62 (m, 3H), 4.77 - 4.70 (m, 1H), 3.76 - 3.65 (m, 2H), 3.62 (s, 2H), 3.39 - 3.33 (m, 2H). Mass spectrum (mass-to-charge ratio): C₂₉H₂₆N₃O₄⁺ [M+H]⁺; theoretical value: 480.2; measured value: 480.2.

### Example 40b:

Compound 40b (3.2 mg, yield 46%) was synthesized according to general experimental operation 2. ¹H NMR (500 MHz, MeOH-*d*₄) *δ* 8.75 - 8.63 (m, 1H), 8.27 - 8.17 (m, 1H), 8.13 - 8.03 (m, 1H), 7.43 - 7.15 (m, 9H), 6.88 - 6.79 (m, 1H), 6.72 - 6.59 (m, 3H), 4.79 - 4.69 (m, 1H), 3.75 - 3.66 (m, 2H), 3.59 (s, 2H), 3.40 - 3.31 (m, 2H). Mass spectrum (mass-to-charge ratio): C₂₉H₂₇N₄O₄⁺ [M+H]⁺; theoretical value: 495.2; measured value: 495.2.

### Example 40c:

Compound 40c (7.3 mg, yield 44%) was obtained. ¹H NMR (400 MHz, CDCl₃) *δ* 8.49 - 8.41 (m, 1H), 8.30 (d, *J* = 4.0 Hz, 1H), 8.16 (d, *J* = 8.3 Hz, 1H), 7.93 - 7.84 (m, 1H), 7.42 - 7.21 (m, 6H), 7.16 - 7.08 (m, 2H), 7.08 - 7.01 (m, 1H), 6.71 - 6.55 (m, 3H), 5.17 - 5.06 (m, 1H), 4.79 - 4.67 (m, 1H), 3.76 - 3.67 (m, 2H), 3.62 (s, 2H), 3.51 - 3.44 (m, 2H), 2.57 (d, *J* = 4.9 Hz, 3H). Mass spectrum (mass-to-charge ratio): C₃₀H₂₉N₄O₃⁺ [M+H]⁺ ; theoretical value: 493.2; measured value: 493.1.

### Example 41:

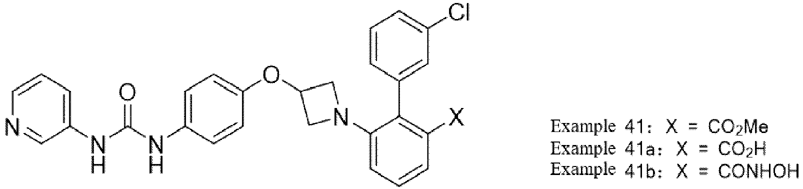

Compound 41 (50 mg, yield 48.31%) was synthesized according to general experimental operation 2. ¹H NMR (400 MHz, CDCl₃) *δ* 8.38 (d, *J* = 2.4 Hz, 1H), 8.28 (dd, *J* = 4.8, 1.3 Hz, 1H), 8.15 - 8.04 (m, 1H), 7.36 - 7.30 (m, 4H), 7.27 - 7.19 (m, 4H), 6.89 - 6.81 (m, 1H), 6.73 - 6.62 (m, 3H), 4.78 - 4.71 (m, 1H), 3.83 - 3.75 (m, 1H), 3.74 - 3.65 (m, 1H), 3.59 (s, 3H), 3.58 - 3.53 (m, 1H), 3.52 - 3.44 (m, 1H). Mass spectrum (mass-to-charge ratio): C₂₉H₂₆ClN₄O₄⁺ [M+H]⁺ ; theoretical value: 529.16371; measured value: 529.16412.

### Example 41a:

Compound 41a (3 mg, yield 30.82%) was synthesized according to general experimental operation 2. ¹H NMR (400 MHz, MeOH-*d*₄) *δ* 8.64 - 8.58 (m, 1H), 8.19 (d, *J=* 3.6 Hz, 1H), 8.04 - 7.98 (m, 1H), 7.40 - 7.30 (m, 7H), 7.29 - 7.24 (m, 1H), 7.17 - 7.13 (m, 1H), 6.79 - 6.75 (m, 1H), 6.74 - 6.69 (m, 2H), 4.82 - 4.79 (m, 1H), 3.83 - 3.72 (m, 2H), 3.48 - 3.41 (m, 2H). Mass spectrum (mass-to-charge ratio): C₂₈H₂₄ClN₄O₄⁺ [M+H]⁺ ; theoretical value: 515.14806; measured value: 515.14850.

### Example 41b:

Compound 41b (11 mg, yield 42.49%) was synthesized according to general experimental operation 2. ¹H NMR (500 MHz, MeOH-*d*₄) *δ* 8.58 (d, *J* = 2.4 Hz, 1H), 8.16 (dd, *J* = 4.8, 1.1 Hz, 1H), 8.02 - 7.95 (m, 1H), 7.40 - 7.26 (m, 8H), 6.89 - 6.84 (m, 1H), 6.76 - 6.71 (m, 1H), 6.71 - 6.66 (m, 2H), 4.82 - 4.76 (m, 1H), 3.86 - 3.71 (m, 2H), 3.49 - 3.36 (m, 2H). Mass spectrum (mass-to-charge ratio): C₂₈H₂₅ClN₅O₄⁺ [M+H]⁺; theoretical value: 530.15896; measured value: 530.15936.

### Example 42:

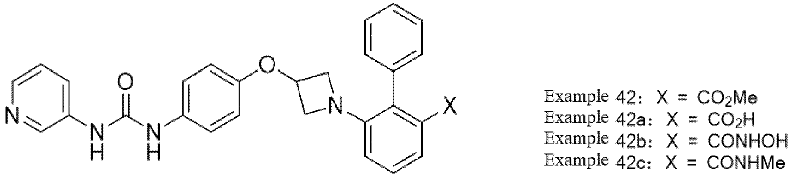

Compound 42 (50 mg, yield 47.32%) was synthesized according to general experimental operation 2. ¹H NMR (500 MHz, MeOH-*d*₄) *δ* 8.85 - 8.76 (m, 1H), 8.42 - 8.33 (m, 1H), 8.25 - 8.16 (m, 1H), 7.66 - 7.41 (m, 9H), 7.35 - 7.23 (m, 1H), 7.03 - 6.93 (m, 1H), 6.92 - 6.84 (m, 2H), 5.00 - 4.91 (m, 1H), 3.99 - 3.91 (m, 2H), 3.68 (s, 3H), 3.64 - 3.57 (m, 2H). Mass spectrum (mass-to-charge ratio): C₂₉H₂₇N₄O₄⁺ [M+H]⁺; theoretical value: 495.20268; measured value: 495.20291.

### Example 42a:

Compound 42a (4.2 mg, yield 36.02%) was synthesized according to general experimental operation 2. ¹H NMR (500 MHz, MeOH-*d*₄) *δ* 8.59 (d, *J* = 2.2 Hz, 1H), 8.17 (dd, *J* = 4.8, 1.4 Hz, 1H), 8.01 - 7.97 (m, 1H), 7.38 - 7.31 (m, 4H), 7.31 - 7.26 (m, 5H), 7.11 (dd, *J* = 7.6, 1.0 Hz, 1H), 6.75 - 6.71 (m, 1H), 6.69 - 6.64 (m, 2H), 4.76 - 4.71 (m, 1H), 3.73 - 3.69 (m, 2H), 3.39 - 3.36 (m, 2H). Mass spectrum (mass-to-charge ratio): C₂₈H₂₅N₄O₄⁺ [M+H]⁺; theoretical value: 481.18703; measured value: 481.18729.

### Example 42b:

Compound 42b (7.5 mg, yield 35.09%) was synthesized according to general experimental operation 2. ¹H NMR (400 MHz, MeOH-*d*₄) *δ* 8.61 (d, *J* = 2.0 Hz, 1H), 8.22 - 8.16 (m, 1H), 8.04 - 7.96 (m, 1H), 7.41 - 7.34 (m, 6H), 7.33 - 7.28 (m, 3H), 6.88 (dd, *J* = 7.5, 0.9 Hz, 1H), 6.75 - 6.71 (m, 1H), 6.71 - 6.66 (m, 2H), 4.81 - 4.73 (m, 1H), 3.79 - 3.71 (m, 2H), 3.42 - 3.38 (m, 2H). Mass spectrum (mass-to-charge ratio): C₂₈H₂₆N₅O₄⁺ [M+H]⁺; theoretical value: 496.19793; measured value: 496.19809.

### Example 42c:

Compound 42c (20 mg, yield 64.91%) was synthesized according to general experimental operation 2. ¹H NMR (500 MHz, CDCl₃): *δ* 8.54 (s, 1H), 8.44 (d, *J* = 2.4 Hz, 1H), 8.19 (dd, *J* = 4.7, 1.1 Hz, 1H), 8.15 - 8.09 (m, 1H), 7.72 (s, 1H), 7.36 - 7.18 (m, 7H), 7.02 (dd, *J* = 7.5, 0.9 Hz, 1H), 6.78 - 6.72 (m, 2H), 6.61 (dd, *J* = 8.3, 0.8 Hz, 1H), 6.46 - 6.39 (m, 2H), 5.42 (q, *J* = 4.8 Hz, 1H), 4.67 - 4.60 (m, 1H), 3.66 - 3.59 (m, 2H), 3.38 - 3.32 (m, 2H), 2.59 (d, *J* = 4.9 Hz, 3H). Mass spectrum (mass-to-charge ratio): C₂₉H₂₈N₅O₃⁺ [M+H]⁺; theoretical value: 494.21867; measured value: 494.21881.

### Example 43:

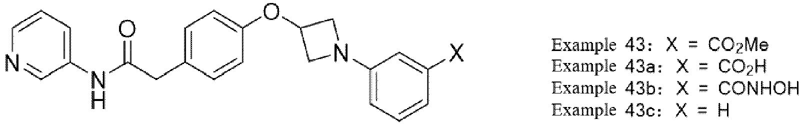

Compound 43 (60 mg, yield 56%) was synthesized according to general experimental operation 2. ¹H NMR (500 MHz, CDCl₃) *δ* 8.49 - 8.39 (m, 1H), 8.36 - 8.29 (m, 1H), 8.18 - 7.98 (m, 1H), 7.49 - 7.40 (m, 1H), 7.39 - 7.20 (m, 6H), 7.17 - 7.11 (m, 1H), 6.84 (d, *J* = 8.6 Hz, 2H), 6.70 - 6.62 (m, 1H), 5.15 - 5.05 (m, 1H), 4.44 - 4.32 (m, 2H), 3.99 - 3.91 (m, 2H), 3.90 (s, 3H), 3.72 (s, 2H). Mass spectrum (mass-to-charge ratio): C₂₄H₂₄N₃O₄⁺ [M+H]⁺; theoretical value: 418.2; measured value: 418.1.

### Example 43a:

Compound 43a (30 mg, yield 68%) was synthesized according to general experimental operation 2. ¹H NMR (400 MHz, MeOH-*d*₄) *δ* 8.78 - 8.67 (m, 1H), 8.30 - 8.20 (m, 1H), 8.16 - 8.07 (m, 1H), 7.45 - 7.22 (m, 5H), 7.17 - 7.08 (m, 1H), 6.91 - 6.80 (m, 2H), 6.79 - 6.69 (m, 1H), 5.19 - 5.07 (m, 1H), 4.40 - 4.31 (m, 2H), 3.90 - 3.79 (m, 2H), 3.67 (s, 2H). Mass spectrum (mass-to-charge ratio): C₂₃H₂₂N₃O₄⁺ [M+H]⁺; theoretical value: 404.2; measured value: 404.1.

### Example 43b:

Compound 43b (2.3 mg, yield 27%) was synthesized according to general experimental operation 2. ¹H NMR (500 MHz, MeOH-*d*₄) *δ* 8.77 - 8.66 (m, 1H), 8.28 - 8.19 (m, 1H), 8.15 - 8.04 (m, 1H), 7.43 - 7.19 (m, 4H), 7.11 - 7.02 (m, 1H), 6.89 - 6.79 (m, 3H), 6.71 - 6.61 (m, 1H), 5.17 - 5.10 (m, 1H), 4.57 (s, 1H), 4.40 - 4.29 (m, 2H), 3.90 - 3.77 (m, 2H), 3.66 (s, 2H). Mass spectrum (mass-to-charge ratio): C₂₃H₂₃N₄O₄⁺ [M+H]⁺; theoretical value: 419.2; measured value: 419.1.

### Example 43c:

Compound 43c (10.5 mg, yield 6.8%) was synthesized according to general experimental operation 2. ¹H NMR (400 MHz, MeOH-*d*₄) *δ* 8.73 (s, 1H), 8.24 (d, *J* = 4.2 Hz, 1H), 8.15 - 8.06 (m, 1H), 7.41 - 7.32 (m, 1H), 7.29 (d, *J* = 8.7 Hz, 2H), 7.22 - 7.13 (m, 2H), 6.88 - 6.78 (m, 2H), 6.77 - 6.68 (m, 1H), 6.54 - 6.45 (m, 2H), 5.12 - 5.03 (m, 1H), 4.33 - 4.21 (m, 2H), 3.81 - 3.72 (m, 2H), 3.65 (s, 2H). Mass spectrum (mass-to-charge ratio): C₂₂H₂₂N₃O₂⁺ [M+H]⁺; theoretical value: 360.2; measured value: 360.0.

### Example 44:

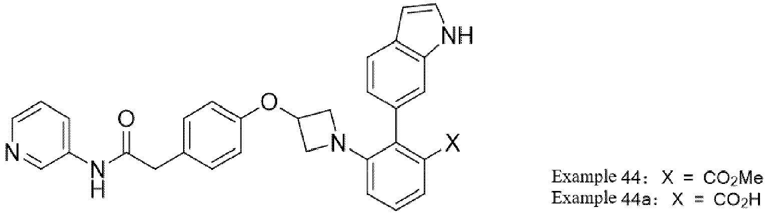

Compound 44 (15 mg, yield 36%) was synthesized according to general experimental operation 2. ¹H NMR (500 MHz, CDCl₃) *δ* 8.64 - 8.53 (m, 1H), 8.41 - 8.33 (m, 1H), 8.33 - 8.22 (m, 1H), 8.14 - 8.03 (m, 1H), 7.70 - 7.56 (m, 2H), 7.32 - 6.99 (m, 8H), 6.70 - 6.62 (m, 1H), 6.62 - 6.56 (m, 2H), 6.56 - 6.48 (m, 1H), 4.71 - 4.59 (m, 1H), 3.75 - 3.61 (m, 2H), 3.59 (s, 2H), 3.55 - 3.39 (m, 5H). Mass spectrum (mass-to-charge ratio): C₃₂H₂₉N₄O₄⁺ [M+H]⁺; theoretical value: 533.2; measured value: 533.2.

### Example 44a:

Compound 44a (3 mg, yield 25%) was synthesized according to general experimental operation 2. ¹H NMR (400 MHz, MeOH-*d*₄) *δ* 8.73 - 8.64 (m, 1H), 8.27 - 8.20 (m, 1H), 8.12 - 8.04 (m, 1H), 7.55 - 7.46 (m, 1H), 7.42 - 7.30 (m, 2H), 7.27 - 7.13 (m, 4H), 7.06 - 6.96 (m, 2H), 6.73 - 6.52 (m, 3H), 6.48 - 6.38 (m, 1H), 4.73 - 4.66 (m, 1H), 3.76 - 3.62 (m, 2H), 3.60 (s, 2H), 3.39 - 3.34 (m, 2H). Mass spectrum (mass-to-charge ratio): C₃₁H₂₇N₄O₄⁺ [M+H]⁺; theoretical value: 519.2; measured value: 519.2.

### Example 45:

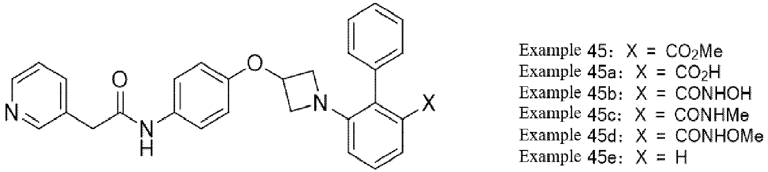

Compound 45 (0 mg, yield 75.86%) was synthesized according to general experimental operation 2. ¹H NMR (500 MHz, CDCl₃) *δ* 8.60 - 8.54 (m, 2H), 7.75 - 7.69 (m, 1H), 7.38 - 7.26 (m, 9H), 7.20 (dd, *J* = 7.6, 1.0 Hz, 1H), 7.07 - 7.03 (br, 1H), 6.68 - 6.64 (m, 1H), 6.61 - 6.55 (m, 2H), 4.73 - 4.64 (m, 1H), 3.70 - 3.64 (m, 4H), 3.51 (s, 3H), 3.49 - 3.45 (m, 2H). Mass spectrum (mass-to-charge ratio): C₃₀H₂₈N₃O₄⁺ [M+H]⁺; theoretical value: 494.20743; measured value: 494.20764.

### Example 45a:

Compound 45a (30 mg, yield 77.19%) was synthesized according to general experimental operation 2. ¹H NMR (500 MHz, MeOH-*d*₄) *δ* 8.51 (d, *J* = 1.6 Hz, 1H), 8.44 (dd, *J* = 4.9, 1.3 Hz, 1H), 7.84 (dt, *J* = 7.9, 1.9 Hz, 1H), 7.46 - 7.37 (m, 3H), 7.37 - 7.25 (m, 6H), 7.11 (dd, *J* = 7.6, 1.0 Hz, 1H), 6.73 (dd, *J* = 8.2, 1.0 Hz, 1H), 6.68 - 6.60 (m, 2H), 4.74 - 4.70 (m, 1H), 3.73 - 3.67 (m, 4H), 3.38 - 3.34 (m, 2H). Mass spectrum (mass-to-charge ratio): C₂₉H₂₆N₃O₄⁺ [M+H]⁺; theoretical value: 480.19178; measured value: 480.19202.

### Example 45b:

Compound 45b (8.1 mg, yield 47.39%) was synthesized according to general experimental operation 2. ¹H NMR (400 MHz, MeOH-*d*₄) *δ* 8.57 - 8.49 (m, 1H), 8.49 - 8.41 (m, 1H), 7.89 - 7.83 (m, 1H), 7.46 - 7.27 (m, 9H), 6.91 - 6.85 (m, 1H), 6.75 - 6.70 (m, 1H), 6.70 - 6.63 (m, 2H), 4.79 - 4.72 (m, 1H), 3.79 - 3.70 (m, 4H), 3.41 - 3.37 (m, 2H). Mass spectrum (mass-to-charge ratio): C₂₉H₂₇N₄O₄⁺ [M+H]⁺; theoretical value: 495.20268; measured value: 495.20288.

### Example 45c:

Compound 45c (10.3 mg, yield 50%) was synthesized according to general experimental operation 2. ¹H NMR (400 MHz, MeOH-*d*₄) *δ* 8.51 (s, 1H), 8.44 (d, *J* = 4.1 Hz, 1H), 7.88 - 7.81 (m, 1H), 7.79 - 7.71 (m, 1H), 7.46 - 7.22 (m, 9H), 6.85 (dd, *J* = 7.5, 1.0 Hz, 1H), 6.71 - 6.61 (m, 3H), 4.77 - 4.69 (m, 1H), 3.78 - 3.67 (m, 4H), 3.41 - 3.33 (m, 2H), 2.52 (d, *J* = 3.9 Hz, 3H). Mass spectrum (mass-to-charge ratio): C₃₀H₂₉N₄O₃⁺ [M+H]⁺; theoretical value: 493.2; measured value: 493.0.

### Example 45d:

Compound 45d (7.3 mg, yield 34%) was synthesized according to general experimental operation 2. ¹H NMR (400 MHz, MeOH-*d*₄) *δ* 8.51 (d, *J* = 1.7 Hz, 1H), 8.44 (dd, *J* = 4.9, 1.4 Hz, 1H), 7.84 (dt, *J* = 7.9, 1.8 Hz, 1H), 7.47 - 7.23 (m, 9H), 6.85 (dd, *J* = 7.5, 0.9 Hz, 1H), 6.74 - 6.68 (m, 1H), 6.68 - 6.62 (m, 2H), 4.78 - 4.70 (m, 1H), 3.79 - 3.73 (m, 2H), 3.71 (s, 2H), 3.42 - 3.37 (m, 2H), 3.28 (s, 3H). Mass spectrum (mass-to-charge ratio): C₃₀H₂₉N₄O₄⁺ [M+H]⁺; theoretical value: 509.2; measured value: 509.2.

### Example 45e:

Compound 45e (9.2 mg, yield 50%) was synthesized according to general experimental operation 2. ¹H NMR (400 MHz, CDCl₃) *δ* 8.63 - 8.46 (m, 2H), 7.73 (d, *J* = 7.8 Hz, 1H), 7.49 - 7.20 (m, 10H), 7.19 - 7.09 (m, 1H), 6.94 - 6.85 (m, 1H), 6.66 - 6.54 (m, 3H), 4.81 - 4.71 (m, 1H), 3.92 - 3.79 (m, 2H), 3.67 (s, 2H), 3.57 - 3.46 (m, 2H). Mass spectrum (mass-to-charge ratio): C₂₈H₂₆N₃O₂⁺ [M+H]⁺; theoretical value: 436.2; measured value: 436.1.

### Example 46:

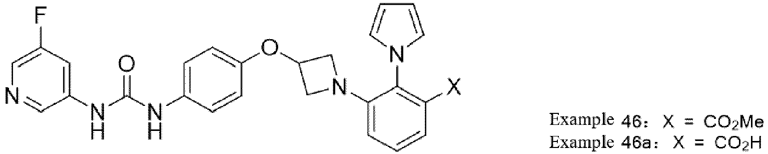

Compound 46 (41 mg, yield 59%) was synthesized according to general experimental operation 2. ¹H NMR (400 MHz, DMSO-*d*₆) *δ* 9.07 (s, 1H), 8.72 (s, 1H), 8.37 (s, 1H), 8.15 (d, *J* = 2.6 Hz, 1H), 7.94 (dt, *J* = 11.7, 2.3 Hz, 1H), 7.37 - 7.29 (m, 3H), 7.01 (dd, *J* = 7.6, 1.1 Hz, 1H), 6.80 (dd, *J* = 8.3, 1.0 Hz, 1H), 6.74 - 6.69 (m, 4H), 6.14 (t, *J* = 2.1 Hz, 2H), 4.93 - 4.79 (m, 1H), 3.81 (dd, *J* = 8.7, 6.3 Hz, 2H), 3.48 (s, 3H), 3.45 (dd, *J* = 9.0, 4.1 Hz, 2H) ppm. Mass spectrum (mass-to-charge ratio): C₂₇H₂₄FN₅O₄⁺ [M+H]⁺; theoretical value: 502.2; measured value: 502.1.

### Example 46a:

Compound 46a (11 mg, yield 79%) was synthesized according to general experimental operation 2. ¹H NMR (500 MHz, DMSO-*d*₆) *δ* 10.06 (s, 1H), 9.58 (s, 1H), 8.39 (s, 1H), 8.11 (s, 1H), 7.92 (d, *J* = 11.8 Hz, 1H), 7.33 (d, *J* = 8.9 Hz, 2H), 7.22 (t, *J* = 7.6 Hz, 1H), 6.92 (d, *J* = 7.5 Hz, 1H), 6.72 (t, *J* = 1.9 Hz, 2H), 6.67 (d, *J* = 8.9 Hz, 2H), 6.59 (d, *J* = 6.5 Hz, 1H), 6.05 (s, 2H), 4.83 (s, 1H), 3.71 (t, *J* = 7.1 Hz, 2H), 3.37 (d, *J* = 6.8 Hz, 2H) ppm. Mass spectrum (mass-to-charge ratio): C₂₆H₂₂FN₅O₄⁺ [M+H]⁺; theoretical value: 488.17286; measured value: 488.17319.

### Example 47:

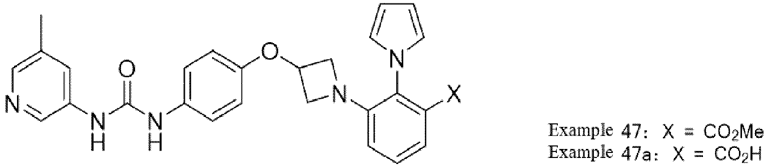

Compound 47 (54 mg, yield 78%) was synthesized according to general experimental operation 2. ¹H NMR (400 MHz, DMSO-*d*₆) *δ* 8.71 (s, 1H), 8.61 (s, 1H), 8.36 (s, 1H), 8.00 (s, 1H), 7.75 (s, 1H), 7.34 (dd, *J* = 15.3, 8.5 Hz, 3H), 7.01 (dd, *J* = 7.6, 1.2 Hz, 1H), 6.80 (dd, *J* = 8.3, 1.2 Hz, 1H), 6.73 (t, *J* = 2.1 Hz, 2H), 6.69 (d, *J* = 9.0 Hz, 2H), 6.14 (t, *J* = 2.1 Hz, 2H), 4.93 - 4.79 (m, 1H), 3.81 (dd, *J* = 8.8, 6.3 Hz, 2H), 3.48 (s, 3H), 3.45 (dd, *J =* 8.9, 4.2 Hz, 2H), 2.26 (s, 3H) ppm. Mass spectrum (mass-to-charge ratio): C₂₈H₂₇N₅O₄⁺ [M+H]⁺; theoretical value: 498.2; measured value: 498.1.

### Example 47a:

Compound 47a (11 mg, yield 75%) was synthesized according to general experimental operation 2. ¹H NMR (500 MHz, DMSO-*d*₆) *δ* 9.56 (s, 1H), 9.40 (s, 1H), 8.41 (s, 1H), 7.98 (s, 1H), 7.72 (s, 1H), 7.32 (d, *J* = 9.0 Hz, 2H), 7.21 (t, *J* = 7.7 Hz, 1H), 6.90 (d, *J* = 7.6 Hz, 1H), 6.72 (t, *J* = 2.0 Hz, 2H), 6.67 (d, *J* = 9.0 Hz, 2H), 6.57 (d, *J* = 7.7 Hz, 1H), 6.06 (s, 2H), 4.83 (d, *J* = 4.6 Hz, 1H), 3.69 (t, *J* = 7.3 Hz, 2H), 3.38 (d, *J* = 11.6 Hz, 2H), 2.25 (s, 3H) ppm. Mass spectrum (mass-to-charge ratio): C₂₇H₂₅N₅O₄⁺ [M+H]⁺; theoretical value: 484.19793; measured value: 484.19809.

### Example 48:

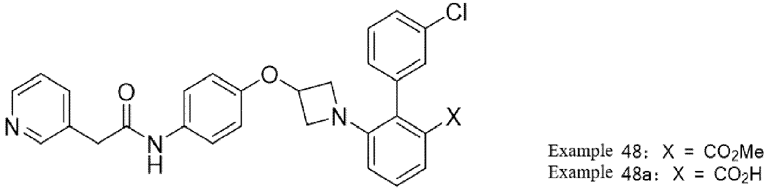

Compound 48 (1.3 mg, yield 2%) was synthesized according to general experimental operation 2. ¹H NMR (400 MHz, CDCl₃) *δ* 8.66 - 8.44 (m, 2H), 7.78 - 7.68 (m, 1H), 7.40 - 7.06 (m, 9H), 6.73 - 6.55 (m, 3H), 4.79 - 4.67 (m, 1H), 3.83 - 3.60 (m, 4H), 3.51 (d, *J* = 47.9 Hz, 5H). Mass spectrum (mass-to-charge ratio): C₃₀H₂₇ClN₃O₄⁺ [M+H]⁺; theoretical value: 528.2; measured value: 528.1.

### Example 48a:

Compound 48a (2 mg, yield 20%) was synthesized according to general experimental operation 2. ¹H NMR (400 MHz, MeOH-*d*₄) *δ* 8.57 - 8.48 (m, 1H), 8.48 - 8.40 (m, 1H), 7.85 (d, *J* = 7.8 Hz, 1H), 7.47 - 7.26 (m, 7H), 7.26 - 7.19 (m, 1H), 7.19 - 7.11 (m, 1H), 6.80 - 6.72 (m, 1H), 6.72 - 6.65 (m, 2H), 4.81 - 4.74 (m, 1H), 3.85 - 3.68 (m, 4H), 3.47 - 3.34 (m, 2H). Mass spectrum (mass-to-charge ratio): C₂₉H₂₅ClN₃O₄⁺ [M+H]⁺; theoretical value: 514.2; measured value: 514.1.

### Example 49:

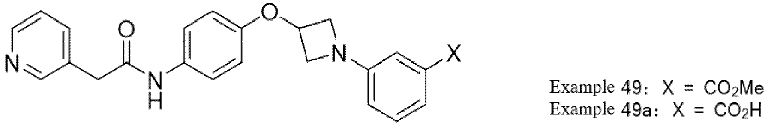

Compound 49 (6.6 mg, yield 13%) was synthesized according to general experimental operation 2. ¹H NMR (400 MHz, CDCl₃) *δ* 8.64 - 8.44 (m, 2H), 7.81 - 7.70 (m, 1H), 7.53 - 7.19 (m, 6H), 7.17 - 7.06 (m, 1H), 6.81 - 6.70 (m, 2H), 6.68 - 6.61 (m, 1H), 5.09 - 5.00 (m, 1H), 4.38 - 4.28 (m, 2H), 3.97 - 3.84 (m, 5H), 3.70 (s, 2H). Mass spectrum (mass-to-charge ratio): C₂₄H₂₄N₃O₄⁺ [M+H]⁺; theoretical value: 418.2; measured value: 418.1.

### Example 49a:

Compound 49a (3.2 mg, yield 66%) was synthesized according to general experimental operation 2. ¹H NMR (500 MHz, MeOH-*d*₄) *δ* 8.53 (s, 1H), 8.45 (d, *J* = 3.9 Hz, 1H), 7.86 (d, *J* = 7.9 Hz, 1H), 7.52 - 7.35 (m, 4H), 7.28 (t, *J* = 7.9 Hz, 1H), 7.13 (s, 1H), 6.88 - 6.79 (m, 2H), 6.78 - 6.69 (m, 1H), 5.15 - 5.07 (m, 1H), 4.39 - 4.28 (m, 2H), 3.87 - 3.80 (m, 2H), 3.73 (s, 2H). Mass spectrum (mass-to-charge ratio): C₂₃H₂₂N₃O₄⁺ [M+H]⁺; theoretical value: 404.2; measured value: 404.1.

### Example 50:

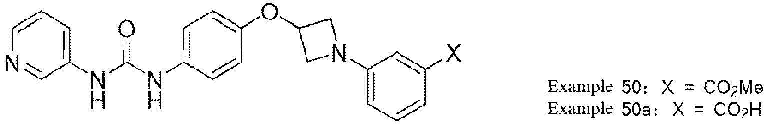

Compound 50 (80 mg, yield 57.04%) was synthesized according to general experimental operation 2. ¹H NMR (500 MHz, MeOH-*d*₄) *δ* 8.61 (d, *J* = 2.6 Hz, 1H), 8.18 (dd, *J* = 4.8, 1.4 Hz, 1H), 8.01 (ddd, *J* = 8.4, 2.6, 1.4 Hz, 1H), 7.43 - 7.35 (m, 4H), 7.30 (t, *J* = 7.9 Hz, 1H), 7.13 (dd, *J* = 2.5, 1.6 Hz, 1H), 6.91 - 6.81 (m, 2H), 6.76 (ddd, *J* = 8.1, 2.6, 1.1 Hz, 1H), 5.13 (ddd, *J* = 6.1, 4.1, 1.9 Hz, 1H), 4.40 - 4.29 (m, 2H), 3.92 - 3.78 (m, 6H). Mass spectrum (mass-to-charge ratio): C₂₃H₂₃N₄O₄⁺ [M+H]⁺; theoretical value: 419.17138 measured value: 419.17139.

### Example 50a:

Compound 50a (17 mg, yield 70.36%) was synthesized according to general experimental operation 2. ¹H NMR (400 MHz, DMSO-*d*₆) *δ* 9.62 (s, 1H), 9.45 (s, 1H), 8.65 (s, 1H), 8.25 - 8.05 (m, 1H), 8.01 - 7.86 (m, 1H), 7.43 (d, *J* = 8.5 Hz, 2H), 7.34 - 7.22 (m, 3H), 7.04 (s, 1H), 6.85 (d, *J* = 8.6 Hz, 2H), 6.69 (d, *J* = 7.0 Hz, 1H), 5.12 (h, *J* = 4.3 Hz, 1H), 4.34 (dd, *J* = 8.4, 6.1 Hz, 2H), 3.80 (dd, *J* = 8.4, 4.0 Hz, 2H). Mass spectrum (mass-to-charge ratio): C₂₂H₂₁N₄O₄⁺ [M+H]⁺; theoretical value: 405.15573; measured value: 405.15573.

### Example 51:

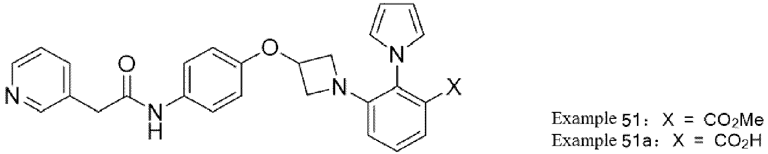

Compound 51 (55 mg, yield 75.31%) was synthesized according to general experimental operation 2. ¹H NMR (500 MHz, CDCl₃) *δ* 8.61 - 8.54 (m, 2H), 7.76 - 7.69 (m, 1H), 7.35 - 7.30 (m, 3H), 7.29 - 7.27 (m, 1H), 7.14 (dd, *J* = 7.6, 1.1 Hz, 1H), 7.01 (br, 1H), 6.69 (t, *J* = 2.1 Hz, 2H), 6.67 (d, *J* = 7.3 Hz, 1H), 6.63 (d, *J* = 9.0 Hz, 2H), 6.24 (t, *J* = 2.1 Hz, 2H), 4.82 - 4.75 (m, 1H), 3.80 - 3.74 (m, 2H), 3.70 (s, 2H), 3.65 - 3.57 (m, 5H). Mass spectrum (mass-to-charge ratio): C₂₈H₂₇N₄O₄⁺ [M+H]⁺; theoretical value: 483.20268; measured value: 483.20282.

### Example 51a:

Compound 51a (15 mg, yield 61.80%) was synthesized according to general experimental operation 2. ¹H NMR (400 MHz, MeOH-*d*₄) *δ* 8.54 (d, *J* = 1.6 Hz, 1H), 8.46 (dd, *J* = 4.9, 1.3 Hz, 1H), 7.90 - 7.84 (m, 1H), 7.48 - 7.41 (m, 3H), 7.33 (t, *J* = 7.9 Hz, 1H), 7.09 (dd, *J* = 7.6, 1.1 Hz, 1H), 6.77 (dd, *J* = 8.2, 1.1 Hz, 1H), 6.74 - 6.66 (m, 4H), 6.20 (t, *J* = 2.1 Hz, 2H), 4.82 - 4.79 (m, 1H), 3.86 - 3.78 (m, 2H), 3.74 (s, 2H), 3.57 - 3.51 (m, 2H). Mass spectrum (mass-to-charge ratio): C₂₇H₂₅N₄O₄⁺ [M+H]⁺; theoretical value: 469.18703; measured value: 469.18716.

### Example 52:

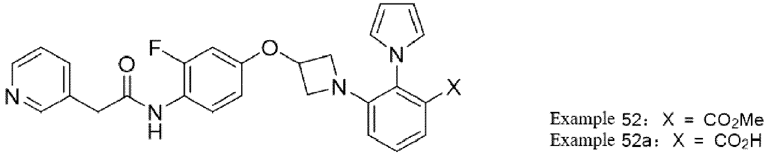

Compound 52 (65 mg, yield 82.55%) was synthesized according to general experimental operation 2. ¹H NMR (400 MHz, CDCl₃) *δ* 8.61 ( m, 2H), 8.03 (t, *J* = 9.0 Hz, 1H), 7.81 - 7.69 (m, 1H), 7.39 - 7.29 (m, 2H), 7.20 (br, 1H), 7.19 - 7.15 (m, 1H), 6.75 - 6.66 (m, 3H), 6.52 - 6.47 (m, 1H), 6.46 - 6.42 (m, 1H), 6.26 (t, *J* = 2.0 Hz, 2H), 4.83 - 4.75 (m, 1H), 3.83 - 3.77 (m, 2H), 3.76 (s, 2H), 3.66 - 3.59 (m, 5H). Mass spectrum (mass-to-charge ratio): C₂₈H₂₆FN₄O₄⁺ [M+H]⁺; theoretical value: 501.19326; measured value: 501.19354.

### Example 52a:

Compound 52a (20 mg, yield 51.44%) was synthesized according to general experimental operation 2. ¹H NMR (400 MHz, MeOH-*d*₄) *δ* 8.56 (s, 1H), 8.47 (d, *J* = 4.3 Hz, 1H), 7.94 - 7.84 (m, 1H), 7.63 - 7.53 (m, 1H), 7.46 (dd, *J* = 7.8, 4.9 Hz, 1H), 7.33 (t, *J* = 7.9 Hz, 1H), 7.08 (dd, *J* = 7.6, 1.3 Hz, 1H), 6.77 (dd, *J* = 8.2, 1.2 Hz, 1H), 6.72 (t, *J* = 2.1 Hz, 2H), 6.63 (dd, *J* = 12.0, 2.7 Hz, 1H), 6.58 - 6.52 (m, 1H), 6.20 (t, *J* = 2.1 Hz, 2H), 5.09 - 5.01 (m, 1H), 3.88 - 3.79 (m, 4H), 3.55 - 3.49 (m, 2H). Mass spectrum (mass-to-charge ratio): C₂₇H₂₄FN₄O₄⁺ [M+H]⁺; theoretical value: 487.17761; measured value: 487.17767.

### Example 53:

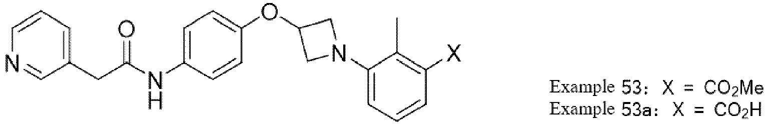

Compound 53 (90 mg, yield 65.15%) was synthesized according to general experimental operation 2. ¹H NMR (500 MHz, CDCl₃) *δ* 8.61 - 8.53 (m, 2H), 7.78 - 7.67 (m, 1H), 7.40 - 7.35 (m, 2H), 7.35 - 7.29 (m, 2H), 7.15 (t, *J* = 7.9 Hz, 1H), 7.09 (br, 1H), 6.78 - 6.74 (m, 2H), 6.70 (d, *J* = 7.5 Hz, 1H), 5.05 - 4.93 (m, 1H), 4.37 - 4.29 (m, 2H), 3.92 - 3.85 (m, 5H), 3.71 (s, 2H), 2.34 (s, 3H). Mass spectrum (mass-to-charge ratio): C₂₅H₂₆N₃O₄⁺ [M+H]⁺; theoretical value: 432.19178; measured value: 432.19177.

### Example 53a:

Compound 53a (12 mg, yield 72.96%) was synthesized according to general experimental operation 2. ¹H NMR (400 MHz, MeOH-*d*₄) *δ* 8.55 (d, *J* = 1.3 Hz, 1H), 8.50 - 8.42 (m, 1H), 7.92 - 7.86 (m, 1H), 7.52 - 7.42 (m, 3H), 7.31 - 7.26 (m, 1H), 7.17 (t, *J* = 7.9 Hz, 1H), 6.90 - 6.84 (m, 2H), 6.83 - 6.77 (m, 1H), 5.08 - 5.05 (m, 1H), 4.43 - 4.35 (m, 2H), 3.89 - 3.82 (m, 2H), 3.76 (s, 2H), 2.36 (s, 3H). Mass spectrum (mass-to-charge ratio): C₂₄H₂₂N₃O₄⁻[M-H]⁻; theoretical value: 416.16158; measured value: 416.16177.

### Example 54:

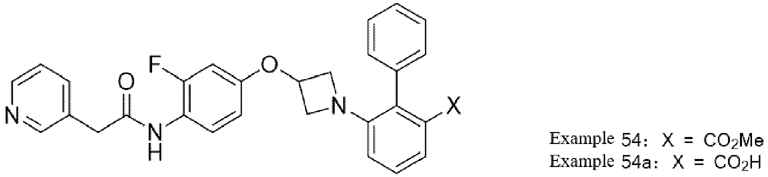

Compound 54 (45 mg, yield 73.64%) was synthesized according to general experimental operation 2. ¹H NMR (500 MHz, CDCl₃) *δ* 8.62 - 8.54 (m, 2H), 7.99 (t, *J* = 9.0 Hz, 1H), 7.75 - 7.68 (m, 1H), 7.38 - 7.26 (m, 7H), 7.21 (d, *J* = 6.7 Hz, 1H), 7.13 (br, 1H), 6.70 - 6.62 (m, 1H), 6.43 (dd, *J* = 12.2, 2.7 Hz, 1H), 6.40 - 6.34 (m, 1H), 4.69 - 4.64 (m, 1H), 3.73 (s, 2H), 3.70 - 3.63 (m, 2H), 3.51 (s, 3H), 3.47 - 3.42 (m, 2H). Mass spectrum (mass-to-charge ratio): C₃₀H₂₇FN₃O₄⁺ [M+H]⁺; theoretical value: 512.19801; measured value: 512.19824.

### Example 54a:

Compound 54a (6 mg, yield 41.13%) was synthesized according to general experimental operation 2. ¹H NMR (500 MHz, MeOH-*d*₄) *δ* 8.52 (d, *J* = 1.8 Hz, 1H), 8.44 (dd, *J* = 4.9, 1.3 Hz, 1H), 7.87 - 7.83 (m, 1H), 7.53 (t, *J* = 8.9 Hz, 1H), 7.45 - 7.40 (m, 1H), 7.37 - 7.26 (m, 6H), 7.14 - 7.08 (m, 1H), 6.77 - 6.71 (m, 1H), 6.57 (dd, *J* = 12.1, 2.7 Hz, 1H), 6.53 - 6.46 (m, 1H), 4.76 - 4.75 (m, 1H), 3.77 (s, 2H), 3.72 (dd, *J* = 8.9, 6.2 Hz, 2H), 3.38 - 3.35 (m, 2H). Mass spectrum (mass-to-charge ratio): C₂₉H₂₅FN₃O₄⁺ [M+H]⁺; theoretical value: 498.18236; measured value: 498.18250.

### Example 55:

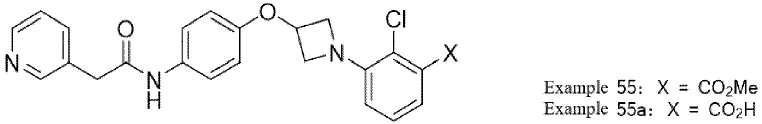

Compound 55 (80 mg, yield 73.64%) was synthesized according to general experimental operation 2. ¹H NMR (500 MHz, CDCl₃) *δ* 8.57 (d, *J* = 2.7 Hz, 2H), 7.77 - 7.71 (m, 1H), 7.41 - 7.36 (m, 2H), 7.34 - 7.29 (m, 2H), 7.20 - 7.11 (m, 2H), 6.76 - 6.72 (m, 2H), 6.66 (dd, *J* = 7.7, 1.9 Hz, 1H), 5.00 - 4.95 (m, 1H), 4.57 - 4.49 (m, 2H), 4.05 - 4.01 (m, 2H), 3.91 (s, 3H), 3.70 (s, 2H). Mass spectrum (mass-to-charge ratio): C₂₄H₂₃ClN₃O₄⁺ [M+H]⁺; theoretical value: 452.13716; measured value: 452.13699.

### Example 55a:

Compound 55a (11 mg, yield 70.95%) was synthesized according to general experimental operation 2. ¹H NMR (500 MHz, MeOH-*d*₄) *δ* 8.54 (d, *J* = 1.7 Hz, 1H), 8.46 (dd, *J* = 4.9, 1.3 Hz, 1H), 7.91 - 7.86 (m, 1H), 7.50 - 7.43 (m, 3H), 7.22 (t, *J* = 7.9 Hz, 1H), 7.11 (dd, *J* = 7.6, 1.4 Hz, 1H), 6.87 - 6.81 (m, 2H), 6.78 (dd, *J* = 8.2, 1.3 Hz, 1H), 5.06 - 5.03 (m, 1H), 4.56 - 4.52 (m, 2H), 4.02 - 3.96 (m, 2H), 3.74 (s, 2H). Mass spectrum (mass-to-charge ratio): C₂₃H₂₁ClN₃O₄⁺ [M+H]⁺; theoretical value: 438.1; measured value: 438.0.

### Example 56:

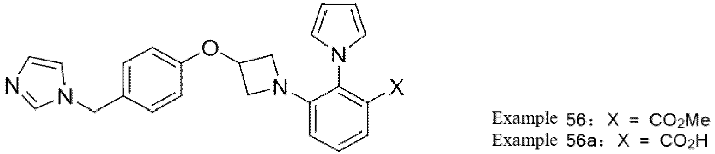

Compound 56 (12 mg, yield 26.49%) was synthesized according to general experimental operation 2. ¹H NMR (500 MHz, CDCl₃) *δ* 7.59 (s, 1H), 7.29 (t, *J* = 7.9 Hz, 1H), 7.19 - 6.94 (m, 5H), 6.70 (t, *J* = 2.1 Hz, 2H), 6.69 - 6.65 (m, 3H), 6.24 (t, *J* = 2.1 Hz, 2H), 5.03 (s, 2H), 4.85 - 4.77 (m, 1H), 3.79 (dd, *J* = 9.1, 6.2 Hz, 2H), 3.64 (dd, *J* = 9.1, 4.8 Hz, 2H), 3.61 (s, 3H). Mass spectrum (mass-to-charge ratio): C₂₅H₂₅N₄O₃⁺ [M+H]⁺; theoretical value: 429.2; measured value: 429.2.

### Example 56a:

Compound 56a (2 mg, yield 20.68%) was synthesized according to general experimental operation 2. ¹H NMR (500 MHz, MeOH-*d*₄) *δ* 7.96 (s, 1H), 7.27 (t, *J* = 7.9 Hz, 1H), 7.23 - 7.19 (m, 2H), 7.19 - 7.16 (m, 1H), 7.10 - 7.06 (m, 1H), 7.00 - 6.95 (m, 1H), 6.76 - 6.71 (m, 4H), 6.69 - 6.66 (m, 1H), 6.15 (t, *J* = 2.0 Hz, 2H), 5.17 (s, 2H), 4.87 - 4.85 (m, 1H), 3.82 - 3.76 (m, 2H), 3.49 (dd, *J* = 8.9, 4.4 Hz, 2H). Mass spectrum (mass-to-charge ratio): C₂₄H₂₃N₄O₃⁺ [M+H]⁺; theoretical value: 415.2; measured value: 415.2.

### Example 57:

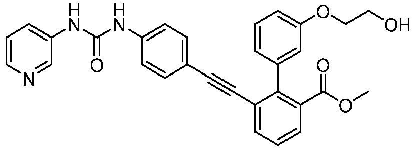

Compound 57 (120 mg, yield 71.0%) was synthesized according to the general experimental operation 3. ¹H NMR (400 MHz, MeOD) δ 8.60 (s, 1H), 8.24 - 8.15 (m, 1H), 8.00 (d, J = 9.3 Hz, 1H), 7.72 (dd, J = 7.8, 3.5 Hz, 2H), 7.46 (t, J = 7.8 Hz, 1H), 7.42 - 7.31 (m, 4H), 7.11 (d, J = 8.7 Hz, 2H), 7.03 (dd, J = 8.3, 1.9 Hz, 1H), 6.96 (s, 1H), 6.88 (d, J = 7.6 Hz, 1H), 4.12 - 4.02 (m, 2H), 3.92 - 3.83 (m, 2H), 3.58 (s, 3H). Mass spectrum (mass-to-charge ratio): C₃₀H₂₆N₃O₅⁺[M+H]⁺; theoretical value: 508.19; measured value: 508.20.

### Example 58:

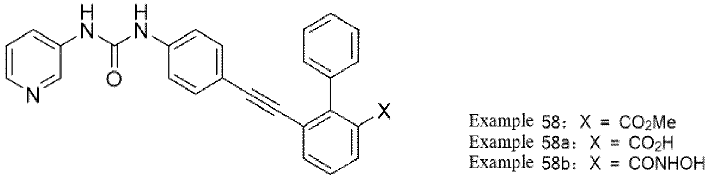

Compound 58 (50 mg, yield 52.80%) was synthesized according to the general experimental operation 3. ¹H NMR (500 MHz, MeOH-*d*₄) *δ* 8.60 (d, *J* = 2.4 Hz, 1H), 8.19 (dd, *J* = 4.8, 1.2 Hz, 1H), 8.01 (ddd, *J* = 8.4, 2.6, 1.4 Hz, 1H), 7.73 (d, *J* = 7.8 Hz, 2H), 7.48 - 7.41 (m, 4H), 7.39 - 7.35 (m, 3H), 7.34 - 7.30 (m, 2H), 7.09 - 7.04 (m, 2H), 3.55 (s, 3H). Mass spectrum (mass-to-charge ratio): C₂₈H₂₂N₃O₃⁺ [M+H]⁺; theoretical value: 448.16557; measured value: 448.16568.

### Example 58a:

Compound 58a (1.4 mg, yield 14.45%) was synthesized according to general experimental operation 3. ¹H NMR (400 MHz, MeOH-d4) *δ* 8.62 - 8.56 (m, 1H), 8.22 - 8.16 (m, 1H), 8.05 - 7.99 (m, 1H), 7.75 - 7.62 (m, 3H), 7.43 - 7.36 (m, 8H), 7.10 - 7.06 (m, 2H). Mass spectrum (mass-to-charge ratio): C₂₇H₁₈N₃O₃⁻[M-H]⁻; theoretical value: 432.13537; measured value: 432.13550.

### Example 58b:

Compound 58b (3.7 mg, yield 43.94%) was synthesized according to general experimental operation 3. ¹H NMR (500 MHz, MeOH-*d*₄) *δ* 8.61 (s, 1H), 8.19 (d, *J* = 4.5 Hz, 1H), 8.01 (d, *J* = 8.9 Hz, 1H), 7.70 - 7.65 (m, 1H), 7.47 - 7.36 (m, 10H), 7.12 - 7.05 (m, 2H). Mass spectrum (mass-to-charge ratio): C₂₇H₁₉N₄O_{3'}[M-H]⁻; theoretical value: 447.14626; measured value: 447.14661.

### Example 59:

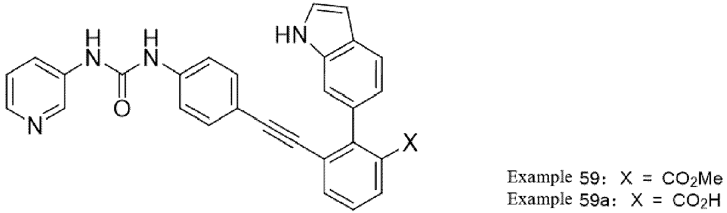

Compound 59 (40 mg, yield 41.15%) was synthesized according to the general experimental operation 3. ¹H NMR (500 MHz, MeOH-*d*₄) *δ* 8.58 (d, *J* = 2.4 Hz, 1H), 8.18 (dd, *J* = 4.8, 1.4 Hz, 1H), 7.99 (ddd, *J* = 8.4, 2.6, 1.4 Hz, 1H), 7.71 (dd, *J* = 7.7, 1.3 Hz, 1H), 7.65 (dd, *J* = 7.8, 1.3 Hz, 1H), 7.60 (dd, *J* = 8.1, 0.5 Hz, 1H), 7.44 - 7.39 (m, 2H), 7.38 - 7.33 (m, 1H), 7.32 - 7.27 (m, 3H), 7.01 - 6.95 (m, 3H), 6.52 (dd, *J* = 3.1, 0.9 Hz, 1H), 3.49 (s, 3H). Mass spectrum (mass-to-charge ratio): C₃₀H₂₃N₄O₃⁺ [M+H]⁺; theoretical value: 487.17647; measured value: 487.17667.

### Example 59a:

Compound 59a (5.5 mg, yield 80.90%) was synthesized according to general experimental operation 3. ¹H NMR (400 MHz, MeOH-*d*₄): *δ* 8.63 - 8.58 (m, 1H), 8.21 - 8.17 (m, 1H), 8.05 - 7.99 (m, 1H), 7.67 - 7.59 (m, 3H), 7.53 - 7.49 (m, 1H), 7.42 - 7.28 (m, 6H), 7.01 - 6.98 (m, 2H), 6.53 - 6.50 (m, 1H). Mass spectrum (mass-to-charge ratio): C₂₉H₂₁N₄O₃⁺ [M+H]⁺; theoretical value: 473.16082; measured value: 473.16101.

### Example 60:

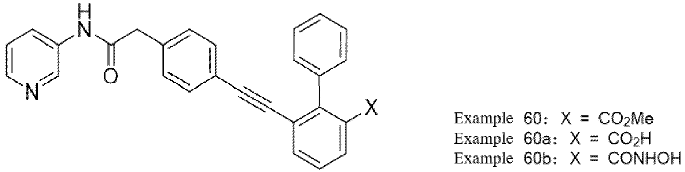

Compound 60 (55 mg, yield 48.51%) was synthesized according to the general experimental operation 3. ¹H NMR (500 MHz, MeOH-*d*₄) *δ* 8.71 (d, *J* = 2.3 Hz, 1H), 8.25 (dd, *J* = 4.8, 1.3 Hz, 1H), 8.10 (ddd, *J* = 8.4, 2.5, 1.5 Hz, 1H), 7.77 - 7.72 (m, 2H), 7.48 - 7.36 (m, 5H), 7.33 - 7.29 (m, 2H), 7.28 - 7.24 (m, 2H), 7.13 - 7.08 (m, 2H), 3.69 (s, 2H), 3.54 (s, 3H). Mass spectrum (mass-to-charge ratio): C₂₉H₂₃N₂O₃⁺ [M+H]⁺; theoretical value: 447.17032; measured value: 447.17050.

### Example 60a:

Compound 60a (7.1 mg, yield 81.45%) was synthesized according to the general experimental operation 3. ¹H NMR (400 MHz, MeOH-*d*₄) *δ* 8.74 (d, *J* = 2.3 Hz, 1H), 8.30 - 8.24 (m, 1H), 8.16 - 8.09 (m, 1H), 7.78 (dd, *J* = 7.7, 1.0 Hz, 1H), 7.73 (dd, *J* = 7.7, 1.1 Hz, 1H), 7.49 - 7.36 (m, 7H), 7.32 - 7.24 (m, 2H), 7.16 - 7.09 (m, 2H), 3.72 (s, 2H). Mass spectrum (mass-to-charge ratio): C₂₈H₂₁N₂O₃⁺ [M+H]⁺; theoretical value: 433.15467; measured value: 433.15485.

### Example 60b:

Compound 60b (4 mg, yield 15.84%) was synthesized according to the general experimental operation 3. ¹H NMR (400 MHz, MeOH-*d*₄) *δ* 8.74 (d, *J* = 1.7 Hz, 1H), 8.30 - 8.25 (m, 1H), 8.15 - 8.09 (m, 1H), 7.75 - 7.68 (m, 1H), 7.50 - 7.38 (m, 8H), 7.29 (d, *J* = 8.1 Hz, 2H), 7.15 (d, *J* = 8.1 Hz, 2H), 3.72 (s, 2H). Mass spectrum (mass-to-charge ratio): C₂₈H₂₀N₃O₃⁻M-H]⁻; theoretical value: 446.15102; measured value: 446.15088.

### Example 61:

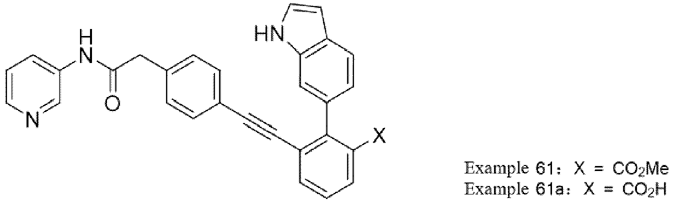

Compound 61 (65 mg, yield 61.43%) was synthesized according to the general experimental operation 3. ¹H NMR (500 MHz, MeOH-*d*₄) *δ* 8.74 - 8.64 (m, 1H), 8.29 - 8.21 (m, 1H), 8.13 - 8.04 (m, 1H), 7.75 - 7.70 (m, 1H), 7.67 (dd, *J* = 7.8, 1.3 Hz, 1H), 7.61 - 7.57 (m, 1H), 7.45 - 7.41 (m, 2H), 7.40 - 7.34 (m, 1H), 7.29 (d, *J* = 3.1 Hz, 1H), 7.21 - 7.16 (m, 2H), 7.04 - 7.00 (m, 2H), 6.97 (dd, *J* = 8.1, 1.5 Hz, 1H), 6.50 (dd, *J* = 3.1, 0.9 Hz, 1H), 3.66 (s, 2H), 3.49 (s, 3H). Mass spectrum (mass-to-charge ratio): C₃₁H₂₄N₃O₃⁺ [M+H]⁺; theoretical value: 486.18122; measured value: 486.18149.

### Example 61a:

Compound 61a (15 mg, yield 38.62%) was synthesized according to the general experimental operation 3. ¹H NMR (500 MHz, MeOH-*d*₄) *δ* 8.69 (d, *J* = 2.2 Hz, 1H), 8.24 (dd, *J* = 4.8, 1.4 Hz, 1H), 8.11 - 8.04 (m, 1H), 7.72 - 7.66 (m, 2H), 7.58 (d, *J* = 8.1 Hz, 1H), 7.47 - 7.44 (m, 1H), 7.43 - 7.35 (m, 2H), 7.28 - 7.24 (m, 1H), 7.21 - 7.16 (m, 2H), 7.05 (dd, *J* = 8.1, 1.5 Hz, 1H), 7.03 - 6.98 (m, 2H), 6.49 (dd, *J* = 3.1, 0.8 Hz, 1H), 3.66 (s, 2H). Mass spectrum (mass-to-charge ratio): C₃₀H₂₀N₃O₃⁻[M-H]⁻; theoretical value: 470.15102; measured value: 470.15100.

### Example 62:

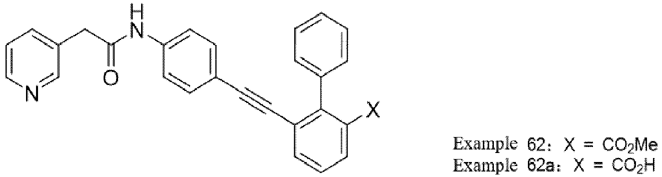

Compound 62 (60 mg, yield 52.92%) was synthesized according to the general experimental operation 3. ¹H NMR (500 MHz, CDCl₃) *δ* 8.59 - 8.56 (m, 1H), 8.54 (s, 1H), 7.79 (dd, *J* = 7.8, 1.3 Hz, 1H), 7.74 - 7.68 (m, 2H), 7.44 - 7.29 (m, 10H), 7.10 - 7.03 (m, 2H), 3.70 (s, 2H), 3.58 (s, 3H). Mass spectrum (mass-to-charge ratio): C₂₉H₂₃N₂O₃⁺ [M+H]⁺; theoretical value: 447.17032; measured value: 447.17038.

### Example 62a:

Compound 62a (45 mg, yield 84.47%) was synthesized according to the general experimental operation 3. ¹H NMR (500 MHz, DMSO-*d*₆) *δ* 10.37 (s, 1H), 8.49 (d, *J* = 1.4 Hz, 1H), 8.44 (dd, *J* = 4.7, 1.3 Hz, 1H), 7.76 - 7.66 (m, 3H), 7.59 - 7.28 (m, 9H), 7.12 - 7.04 (m, 2H), 3.69 (s, 2H). Mass spectrum (mass-to-charge ratio): C₂₈H₂₁N₂O₃⁺ [M+H]⁺; theoretical value: 433.15467; measured value: 433.15475.

### Example 63:

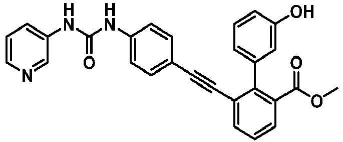

Compound 63 (68 mg, yield 71.38%) was synthesized according to the general experimental operation 3. ¹H NMR (400 MHz, DMSO) δ 9.49 (s, 1H), 9.06 (s, 1H), 8.97 (s, 1H), 8.45 (d, J = 152.9 Hz, 1H), 7.92 (d, J = 8.4 Hz, 1H), 7.75 (dd, J = 7.7, 1.1 Hz, 1H), 7.68 (dd, J = 7.8, 1.1 Hz, 1H), 7.49 (t, J = 7.8 Hz, 1H), 7.44 (d, J = 8.7 Hz, 2H), 7.34 (dt, J = 16.0, 7.2 Hz, 1H), 7.23 (t, J = 7.8 Hz, 1H), 7.15 (d, J = 8.6 Hz, 2H), 6.80 (dd, J = 8.1, 2.0 Hz, 1H), 6.77 - 6.71 (m, 1H), 6.68 (d, J = 7.6 Hz, 1H), 3.53 (s, 3H) ppm. Mass spectrum (mass-to-charge ratio): C₂₈H₂₁O₄N₃⁺ [M+H]⁺; theoretical value: 464.2; measured value: 464.2.

### Example 64: C666-1 cell viability assay

In order to evaluate the inhibitory effect of the small molecular compound of the present invention on EBNA1 at the cellular level, a cytotoxicity test was performed. The small molecule compound of the present invention can selectively kill EB virus-positive cell line (C666-1 cells), which is significantly superior to EB virus-negative cell lines (HONE1 cells and HK1 cells).

As mentioned in the Background of the present invention, nasopharyngeal carcinoma is a kind of disease highly associated with latent infection of EB virus. EB virus in latent state only expresses limited proteins to maintain its genome replication, among which EBNA1 protein is an essential functional protein. Studies have confirmed that inhibition of EBNA1 can reduce the proliferation of the corresponding nasopharyngeal carcinoma cells or kill them. Therefore, EB virus-positive nasopharyngeal carcinoma tumor cells (C666-1 cells) are selected for test to verify the effect of the small molecular compound of the present invention.

During testing, 100 microliters of different cell lines were inoculated onto a transparent 96-well plate, and 5×10³ cells were inoculated in each well for C666-1 cells. After the cells were cultured in an incubator under 5% carbon dioxide at 37°C for 24 hours, 10 microliters of compound with a concentration ranging from 1 mmol/L to 7.8 micromol/L were added to each well (8 points, double dilution, the final concentration was 100-0.78 micromole/L), and treated in the incubator under carbon dioxide at 37°C for 72 hours. Cell viability was assessed using the redox indicator Resazurin. After addition of 10 microliters of 600 micromole/liter Resazurin to each well and incubation at 37 °C for 3 hours. Using a Tecan microplate reader, the fluorescence signals were detected at an emission wavelength of 560 nanometer and an excitation wavelength of 590 nanometer. A software was used to fit the inhibition curve and calculate half-maximal effect concentration (EC₅₀). The selectivity of compound activity was assessed by comparing the EC₅₀ values of EB virus-positive and EB virus-negative cell lines.

| **Example Serial Number** | Formula | Chinese Name | Inhibitor y Rate |
|---|---|---|---|
| **Example 1** | 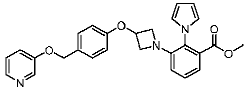 | methyl 3-(3-(4-((pyridin-3-yloxy)methyl)phenoxy)azet idin-1-yl)-2-(1H-pyrrol-1-yl) benzoate | +++ |
| **Example 1a** | 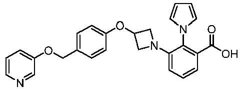 | 3-(3-(4-((pyridin-3-yloxy)methyl)phenoxy)azet idin-1-yl)-2-(1H-pyrrol-1-yl) benzoic acid | +++ |
| **Example 1b** | 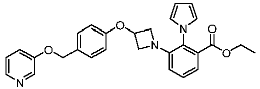 | ethyl 3-(3-(4-((pyridin-3-yloxy)methyl)phenoxy)azet idin-1-yl)-2-(1H-pyrrol-1-yl) benzoate | ++++ |
| **Example 1c** | 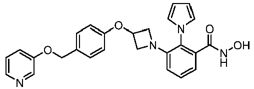 | N-hydroxy-3-(3-(4-((pyridin-3-yloxy)methyl)p henoxy)azetidin-1-yl)-2-(1H-pyrrole-1-yl) benzamide | ++++ |
| **Example 1d** | 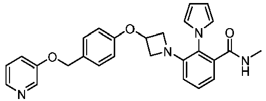 | N-methyl-3-(3-(4-((pyridin-3-yloxy)methyl)ph enoxy)azetidin-1-yl)-2-(1H-pyrrol-1-yl) benzamide | ++++ |
| **Example 1e** | 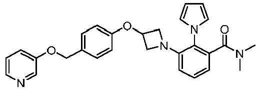 | N,N-dimethyl-3-(3-(4-((pyridin-3-yloxy)methy l)phenoxy)azetidin-1-yl)-2-(1H-pyrrol-1-yl) benzamide | +++ |
| **Example 1f** | 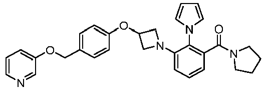 | (3-(3-(4-((pyridin-3-yloxy)methyl)phenoxy)az etidin-1-yl)-2-(1H-pyrrol-1-yl)phenyl)(pyrrolid in-1-yl) methanone | +++ |
| **Example 1g** | 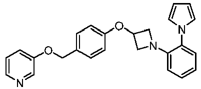 | 3-((4-((1-(2-(1H-pyrrole-1-yl)phenyl)azetidin-3-yl)oxy)benzyl)oxy) pyridine | ++ |
| **Example 2** | 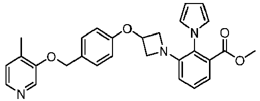 | methyl 3-(3-(4-((4-methylpyridin-3-yloxy)methyl)phe noxy)azetidin-1-yl)-2-(1H-pyrrol-1-yl) benzoate | + |
| **Example 2a** | 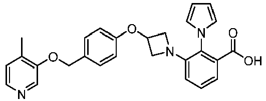 | 3-(3-(4-((4-methylpyridin-3-yloxy)methyl)phe noxy)azetidin-1-yl)-2-(1H-pyrrol-1-yl) benzoic acid | + |
| **Example 3** | 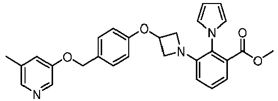 | methyl 3-(3-(4-((5-methylpyridin-3-yloxy)methyl)phe noxy)azetidin-1-yl)-2-(1H-pyrrol-1-yl) benzoate | + |
| **Example 3a** | 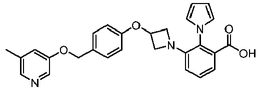 | 3-(3-(4-((5-methylpyridin-3-yloxy)methyl)phe noxy)azetidin-1-yl)-2-(1H-pyrrol-1-yl) benzoic acid | + |
| **Example 4** | 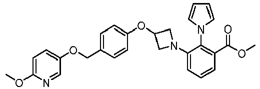 | methyl 3-(3-(4-(((6-methoxypyridin-3-yl)oxy)methyl) phenoxy)azetidin-1-yl)-2-(1H-pyrrol-1-yl) benzoate | + |
| **Example 4a** | 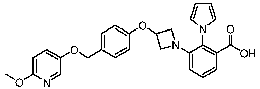 | 3-(3-(4-(((6-methoxypyridin-3-yl)oxy)methyl) phenoxy)azetidin-1-yl)-2-(1H-pyrrol-1-yl) benzoic acid | + |
| **Example 5** | 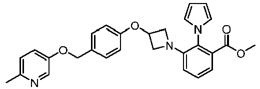 | methyl 3-(3-(4-((6-methylpyridin-3-yloxy)methyl)phe noxy)azetidin-1-yl)-2-(1H-pyrrol-1-yl) benzoate | + |
| **Example 5a** | 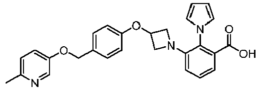 | 3-(3-(4-((6-methylpyridin-3-yloxy)methyl)phe noxy)azetidin-1-yl)-2-(1H-pyrrol-1-yl) benzoic acid | + |
| **Example 6** | 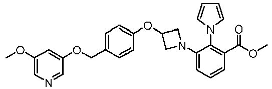 | methyl 3-(3-(4-(((5-methoxypyridin-3-yl)oxy)methyl) phenoxy)azetidin-1-yl)-2-(1H-pyrrol-1-yl) benzoate | + |
| **Example 6a** | 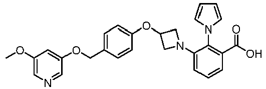 | 3-(3-(4-(((5-methoxypyridin-3-yl)oxy)methyl) phenoxy)azetidin-1-yl)-2-(1H-pyrrol-1-yl) benzoic acid | + |
| **Example 7** | 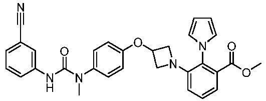 | methyl 3-(3-(4-(3-(3-cyanophenyl)-1-methylureido)ph enoxy)azetidin-1-yl)-2-(1H-pyrrol-1-yl) benzoate | ++ |
| **Example 7a** | 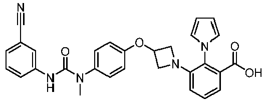 | 3-(3-(4-(3-(3-cyanophenyl)-1-methylureido)ph enoxy)azetidin-1-yl)-2-(1H-pyrrol-1-yl) benzoic acid | ++ |
| **Example 8** | 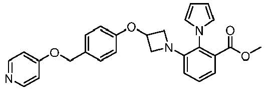 | methyl 3-(3-(4-((pyridin-4-yloxy)methyl)phenoxy)azet idin-1-yl)-2-(1H-pyrrol-1-yl) benzoate | ++ |
| **Example 8a** | 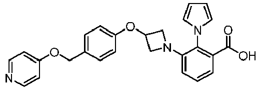 | 3-(3-(4-((pyridin-4-yloxy)methyl)phenoxy)azet idin-1-yl)-2-(1H-pyrrol-1-yl) benzoic acid | + |
| **Example 9** | 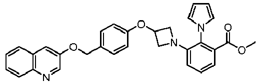 | methyl 2-(1H-pyrrol-1-yl)-3-(3-(4-((quinolin-3-yloxy) methyl)phenoxy)azetidin-1-yl) benzoate | + |
| **Example 9a** | 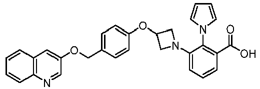 | 2-(1H-pyrrol-1-yl)-3-(3-(4-((quinolin-3-yloxy) methyl)phenoxy)azetidin-1-yl) benzoic acid | + |
| **Example 10** | 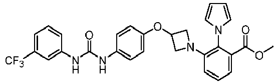 | methyl 2-(1H-pyrrol-1-yl)-3-(3-(4-(3-(3-(trifluorometh yl)phenyl)ureido)phenoxy)azetidin-1-yl) benzoate | ++ |
| **Example 10a** | 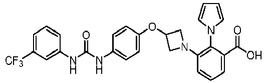 | 2-(1H-pyrrol-1-yl)-3-(3-(4-(3-(3-(trifluorometh yl)phenyl)ureido)phenoxy)azetidin-1-yl) benzoic acid | ++ |
| **Example 10b** | 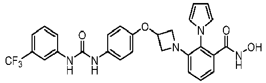 | N-hydroxy-2-(1H-pyrrol-1-yl)-3-(3-(4-(3-(3-(tr ifluoromethyl)phenyl)ureido)phenoxy)azetidin -1-yl) benzamide | +++ |
| **Example 11** | 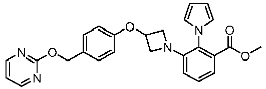 | methyl 3 -(3-(4-((pyrimidin-2-yloxy)methyl)phenoxy)a zetidin-1-yl)-2-(1H-pyrrol-1-yl) benzoate | + |
| **Example 11a** | 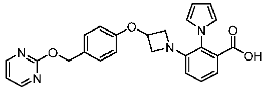 | 3-(3-(4-((pyrimidin-2-yloxy)methyl)phenoxy)a zetidin-1-yl)-2-(1H-pyrrol-1-yl) benzoic acid | + |
| **Example 12** | 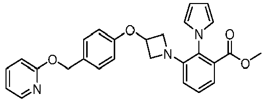 | methyl 3-(3-(4-((pyridin-2-yloxy)methyl)phenoxy)azet idin-1-yl)-2-(1H-pyrrol-1-yl) benzoate | + |
| **Example 12a** | 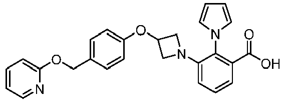 | 3-(3-(4-((pyridin-2-yloxy)methyl)phenoxy)azet idin-1-yl)-2-(1H-pyrrol-1-yl) benzoic acid | + |
| **Example 13** | 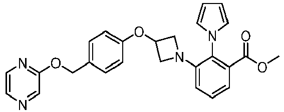 | methyl 3-(3-(4-((pyrazin-2-yloxy)methyl)phenoxy)aze tidin-1-yl)-2-(1H-pyrrol-1-yl) benzoate | + |
| **Example 13a** | 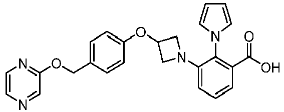 | 3-(3-(4-((pyrazin-2-yloxy)methyl)phenoxy)aze tidin-1-yl)-2-(1H-pyrrol-1-yl) benzoic acid | + |
| **Example 14** | 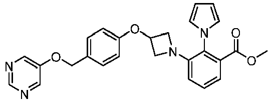 | methyl 3-(3-(4-((pyrimidin-5-yloxy)methyl)phenoxy)a zetidin-1-yl)-2-(1H-pyrrol-1-yl) benzoate | +++ |
| **Example 14a** | 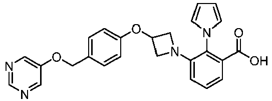 | 3-(3-(4-((pyrimidin-5-yloxy)methyl)phenoxy)a zetidin-1-yl)-2-(1H-pyrrol-1-yl) benzoic acid | +++ |
| **Example 14b** | 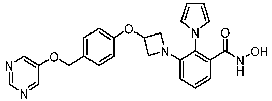 | N-hydroxy-3-(3-(4-((pyrimidin-5-oxy)methyl) phenoxy)azetidin-1-yl)-2-(1H-pyrrol-1-yl) benzamide | +++ |
| **Example 15** | 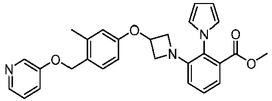 | methyl 3-(3-(3-methyl-4-((pyridin-3-yloxy)methyl)phe noxy)azetidin-1-yl)-2-(1H-pyrrol-1-yl) benzoate | ++ |
| **Example 15a** | 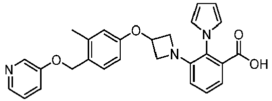 | 3-(3-(3-methyl-4-((pyridin-3-yloxy)methyl)phe noxy)azetidin-1-yl)-2-(1H-pyrrol-1-yl) benzoic acid | ++ |
| **Example 16** | 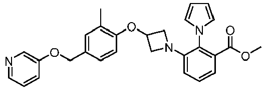 | methyl 3-(3-(2-methyl-4-((pyridin-3-yloxy)methyl)phe noxy)azetidin-1-yl)-2-(1H-pyrrol-1-yl) benzoate | ++ |
| **Example 16a** | 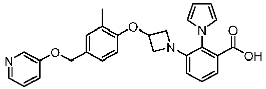 | 3-(3-(2-methyl-4-((pyridin-3-yloxy)methyl)phe noxy)azetidin-1-yl)-2-(1H-pyrrol-1-yl) benzoic acid | + |
| **Example 17** | 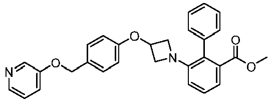 | methyl 6-(3-(4-((pyridin-3-yloxy)methyl)phenoxy)azet idin-1-yl)-[1,1'-biphenyl]-2-carboxylate | +++ |
| **Example 17a** | 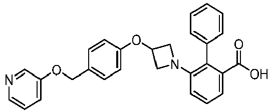 | 6-(3-(4-((pyridin-3-yloxy)methyl)phenoxy)azet idin-1-yl)-[1,1'-biphenyl]-2-carboxylic acid | +++ |
| **Example 17b** | 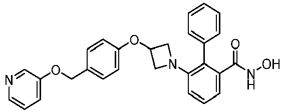 | N-hydroxy-6-(3-(4-((pyridin-3-yloxy)methyl) phenoxy)azetidin-1-yl)-[1,1'-biphenyl] -2-carbo xamide | +++ |
| **Example 18** | 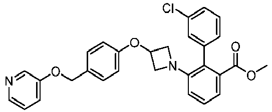 | methyl 3'-chloro-6-(3-(4-((pyridin-3-yloxy)methyl)phe noxy)azetidin-1-yl)-[1,1'-biphenyl]-2-carboxyl ate | +++ |
| **Example 19** | 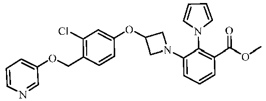 | methyl 3-(3-(3-chloro-4-((pyridin-3-yloxy)methyl)phe noxy)azetidin-1-yl)-2-(1H-pyrrol-1-yl) benzoate | ++ |
| **Example 19a** | 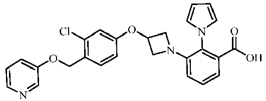 | 3-(3-(3-chloro-4-((pyridin-3-yloxy)methyl)phe noxy)azetidin-1-yl)-2-(1H-pyrrol-1-yl) benzoic acid | ++ |
| **Example 20** | 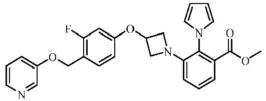 | methyl 3-(3-(3-fluoro-4-((pyridin-3-yloxy)methyl)phe noxy)azetidin-1-yl)-2-(1H-pyrrol-1-yl) benzoate | +++ |
| **Example 20a** | 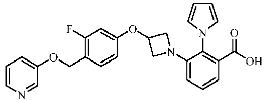 | 3-(3-(3-fluoro-4-((pyridin-3-yloxy)methyl)phe noxy)azetidin-1-yl)-2-(1H-pyrrol-1-yl) benzoic acid | ++ |
| **Example 21** | 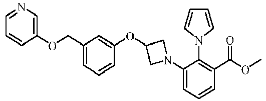 | methyl 3-(3-(3-((pyridin-3-yloxy)methyl)phenoxy)azet idin-1-yl)-2-(1H-pyrrol-1-yl) benzoate | ++ |
| **Example 22** | 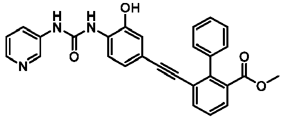 | methyl 6-((3-hydroxy-4-(3-(pyridin-3-yl)ureido)pheny 1)ethynyl)-[1,1'-biphenyl]-2-carboxylate | ++++ |
| **Example 23** | 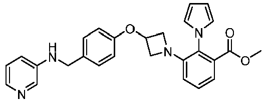 | methyl 3-(3-(4-((pyridin-3-ylamino)methyl)phenoxy)a zetidin-1-yl)-2-(1H-pyrrol-1-yl) benzoate | ++ |
| **Example 23a** | 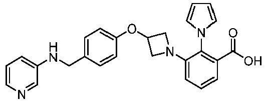 | 3-(3-(4-((pyridin-3-ylamino)methyl)phenoxy)a zetidin-1-yl)-2-(1H-pyrrol-1-yl) benzoic acid | ++ |
| **Example 23b** | 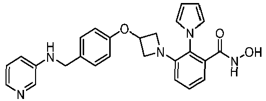 | N-hydroxy-3-(3-(4-((pyridin-3-ylamino)methyl )phenoxy)azetidin-1-yl)-2-(1H-pyrrol-1-yl) benzamide | ++++ |
| **Example 24** | 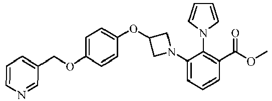 | methyl 3-(3-(4-(pyridin-3-ylmethoxy)phenoxy)azetidi n-1-yl)-2-(1H-pyrrol-1-yl) benzoate | +++ |
| **Example 24a** | 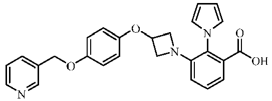 | 3-(3-(4-(pyridin-3-ylmethoxy)phenoxy)azetidi n-1-yl)-2-(1H-pyrrol-1-yl) benzoic acid | ++ |
| **Example 24b** | 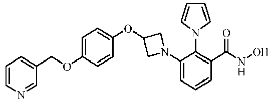 | N-hydroxy-3-(3-(4-(pyridin-3-ylmethoxy)phen oxy)azetidin-1-yl)-2-(1H-pyrrol-1-yl) benzamide | ++++ |
| **Example 25** | 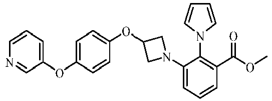 | methyl 3-(3-(4-(pyridin-3-yloxy)phenoxy)azetidin-1-y 1)-2-(1H-pyrrol-1-yl) benzoate | + |
| **Example 25a** | 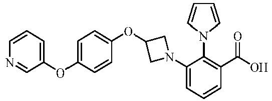 | 3-(3-(4-(pyridin-3-yloxy)phenoxy)azetidin-1-y 1)-2-(1H-pyrrol-1-yl) benzoic acid | + |
| **Example 26** | 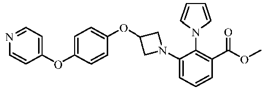 | methyl 3-(3-(4-(pyridin-4-yloxy)phenoxy)azetidin-1-y 1)-2-(1H-pyrrol-1-yl) benzoate | +++ |
| **Example 26a** | 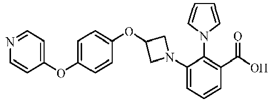 | 3-(3-(4-(pyridin-4-yloxy)phenoxy)azetidin-1-y 1)-2-(1H-pyrrol-1-yl) benzoic acid | ++ |
| **Example 27** | 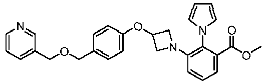 | methyl 3-(3-(4-((pyridin-3-ylmethoxy)methyl)phenox y)azetidin-1-yl)-2-(1H-pyrrol-1-yl) benzoate | ++ |
| **Example 27a** | 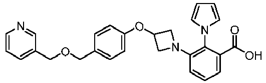 | 3 -(3-(4-((pyridin-3-ylmethoxy)methyl)phenox y)azetidin-1-yl)-2-(1H-pyrrol-1-yl) benzoic acid | + |
| **Example 27b** | 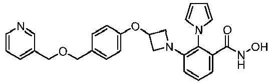 | N-Hydroxy-3-(3-(4-((pyridin-3-ylmethoxy)met hyl)phenoxy)azetidin-1-yl)-2-(1H-pyrrol-1-yl) benzamide | ++++ |
| **Example 28** | 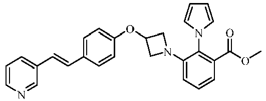 | methyl (E)-3-(3-(4-(2-(pyridin-3-yl)vinyl)phenoxy)aze tidin-1-yl)-2-(1H-pyrrol-1-yl) benzoate | + |
| **Example 28a** | 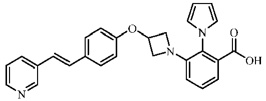 | (E)-3-(3-(4-(2-(pyridin-3-yl)vinyl)phenoxy)aze tidin-1-yl)-2-(1H-pyrrol-1-yl) benzoic acid | +++ |
| **Example 28b** | 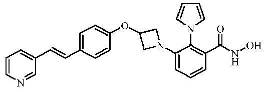 | (E)-N-hydroxy-3-(3-(4-(2-(pyridin-3-yl)vinyl) phenoxy)azetidin-1-yl)-2-(1H-pyrrol-1-yl) benzamide | ++++ |
| **Example 29** | 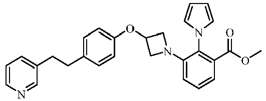 | methyl 3-(3-(4-(2-(pyridin-3-yl)ethyl)phenoxy)azetidi n-1-yl)-2-(1H-pyrrol-1-yl) benzoate | + |
| **Example 29a** | 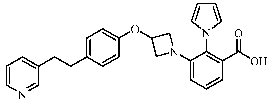 | 3-(3-(4-(2-(pyridin-3-yl)ethyl)phenoxy)azetidi n-1-yl)-2-(1H-pyrrol-1-yl) benzoic acid | + |
| **Example 30** | 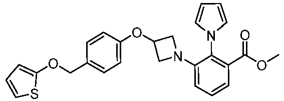 | methyl 2-(1H-pyrrol-1-yl)-3-(3-(4-((thiophen-2-yloxy) methyl)phenoxy)azetidin-1-yl) benzoate | + |
| **Example 30a** | 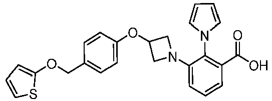 | 2-(1H-pyrrol-1-yl)-3-(3-(4-((thiophen-2-yloxy) methyl)phenoxy)azetidin-1-yl) benzoic acid | + |
| **Example 31** | 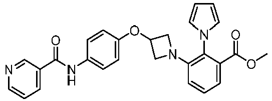 | methyl 3-(3-(4-(nicotinamido)phenoxy)azetidin-1-yl)-2-(1H-pyrrol-1-yl) benzoate | ++ |
| **Example 31a** | 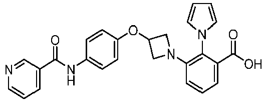 | 3-(3-(4-(nicotinoamido)phenoxy)azetidin-1-yl) -2-(1H-pyrrol-1-yl) benzoic acid | ++ |
| **Example 31b** | 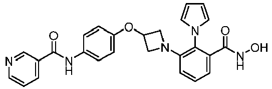 | N-(4-((1-(3-(hydroxycarbamoyl)-2-(1H-pyrrol-1-yl)phenyl)azetidin-3-yl)oxy)phenyl) nicotinamide | ++++ |
| **Example 32** | 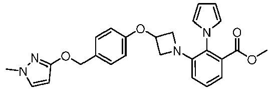 | methyl 3-(3-(4-(((1-methyl-1H-pyrazol-3-yl)oxy)meth yl)phenoxy)azetidin-1-yl)-2-(1H-pyrrol-1-yl) benzoate | + |
| **Example 32a** | 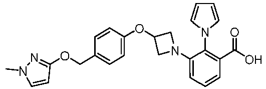 | 3-(3-(4-(((1-methyl-1H-pyrazol-3-yl)oxy)meth yl)phenoxy)azetidin-1-yl)-2-(1H-pyrrol-1-yl) benzoic acid | + |
| **Example 33** | 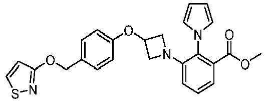 | methyl 3-(3-(4-((isothiazol-3-yloxy)methyl)phenoxy)a zetidin-1-yl)-2-(1H-pyrrol-1-yl) benzoate | + |
| **Example 33a** | 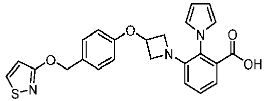 | 3-(3-(4-((isothiazol-3-yloxy)methyl)phenoxy)a zetidin-1-yl)-2-(1H-pyrrol-1-yl) benzoic acid | + |
| **Example 34** | 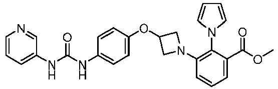 | methyl 3-(3-(4-(3-(pyridin-3-yl)ureido)phenoxy)azetid in-1-yl)-2-(1H-pyrrol-1-yl) benzoate | ++++ |
| **Example 34a** | 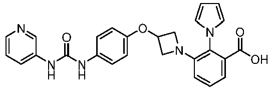 | 3-(3-(4-(3-(pyridin-3-yl)ureido)phenoxy)azetid in-1-yl)-2-(1H-pyrrol-1-yl) benzoic acid | +++ |
| **Example 34b** | 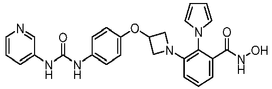 | N-hydroxy-3-(3-(4-(3-(pyridin-3-yl)ureido)phe noxy)azetidin-1-yl)-2-(1H-pyrrol-1-yl) benzamide | ++++ |
| **Example 35** | 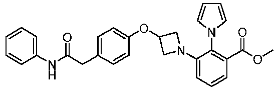 | methyl 3-(3-(4-(2-oxo-2-(phenylamino)ethyl)phenoxy) azetidin-1-yl)-2-(1H-pyrrol-1-yl) benzoate | + |
| **Example 35a** | 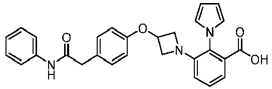 | 3-(3-(4-(2-oxo-2-(phenylamino)ethyl)phenoxy) azetidin-1-yl)-2-(1H-pyrrol-1-yl) benzoic acid | + |
| **Example 36** | 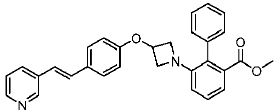 | methyl (E)-6-(3-(4-(2-(pyridin-3-yl)vinyl)phenoxy)aze tidin-1-yl)-[1,1'-biphenyl]-2-carboxylate | ++ |
| **Example 36a** | 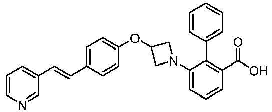 | (E)-6-(3-(4-(2-(pyridin-3-yl)vinyl)phenoxy)aze tidin-1-yl)-[1,1'-biphenyl]-2-carboxylic acid | ++ |
| **Example 36b** | 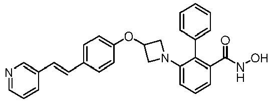 | (E)-N-hydroxy-6-(3-(4-(2-(pyridin-3-yl)vinyl) phenoxy)azetidin-1-yl)-[1,1'-biphenyl]-2-carbo xamide | ++++ |
| **Example 37** | 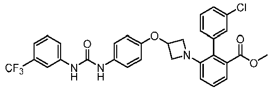 | methyl 3'-chloro-6-(3-(4-(3-(3-(trifluoromethyl)phenyl )ureido)phenoxy)azetidin-1-yl)-[1,1'-biphenyl] -2-carboxylate | ++ |
| **Example 37a** | 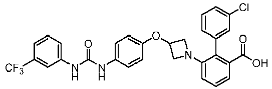 | 3'-chloro-6-(3-(4-(3-(3-(trifluoromethyl)phenyl )ureido)phenoxy)azetidin-1-yl)-[1,1'-biphenyl] -2-carboxylic acid | + |
| **Example 38** | 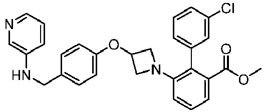 | methyl 3'-chloro-6-(3-(4-((pyridin-3-ylamino)methyl)p henoxy)azetidin-1-yl)-[1,1'-biphenyl]-2-carbox ylate | ++ |
| **Example 38a** | 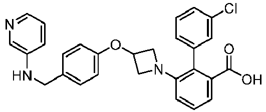 | 3'-chloro-6-(3-(4-((pyridin-3-ylamino)methyl)p henoxy)azetidin-1-yl)-[1,1'-biphenyl]-2-carbox ylic acid | ++ |
| **Example 38b** | 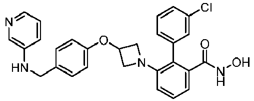 | 3'-chloro-N-hydroxy-6-(3-(4-((pyridin-3-ylami no)methyl)phenoxy)azetidin-1-yl)-[1,1'-biphen yl]-2-carboxamide | ++++ |
| **Example 39** | 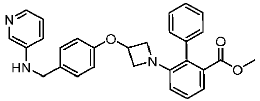 | methyl 6-(3-(4-((pyridin-3-ylamino)methyl)phenoxy)a zetidin-1-yl)-[1,1'-biphenyl]-2-carboxylate | + |
| **Example 39a** | 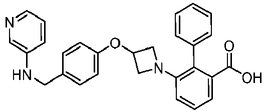 | 6-(3-(4-((pyridin-3-ylamino)methyl)phenoxy)a zetidin-1-yl)-[1,1'-biphenyl]-2-carboxylic acid | + |
| **Example 39b** | 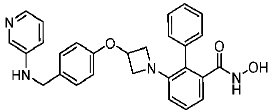 | N-hydroxy-6-(3-(4-((pyridin-3-ylamino)methyl )phenoxy)azetidin-1-yl)-[1,1'-biphenyl]-2-carb oxamide | ++++ |
| **Example 40** | 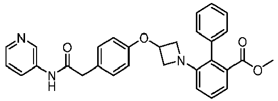 | methyl 6-(3-(4-(2-oxo-2-(pyridin-3-ylamino)ethyl)phe noxy)azetidin-1-yl)-[1,1'-biphenyl]-2-carboxyl ate | ++++ |
| **Example 40a** | 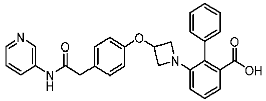 | 6-(3-(4-(2-oxo-2-(pyridin-3-ylamino)ethyl)phe noxy)azetidin-1-yl)-[1,1'-biphenyl]-2-carboxyli c acid | +++ |
| **Example 40b** | 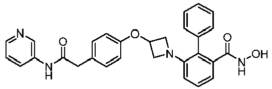 | N-hydroxy-6-(3-(4-(2-oxo-2-(pyridin-3-ylamin o)ethyl)phenoxy)azetidin-1-yl)-[1,1'-biphenyl]-2-carboxamide | ++++ |
| **Example 40c** | 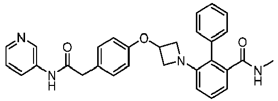 | N-methyl-6-(3-(4-(2-oxo-2-(pyridin-3-ylamino )ethyl)phenoxy)azetidin-1-yl)-[1,1'-biphenyl]-2 -carboxamide | +++ |
| **Example 41** | 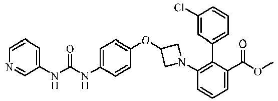 | methyl 3'-chloro-6-(3-(4-(3-(pyridin-3-yl)ureido)phen oxy)azetidin-1-yl)-[1,1'-biphenyl]-2-carboxylat e | ++++ |
| **Example 41a** | 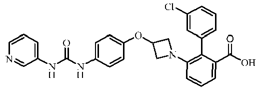 | 3'-chloro-6-(3-(4-(3-(pyridin-3-yl)ureido)phen oxy)azetidin-1-yl)-[1,1'-biphenyl]-2-carboxylic acid | +++ |
| **Example 41b** | 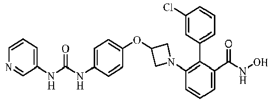 | 3'-chloro-N-hydroxy-6-(3-(4-(3-(pyridin-3-yl)u reido)phenoxy)azetidin-1-yl)-[1,1'-biphenyl]-2 -carboxamide | ++++ |
| **Example 42** | 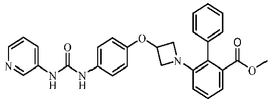 | methyl 6-(3-(4-(3-(pyridin-3-yl)ureido)phenoxy)azetid in-1-yl)-[1,1'-biphenyl]-2-carboxylate | ++++ |
| **Example 42a** | 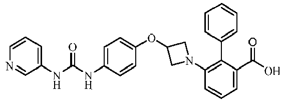 | 6-(3-(4-(3-(pyridin-3-yl)ureido)phenoxy)azetid in-1-yl)-[1,1'-biphenyl]-2-carboxylic acid | +++ |
| **Example 42b** | 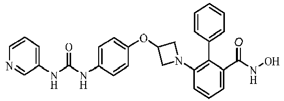 | N-hydroxy-6-(3-(4-(3-(pyridin-3-yl)ureido)phe noxy)azetidin-1-yl)-[1,1'-biphenyl]-2-carboxa mide | ++++ |
| **Example 42c** | 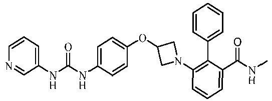 | N-methyl-6-(3-(4-(3-(pyridin-3-yl)ureido)phen oxy)azetidin-1-yl)-[1,1'-biphenyl]-2-carboxami de | ++++ |
| **Example 43** | 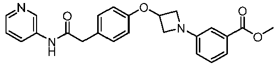 | methyl 3-(3-(4-(2-oxo-2-(pyridin-3-ylamino)ethyl)phe noxy)azetidin-1-yl) benzoate | +++ |
| **Example 43a** | 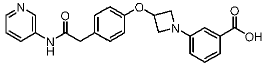 | 3-(3-(4-(2-oxo-2-(pyridin-3-ylamino)ethyl)phe noxy)azetidin-1-yl) benzoic acid | +++ |
| **Example 43b** | 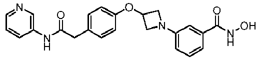 | N-hydroxy-3-(3-(4-(2-oxo-2-(pyridin-3-ylamin o)ethyl)phenoxy)azetidin-1-yl) benzamide | ++++ |
| **Example 43c** | 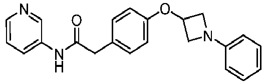 | 2-(4-((1-phenylazetidin-3-yl)oxy)phenyl)-N-(p yridin-3-yl) acetamide | ++ |
| **Example 44** | 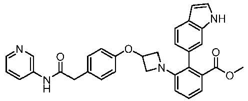 | methyl 2-(1H-indol-6-yl)-3-(3-(4-(2-oxo-2-(pyridin-3-ylamino)ethyl)phenoxy)azetidin-1-yl) benzoate | ++++ |
| **Example 44a** | 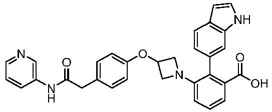 | 2-(1H-indol-6-yl)-3-(3-(4-(2-oxo-2-(pyridin-3-ylamino)ethyl)phenoxy)azetidin-1-yl) benzoic acid | +++ |
| **Example 45** | 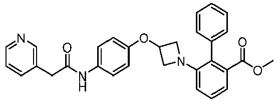 | methyl 6-(3-(4-(2-(pyridin-3-yl)acetamido)phenoxy)az etidin-1-yl)-[1,1'-biphenyl] -2-carboxylate | ++++ |
| **Example 45a** | 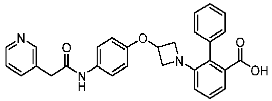 | 6-(3-(4-(2-(pyridin-3-yl)acetamido)phenoxy)az etidin-1-yl)-[1,1'-biphenyl]-2-carboxylic acid | ++ |
| **Example 45b** | 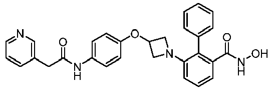 | N-hydroxy-6-(3-(4-(2-(pyridine-3-yl)acetamid o)phenoxy)azetidin-1-yl)-[1,1'-biphenyl]-2-car boxamide | ++++ |
| **Example 45c** | 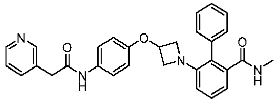 | N-methyl-6-(3-(4-(2-(pyridin-3-yl)acetamido)p henoxy)azetidin-1-yl)-[1,1'-biphenyl]-2-carbox amide | ++++ |
| **Example 45d** | 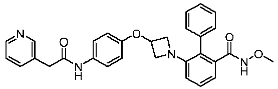 | N-methoxy-6-(3-(4-(2-(pyridin-3-yl)acetamido )phenoxy)azetidin-1-yl)-[1,1'-biphenyl]-2-carb oxamide | ++ |
| **Example 45e** | 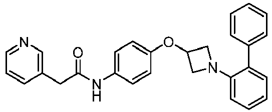 | N-(4-((1-([1,1'-biphenyl]-2-yl)azetidin-3-yl)ox y)phenyl)-2-(pyridin-3-yl)acetamide | ++ |
| **Example 46** | 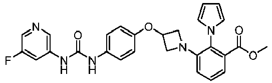 | methyl 3-(3-(4-(3-(5-fluoropyridin-3-yl)ureido)phenox y)azetidin-1-yl)-2-(1H-pyrrol-1-yl) benzoate | +++ |
| **Example 46a** | 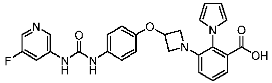 | 3-(3-(4-(3-(5-fluoropyridin-3-yl)ureido)phenox y)azetidin-1-yl)-2-(1H-pyrrol-1-yl) benzoic acid | +++ |
| **Example 47** | 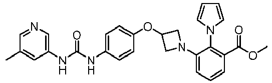 | methyl 3-(3-(4-(3-(5-methylpyridin-3-yl)ureido)pheno xy)azetidin-1-yl)-2-(1H-pyrrol-1-yl) benzoate | ++ |
| **Example 47a** | 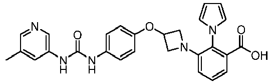 | 3-(3-(4-(3-(5-methylpyridin-3-yl)ureido)pheno xy)azetidin-1-yl)-2-(1H-pyrrol-1-yl) benzoic acid | ++++ |
| **Example 48** | 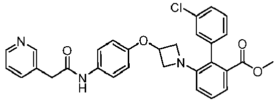 | methyl 3'-chloro-6-(3-(4-(2-(pyridin-3-yl)acetamido)p henoxy)azetidin-1-yl)-[1,1'-biphenyl]-2-carbox ylate | ++++ |
| **Example 48a** | 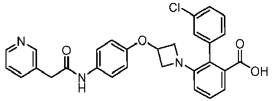 | 3'-chloro-6-(3-(4-(2-(pyridin-3-yl)acetamido)p henoxy)azetidin-1-yl)-[1,1'-biphenyl]-2-carbox ylic acid | ++ |
| **Example 49** | 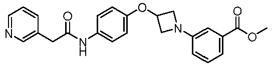 | methyl 3-(3-(4-(2-(pyridin-3-yl)acetamido)phenoxy)az etidin-1-yl) benzoate | +++ |
| **Example 49a** | 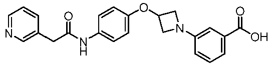 | 3-(3-(4-(2-(pyridin-3-yl)acetamido)phenoxy)az etidin-1-yl) benzoic acid | ++ |
| **Example 50** | 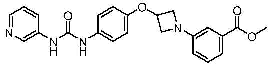 | methyl 3-(3-(4-(3-(pyridin-3-yl)ureido)phenoxy)azetid in-1-yl) benzoate | +++ |
| **Example 50a** | 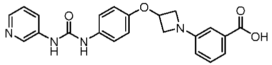 | 3-(3-(4-(3-(pyridin-3-yl)ureido)phenoxy)azetid in-1-yl) benzoic acid | +++ |
| **Example 51** | 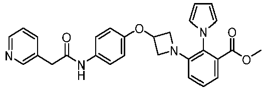 | methyl 3-(3-(4-(2-(pyridin-3-yl)acetamido)phenoxy)az etidin-1-yl)-2-(1H-pyrrol-1-yl) benzoate | ++++ |
| **Example 51a** | 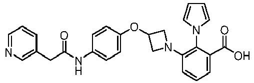 | 3-(3-(4-(2-(pyridin-3-yl)acetamido)phenoxy)az etidin-1-yl)-2-(1H-pyrrol-1-yl) benzoic acid | ++++ |
| **Example 52** | 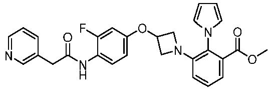 | methyl 3-(3-(3-fluoro-4-(2-(pyridin-3-yl)acetamido)ph enoxy)azetidin-1-yl)-2-(1H-pyrrol-1-yl) benzoate | ++++ |
| **Example 52a** | 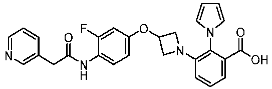 | 3-(3-(3-fluoro-4-(2-(pyridin-3-yl)acetamido)ph enoxy)azetidin-1-yl)-2-(1H-pyrrol-1-yl) benzoic acid | ++++ |
| **Example 53** | 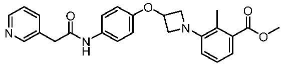 | methyl 2-methyl-3-(3-(4-(2-(pyridin-3-yl)acetamido)p henoxy)azetidin-1-yl) benzoate | ++ |
| **Example 53a** | 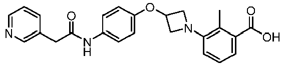 | 2-methyl-3-(3-(4-(2-(pyridin-3-yl)acetamido)p henoxy)azetidin-1-yl) benzoic acid | ++ |
| **Example 54** | 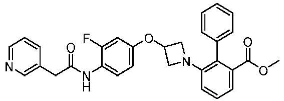 | methyl 6-(3-(3-fluoro-4-(2-(pyridin-3-yl)acetamido)ph enoxy)azetidin-1-yl)-[1,1'-biphenyl]-2-carboxy late | +++ |
| **Example 54a** | 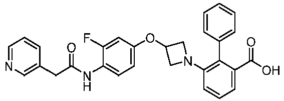 | 6-(3-(3-fluoro-4-(2-(pyridin-3-yl)acetamido)ph enoxy)azetidin-1-yl)-[1,1'-biphenyl]-2-carboxy lic acid | +++ |
| **Example 55** | 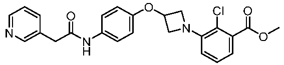 | methyl 2-chloro-3-(3-(4-(2-(pyridin-3-yl)acetamido)ph enoxy)azetidin-1-yl) benzoate | +++ |
| **Example 55a** | 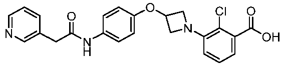 | 2-chloro-3-(3-(4-(2-(pyridin-3-yl)acetamido)ph enoxy)azetidin-1-yl) benzoic acid | +++ |
| **Example 56** | 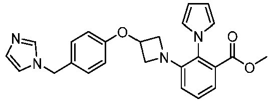 | methyl 3-(3-(4-((1H-imidazol-1-yl)methyl)phenoxy)az etidin-1-yl)-2-(1H-pyrrol-1-yl) benzoate | ++ |
| **Example 56a** | 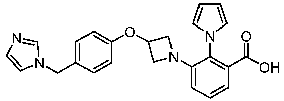 | 3-(3-(4-((1H-imidazol-1-yl)methyl)phenoxy)az etidin-1-yl)-2-(1H-pyrrol-1-yl) benzoic acid | ++ |
| **Example 57** | 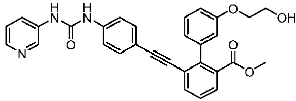 | methyl 3'-(2-hydroxyethoxy)-6-((4-(3-(pyridin-3-yl)ur eido)phenyl)ethynyl)-[1,1'-biphenyl]-2-carbox ylate | +++ |
| **Example 58** | 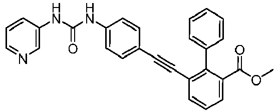 | methyl 6-((4-(3-(pyridin-3-yl)ureido)phenyl)ethynyl)-[ 1,1'-biphenyl]-2-carboxylate | ++++ |
| **Example 58a** | 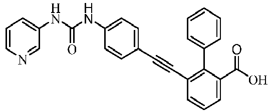 | 6-((4-(3-(pyridin-3-yl)ureido)phenyl)ethynyl)-[ 1,1'-biphenyl]-2-carboxylic acid | +++ |
| **Example 58b** | 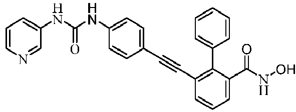 | N-hydroxy-6-((4-(3-(pyridin-3-yl)ureido)phen yl)ethynyl)-[1,1'-biphenyl]-2-carboxamide | ++++ |
| **Example 59** | 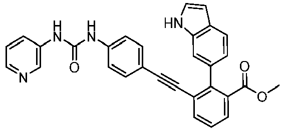 | methyl 2-(1H-indol-6-yl)-3-((4-(3-(pyridin-3-yl)ureido )phenyl)ethynyl) benzoate | ++++ |
| **Example 59a** | 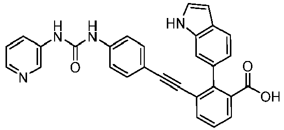 | 2-(1H-indol-6-yl)-3-((4-(3-(pyridin-3-yl)ureido )phenyl)ethynyl) benzoic acid, | ++++ |
| **Example 60** | 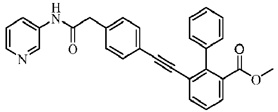 | methyl 6-((4-(2-oxo-2-(pyridin-3-ylamino)ethyl)pheny 1)ethynyl)-[1,1'-biphenyl]-2-carboxylate | ++++ |
| **Example 60a** | 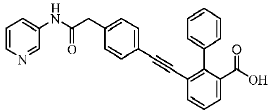 | 6-((4-(2-oxo-2-(pyridin-3-ylamino)ethyl)pheny 1)ethynyl)-[1,1'-biphenyl]-2-carboxylicacid | ++++ |
| **Example 60b** | 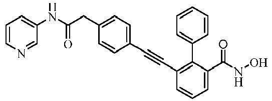 | N-hydroxy-6-((4-(2-oxo-2-(pyridin-3-ylamino) ethyl)phenyl)ethynyl)-[1,1'-biphenyl]-2-carbox amide | ++++ |
| **Example 61** | 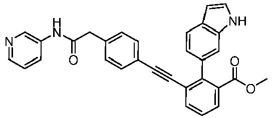 | methyl 2-(1H-indol-6-yl)-3-((4-(2-oxo-2-(pyridin-3-yl amino)ethyl)phenyl)ethynyl) benzoate | ++++ |
| **Example 61a** | 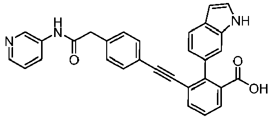 | 2-(1H-indol-6-yl)-3-((4-(2-oxo-2-(pyridin-3-yl amino)ethyl)phenyl)ethynyl) benzoic acid | ++++ |
| **Example 62** | 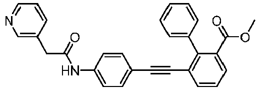 | methyl 6-((4-(2-(pyridin-3-yl)acetamido)phenyl)ethyn yl)-[1,1'-biphenyl]-2-carboxylate | ++++ |
| **Example 62a** | 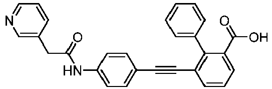 | 6-((4-(2-(pyridin-3-yl)acetamido)phenyl)ethyn yl)-[1,1'-biphenyl]-2-carboxylic acid | ++++ |
| **Example 63** | 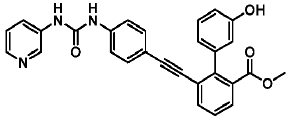 | methyl 3'-hydroxyl-6-((4-(3-(pyridin-3-yl)ureido)phen yl)ethynyl)-[1,1'-biphenyl]-2-carboxylate | ++++ |

| | | | |
|---|---|---|---|
| EC₅₀: < 1 uM ++++; 1 -10 uM +++; 10 - 50 uM ++; > 50 uM + | | | |

The data in the above table shows that the compounds of the present invention can effectively inhibit the proliferation vitality of C666-1 cells in a dose-dependent manner.

Although the technology has been described with reference to specific exemplary embodiments, it should be understood that the present invention as claimed should not be unduly limited to such specific embodiments. In fact, it will be apparent to those skilled in the art that other embodiments and modifications of this invention can be designed without departing from the essence and scope of the present invention. It is intended that the appended claims be interpreted to cover all such embodiments and equivalents.

## Claims

1. A compound of general formula (I), or its enantiomer, diastereomer, tautomer, salt, crystal form, solvate and/or isotope-substituted derivative: 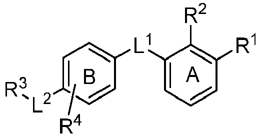 wherein:
R¹ is selected from -H, -COOH, -C(=O)-O-R^{1a}, -C(=O)-NHR^{1b}, and -C(=O)-NR^{1b}R^{1c};
wherein,
R^{1a}, R^{1b}, and R^{1c} are the same or different and are each independently selected from: hydrogen, optionally substituted C1-C4 linear alkyl, optionally substituted C3-C4 branched alkyl, optionally substituted C3-C4 cycloalkyl, optionally substituted halogenated C1-C4 linear alkyl, optionally substituted halogenated C3-C4 branched alkyl, and optionally substituted halogenated C3-C4 cycloalkyl; or,
R^{1b} is selected from: hydroxyl, and C1-C4 alkoxy; or,
R^{1b} and R^{1c}, taken together with atoms to which they are attached, form a cyclic group, the cyclic group being selected from: optionally substituted pyrrolidinyl, optionally substituted piperidinyl, optionally substituted piperazinyl, or optionally substituted morpholinyl;
R² is selected from the following groups:
hydrogen, halogen, optionally substituted C1-C4 alkyl, optionally substituted pyrrolyl, optionally substituted indolyl, and optionally substituted phenyl;
L¹ is selected from the following groups: 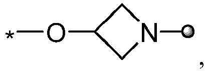 and ethynyl;
wherein the round dot 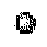 represents a junction where L¹ is linked to the A ring in the compound of general formula (I), the A ring being located on a right side of L¹;
wherein the asterisk * represents a junction where L¹ is linked to the B ring in the compound of general formula (I), the B ring being located on a left side of L¹;
L² is selected from the following groups:
•-(CH₂)_{q}-O-(CH₂)ₚ-*, •-(CH₂)_{q}-NH-(CH₂)ₚ-*, •-NH-C(=O)-NH-*, •-N(CH₃)-C(=O)-NH-*, -CH=CH-, -(CH₂)ₙ-, •-NH-C(=O)-(CH₂)ₚ-* and •-(CH₂)_{q}-C(=O)-NH-*;
preferably, L² is selected from: •-CH₂-O-*, •-O-CH₂-*, -O-, -CH₂-O-CH₂-, •-CH₂-NH-*, •-CH₂-NH-CH₂-*, •-NH-CH₂-*, -NH-C(=O)-NH-, •-N(CH₃)-C(=O)-NH-*, -CH=CH-, -CH₂-, -CH₂-CH₂-, •-NH-C(=O)-*, •-NH-C(=O)-CH₂-*, •-C(=O)-NH-*, and •-CH₂-C(=O)-NH-*;
wherein the round dot • represents a junction where L² is linked to the B ring in the compound of general formula (I), the B ring being located on a right side of L²;
wherein the asterisk * represents a junction where L² is linked to R³ in the compound of general formula (I) , the R³ being located on a left side of L²;
p and q are each independently 0 or 1 or 2;
n is 1 or 2 or 3;
R³ is selected from: optionally substituted aryl and heteroaryl;
the aryl is selected from: phenyl;
the heteroaryl is selected from: thienyl, pyrazolyl, imidazolyl, isothiazolyl, pyridyl, pyrimidyl, pyrazinyl, and quinolinyl;
the aryl and heteroaryl are optionally substituted by hydrogen, fluorine, chlorine, cyano, C1-C4 alkyl, halogenated C1-C4 alkyl, C1-C4 alkoxy, or halogenated C1-C4 alkoxy one or more times identically or differently;
R⁴ is selected from: hydrogen, halogen, optionally substituted C1-C4 linear alkyl, optionally substituted C3-C4 branched alkyl, optionally substituted halogenated C1-C4 linear alkyl, optionally substituted halogenated C3-C4 branched alkyl, and hydroxyl.

2. The compound, or its enantiomer, diastereomer, tautomer, salt, crystal form, solvate and/or isotope-substituted derivative according to claim 1, wherein:
when L² is selected from -(CH₂)_{q}-O-(CH₂)ₚ-,
R³ is selected from aryl, and heteroaryl;
the aryl is selected from: phenyl;
the heteroaryl is selected from: thienyl, pyrazolyl, isothiazolyl, pyridyl, pyrimidyl, pyrazinyl, and quinolinyl;
the aryl and heteroaryl are optionally substituted by hydrogen, C1-C4 alkyl, or C1-C4 alkoxy one or more times identically or differently;
or
when L² is selected from -(CH₂)_{q}-NH-(CH₂)ₚ-, •-NH-C(=O)-(CH₂)ₚ-*, •-(CH₂)_{q}-C(=O)-NH-*, and -CH=CH-,
R³ is selected from aryl, and heteroaryl;
the aryl is selected from: phenyl;
the heteroaryl is selected from: pyridyl;
or
when L² is selected from -NH-C(=O)-NH- and •-N(CH₃)-C(=O)-NH-*,
R³ is selected from aryl and heteroaryl;
the aryl is selected from: phenyl;
the heteroaryl is selected from: pyridyl;
the aryl and heteroaryl are substituted by hydrogen, fluorine, cyano, C1-C4 alkyl, or halogenated C1-C4 alkyl one or more times identically or differently;
or
when L² is selected from -(CH₂)ₙ-;
R³ is selected from aryl and heteroaryl;
the aryl is selected from: phenyl;
the heteroaryl is selected from: pyridyl and imidazolyl.

3. The compound, or its enantiomer, diastereomer, tautomer, salt, crystal form, solvate and/or isotope-substituted derivative according to claim 1, wherein:
when L¹ is selected from oxy-azetidinyl,
R² is selected from: hydrogen, halogen, optionally substituted C1-C4 alkyl, optionally substituted pyrrolyl, optionally substituted indolyl, and optionally substituted phenyl;
L² is selected from: -(CH₂)_{q}-O-(CH₂)ₚ-, -(CH₂)_{q}-NH-(CH₂)ₚ-, -NH-C(=O)-NH-, •-N(CH₃)-C(=O)-NH-*, -CH=CH-, -(CH₂)ₙ-, •-NH-C(=O)-(CH₂)ₚ-*, and •-(CH₂)_{q}-C(=O)-NH-*;
R³ is selected from: phenyl, thienyl, pyrazolyl, imidazolyl, isothiazolyl, pyridyl, pyrimidinyl, pyrazinyl, quinolinyl, cyanophenyl, trifluoromethylphenyl, fluoropyridyl, methylpyridyl, methoxypyridyl, and methylpyrazolyl.

4. The compound, or its enantiomer, diastereomer, tautomer, salt, crystal form, solvate and/or isotope-substituted derivative according to claim 1, wherein:
when L¹ is selected from ethynyl,
R² is selected from: optionally substituted indolyl and optionally substituted phenyl;
L² is selected from: -(CH₂)_{q}-O-(CH₂)ₚ-, -NH-C(=O)-NH-, •-N(CH₃)-C(=O)-NH-*, •-NH-C(=O)-(CH₂)ₚ-*, and •-(CH₂)_{q}-C(=O)-NH-*;
R³ is selected from: pyridyl, methylpyridyl, and methoxypyridyl.

5. The compound, or its enantiomer, diastereomer, tautomer, salt, crystal form, solvate and/or isotope-substituted derivative according to claim 1, wherein the compound is selected from:
methyl 3 -(3 -(4-((pyridin-3 -yloxy)methyl)phenoxy)azetidin-1 -yl)-2-(1H-pyrrol-1 -yl) benzoate, 3-(3-(4-((pyridin-3-yloxy)methyl)phenoxy)azetidin-1-yl)-2-(1H-pyrrol-1-yl) benzoic acid, ethyl 3-(3-(4-((pyridin-3-yloxy)methyl)phenoxy)azetidin-1-yl)-2-(1H-pyrrol-1-yl) benzoate, N-hydroxy-3-(3-(4-((pyridin-3-yloxy)methyl)phenoxy)azetidin-1-yl)-2-(1H-pyrrol-1-yl) benzamide, N-methyl-3-(3-(4-((pyridin-3-yloxy)methyl)phenoxy)azetidin-1-yl)-2-(1H-pyrrol-1-yl) benzamide, N,N-dimethyl-3-(3-(4-((pyridin-3-yloxy)methyl)phenoxy)azetidin-1-yl)-2-(1H-pyrrol-1-yl) benzamide, (3-(3-(4-((pyridin-3-yloxy)methyl)phenoxy)azetidin-1-yl)-2-(1H-pyrrol-1-yl)phenyl)(pyrrolid in-1-yl) methanone, 3-((4-((1-(2-(1H-pyrrole-1-yl)phenyl)azetidin-3-yl)oxy)benzyl)oxy) pyridine, methyl 3 -(3 -(4-((4-methylpyridin-3 -yloxy)methyl)phenoxy)azetidin-1-yl)-2-(1H-pyrrol-1-yl) benzoate, 3-(3-(4-((4-methylpyridin-3-yloxy)methyl)phenoxy)azetidin-1-yl)-2-(1H-pyrrol-1-yl) benzoic acid, methyl 3-(3-(4-((5-methylpyridin-3-yloxy)methyl)phenoxy)azetidin-1-yl)-2-(1H-pyrrol-1-yl) benzoate, 3-(3-(4-((5-methylpyridin-3-yloxy)methyl)phenoxy)azetidin-1-yl)-2-(1H-pyrrol-1-yl) benzoic acid, methyl 3-(3-(4-(((6-methoxypyridin-3-yl)oxy)methyl)phenoxy)azetidin-1-yl)-2-(1H-pyrrole-1-yl) benzoate, 3-(3-(4-(((6-methoxypyridin-3-yl)oxy)methyl)phenoxy)azetidin-1-yl)-2-(1H-pyrrol-1-yl) benzoic acid, methyl 3-(3-(4-((6-methylpyridin-3-yloxy)methyl)phenoxy)azetidin-1-yl)-2-(1H-pyrrol-1-yl) benzoate, 3-(3-(4-((6-methylpyridin-3-yloxy)methyl)phenoxy)azetidin-1-yl)-2-(1H-pyrrol-1-yl) benzoic acid, methyl 3-(3-(4-(((5-methoxypyridin-3-yl)oxy)methyl)phenoxy)azetidin-1-yl)-2-(1H-pyrrol-1-yl) benzoate, 3-(3-(4-(((5-methoxypyridin-3-yl)oxy)methyl)phenoxy)azetidin-1-yl)-2-(1H-pyrrol-1-yl) benzoic acid, methyl 3-(3-(4-(3-(3-cyanophenyl)-1-methylureido)phenoxy)azetidin-1-yl)-2-(1H-pyrrol-1-yl) benzoate, 3-(3-(4-(3-(3-cyanophenyl)-1-methylureido)phenoxy)azetidin-1-yl)-2-(1H-pyrrole-1-yl) benzoic acid, methyl 3-(3-(4-((pyridin-4-yloxy)methyl)phenoxy)azetidin-1-yl)-2-(1H-pyrrole-1-yl) benzoate, 3-(3-(4-((pyridin-4-yloxy)methyl)phenoxy)azetidin-1-yl)-2-(1H-pyrrole-1-yl) benzoic acid, methyl 2-(1H-pyrrol-1-yl)-3-(3-(4-((quinolin-3-yloxy)methyl)phenoxy)azetidin-1-yl) benzoate, 2-(1H-pyrrol-1 -yl)-3 -(3 -(4-((quinolin-3 -yloxy)methyl)phenoxy)azetidin-1 -yl) benzoic acid, methyl 2-(1H-pyrrol-1-yl)-3-(3-(4-(3-(3-(trifluoromethyl)phenyl)ureido)phenoxy)azetidin-1-yl) benzoate, 2-(1H-pyrrol-1-yl)-3-(3-(4-(3-(3-(trifluoromethyl)phenyl)ureido)phenoxy)azetidin-1-yl) benzoic acid, N-hydroxy-2-(1 H-pyrrol-1-yl)-3-(3-(4-(3-(3-(trifluoromethyl)phenyl)ureido)phenoxy)azetidin -1-yl) benzamide, methyl 3 -(3 -(4-((pyrimidin-2-yloxy)methyl)phenoxy)azetidin-1 -yl)-2-(1H-pyrrol-1 -yl) benzoate, 3-(3-(4-((pyrimidin-2-yloxy)methyl)phenoxy)azetidin-1-yl)-2-(1H-pyrrol-1-yl) benzoic acid, methyl 3-(3-(4-((pyridin-2-yloxy)methyl)phenoxy)azetidin-1-yl)-2-(1H-pyrrol-1-yl) benzoate, 3-(3-(4-((pyridin-2-yloxy)methyl)phenoxy)azetidin-1-yl)-2-(1H-pyrrol-1-yl) benzoic acid, methyl 3-(3-(4-((pyrazin-2-yloxy)methyl)phenoxy)azetidin-1-yl)-2-(1H-pyrrol-1-yl) benzoate, 3-(3-(4-((pyrazin-2-yloxy)methyl)phenoxy)azetidin-1-yl)-2-(1H-pyrrol-1-yl) benzoic acid, methyl 3 -(3 -(4-((pyrimidin-5 -yloxy)methyl)phenoxy)azetidin-1 -yl)-2-(1H-pyrrol-1 -yl) benzoate, 3-(3-(4-((pyrimidin-5-yloxy)methyl)phenoxy)azetidin-1-yl)-2-(1H-pyrrol-1-yl) benzoic acid, N-hydroxy-3-(3-(4-((pyrimidin-5-oxy)methyl)phenoxy)azetidin-1-yl)-2-(1H-pyrrol-1-yl) benzamide, methyl 3-(3-(3-methyl-4-((pyridin-3-yloxy)methyl)phenoxy)azetidin-1-yl)-2-(1H-pyrrol-1 -yl) benzoate, 3-(3-(3-methyl-4-((pyridin-3-yloxy)methyl)phenoxy)azetidin-1 -yl)-2-(1H-pyrrol-1 -yl) benzoic acid, methyl 3-(3-(2-methyl-4-((pyridin-3-yloxy)methyl)phenoxy)azetidin-1 -yl)-2-(1H-pyrrol-1 -yl) benzoate, 3-(3-(2-methyl-4-((pyridin-3-yloxy)methyl)phenoxy)azetidin-1 -yl)-2-(1H-pyrrol-1 -yl) benzoic acid, methyl 6-(3-(4-((pyridin-3-yloxy)methyl)phenoxy)azetidin-1-yl)-[1,1'-biphenyl]-2-carboxylate, 6-(3-(4-((pyridin-3-yloxy)methyl)phenoxy)azetidin-1-yl)-[1,1'-biphenyl]-2-carboxylic acid, N-hydroxy-6-(3-(4-((pyridin-3-yloxy)methyl) phenoxy)azetidin-1-yl)-[1,1'-biphenyl]-2-carboxamide, methyl 3'-chloro-6-(3-(4-((pyridin-3-yloxy)methyl)phenoxy)azetidin-1-yl)-[1,1'-biphenyl]-2-carboxyl ate, methyl 3-(3-(3-chloro-4-((pyridin-3-yloxy)methyl)phenoxy)azetidin-1-yl)-2-(1H-pyrrol-1-yl) benzoate, 3-(3-(3-chloro-4-((pyridin-3-yloxy)methyl)phenoxy)azetidin-1-yl)-2-(1H-pyrrol-1-yl) benzoic acid, methyl 3-(3-(3-fluoro-4-((pyridin-3-yloxy)methyl)phenoxy)azetidin-1-yl)-2-(1H-pyrrol-1-yl) benzoate, 3 -(3 -(3 -fluoro-4-((pyridin-3 -yloxy)methyl)phenoxy)azetidin-1-yl)-2-(1H-pyrrole-1 -yl) benzoic acid, methyl 3-(3-(3-((pyridin-3-yloxy)methyl)phenoxy)azetidin-1-yl)-2-(1H-pyrrole-1-yl) benzoate, methyl 6-((3-hydroxy-4-(3-(pyridin-3-yl)ureido)phenyl)ethynyl)-[1,1'-biphenyl]-2-carboxylate, methyl 3-(3-(4-((pyridin-3-ylamino)methyl)phenoxy)azetidin-1-yl)-2-(1H-pyrrole-1 -yl) benzoate, 3-(3-(4-((pyridin-3-ylamino)methyl)phenoxy)azetidin-1-yl)-2-(1H-pyrrole-1 -yl) benzoic acid, N-hydroxy-3-(3-(4-((pyridin-3-ylamino)methyl)phenoxy)azetidin-1-yl)-2-(1H-pyrrole-1-yl) benzamide, methyl 3-(3-(4-(pyridin-3-ylmethoxy)phenoxy)azetidin-1-yl)-2-(1H-pyrrole-1-yl) benzoate, 3-(3-(4-(pyridin-3-ylmethoxy)phenoxy)azetidin-1-yl)-2-(1H-pyrrole-1-yl) benzoic acid, N-hydroxy-3-(3-(4-(pyridin-3-ylmethoxy)phenoxy)azetidin-1-yl)-2-(1H-pyrrole-1-yl) benzamide, methyl 3-(3-(4-(pyridin-3-yloxy)phenoxy)azetidin-1-yl)-2-(1H-pyrrol-1-yl) benzoate, 3-(3-(4-(pyridin-3-yloxy)phenoxy)azetidin-1-yl)-2-(1H-pyrrol-1-yl) benzoic acid, methyl 3-(3-(4-(pyridin-4-yloxy)phenoxy)azetidin-1-yl)-2-(1H-pyrrol-1-yl) benzoate, 3-(3-(4-(pyridin-4-yloxy)phenoxy)azetidin-1-yl)-2-(1H-pyrrol-1-yl) benzoic acid, methyl 3-(3-(4-((pyridin-3-ylmethoxy)methyl)phenoxy)azetidin-1-yl)-2-(1H-pyrrol-1-yl) benzoate, 3-(3-(4-((pyridin-3-ylmethoxy)methyl)phenoxy)azetidin-1-yl)-2-(1H-pyrrol-1-yl) benzoic acid, N-hydroxy-3-(3-(4-((pyridin-3-ylmethoxy)methyl)phenoxy)azetidin-1-yl)-2-(1H-pyrrol-1-yl) benzamide, methyl (E)-3-(3-(4-(2-(pyridin-3-yl)vinyl)phenoxy)azetidin-1-yl)-2-(1H-pyrrole-1-yl) benzoate, (E)-3-(3-(4-(2-(pyridin-3-yl)vinyl)phenoxy)azetidin-1-yl)-2-(1H-pyrrol-1-yl) benzoic acid, (E)-N-hydroxy-3-(3-(4-(2-(pyridin-3-yl)vinyl)phenoxy)azetidin-1-yl)-2-(1H-pyrrol-1-yl) benzamide, methyl 3-(3-(4-(2-(pyridin-3-yl)ethyl)phenoxy)azetidin-1-yl)-2-(1H-pyrrol-1-yl) benzoate, 3-(3-(4-(2-(pyridin-3-yl)ethyl)phenoxy)azetidin-1-yl)-2-(1H-pyrrol-1-yl) benzoic acid, methyl 2-(1H-pyrrol-1 -yl)-3-(3-(4-((thiophen-2-yloxy)methyl)phenoxy)azetidin-1 -yl) benzoate, 2-(1H-pyrrol-1 -yl)-3-(3-(4-((thiophen-2-yloxy)methyl)phenoxy)azetidin-1 -yl) benzoic acid, methyl 3-(3-(4-(nicotinamido)phenoxy)azetidin-1-yl)-2-(1H-pyrrol-1-yl) benzoate, 3-(3-(4-(nicotinamido)phenoxy)azetidin-1-yl)-2-(1H-pyrrol-1-yl) benzoic acid, N-(4-((1-(3-(hydroxycarbamoyl)-2-(1H-pyrrol-1-yl)phenyl)azetidin-3-yl)oxy)phenyl) nicotinamide, methyl 3-(3-(4-(((1-methyl-1H-pyrazol-3-yl)oxy)methyl)phenoxy)azetidin-1-yl)-2-(1H-pyrrol-1-yl) benzoate, 3-(3-(4-(((1-methyl-1H-pyrazol-3-yl)oxy)methyl)phenoxy)azetidin-1-yl)-2-(1H-pyrrol-1-yl) benzoic acid, methyl 3-(3-(4-((isothiazol-3-yloxy)methyl)phenoxy)azetidin-1-yl)-2-(1H-pyrrol-1-yl) benzoate, 3-(3-(4-((isothiazol-3-yloxy)methyl)phenoxy)azetidin-1-yl)-2-(1H-pyrrol-1-yl) benzoic acid, methyl 3-(3-(4-(3-(pyridin-3-yl)ureido)phenoxy)azetidin-1-yl)-2-(1H-pyrrol-1-yl) benzoate, 3-(3-(4-(3-(pyridin-3-yl)ureido)phenoxy)azetidin-1-yl)-2-(1H-pyrrol-1-yl) benzoic acid, N-hydroxy-3-(3-(4-(3-(pyridin-3-yl)ureido)phenoxy)azetidin-1-yl)-2-(1H-pyrrol-1-yl) benzamide, methyl 3-(3-(4-(2-oxo-2-(phenylamino)ethyl)phenoxy)azetidin-1-yl)-2-(1H-pyrrol-1-yl) benzoate, 3-(3-(4-(2-oxo-2-(phenylamino)ethyl)phenoxy)azetidin-1-yl)-2-(1H-pyrrol-1-yl) benzoic acid, methyl (E)-6-(3-(4-(2-(pyridin-3-yl)vinyl)phenoxy)azetidin-1-yl)-[1,1'-biphenyl]-2-carboxylate, (E)-6-(3-(4-(2-(pyridin-3-yl)vinyl)phenoxy)azetidin-1-yl)-[1,1'-biphenyl]-2-carboxylic acid, (E)-N-hydroxy-6-(3-(4-(2-(pyridin-3-yl)vinyl)phenoxy)azetidin-1-yl)-[1,1'-biphenyl]-2-carbo xamide, methyl 3'-chloro-6-(3-(4-(3-(3-(trifluoromethyl)phenyl)ureido)phenoxy)azetidin-1-yl)-[1,1'-biphenyl] -2-carboxylate, 3'-chloro-6-(3-(4-(3-(3-(trifluoromethyl)phenyl)ureido)phenoxy)azetidin-1-yl)-[1,1'-biphenyl] -2-carboxylic acid, methyl 3'-chloro-6-(3-(4-((pyridin-3-ylamino)methyl)phenoxy)azetidin-1-yl)-[1,1'-biphenyl]-2-carbo xylate, 3'-chloro-6-(3-(4-((pyridin-3-ylamino)methyl)phenoxy)azetidin-1-yl)-[1,1'-biphenyl]-2-carbo xylic acid, 3'-chloro-N-hydroxy-6-(3-(4-((pyridin-3-ylamino)methyl)phenoxy)azetidin-1-yl)-[1,1'-biphen yl]-2-carboxamide, methyl 6-(3-(4-((pyridin-3-ylamino)methyl)phenoxy)azetidin-1-yl)-[1,1'-biphenyl]-2-carboxylate, 6-(3-(4-((pyridin-3-ylamino)methyl)phenoxy)azetidin-1-yl)-[1,1'-biphenyl]-2-carboxylic acid, N-hydroxy-6-(3-(4-((pyridin-3-ylamino)methyl)phenoxy)azetidin-1-yl)-[1,1'-biphenyl]-2-carb oxamide, methyl 6-(3-(4-(2-oxo-2-(pyridin-3-ylamino)ethyl)phenoxy)azetidin-1-yl)-[1,1'-biphenyl]-2-carboxyl ate, 6-(3-(4-(2-oxo-2-(pyridin-3-ylamino)ethyl)phenoxy)azetidin-1-yl)-[1,1'-biphenyl]-2-carboxyli c acid, N-hydroxy-6-(3-(4-(2-oxo-2-(pyridine-3-ylamino)ethyl)phenoxy)azetidin-1-yl)-[1,1'-biphenyl ]-2-carboxamide, N-methyl-6-(3-(4-(2-oxo-2-(pyridin-3-ylamino)ethyl)phenoxy)azetidin-1-yl)-[1,1'-biphenyl]-2-carboxamide, methyl 3'-chloro-6-(3-(4-(3-(pyridin-3-yl)ureido)phenoxy)azetidin-1-yl)-[1,1'-biphenyl]-2-carboxylat e, 3'-chloro-6-(3-(4-(3-(pyridin-3-yl)ureido)phenoxy)azetidin-1-yl)-[1,1'-biphenyl]-2-carboxylic acid, 3'-chloro-N-hydroxy-6-(3-(4-(3-(pyridin-3-yl)ureido)phenoxy)azetidin-1-yl)-[1,1'-biphenyl]-2 -carboxamide, methyl 6-(3-(4-(3-(pyridin-3-yl)ureido)phenoxy)azetidin-1 -yl)-[1,1 '-biphenyl]-2-carboxylate, 6-(3-(4-(3-(pyridin-3-yl)ureido)phenoxy)azetidin-1-yl)-[1,1'-biphenyl]-2-carboxylic acid, N-hydroxy-6-(3-(4-(3-(pyridin-3-yl)ureido)phenoxy)azetidin-1 -yl)-[1,1'-biphenyl]-2-carboxa mide, N-methyl-6-(3-(4-(3-(pyridine-3-yl)ureido)phenoxy)azetidin-1-yl)-[1,1'-biphenyl]-2-carboxa mide, methyl 3-(3-(4-(2-oxo-2-(pyridin-3-ylamino)ethyl)phenoxy)azetidin-1-yl) benzoate, 3-(3-(4-(2-oxo-2-(pyridin-3-ylamino)ethyl)phenoxy)azetidin-1-yl) benzoic acid, N-hydroxy-3-(3-(4-(2-oxo-2-(pyridin-3-ylamino)ethyl)phenoxy)azetidin-1-yl) benzamide, 2-(4-((1-phenylazetidin-3-yl)oxy)phenyl)-N-(pyridin-3-yl) acetamide, methyl 2-(1H-indol-6-yl)-3 -(3 -(4-(2-oxo-2-(pyridin-3 -ylamino)ethyl)phenoxy)azetidin-1 -yl) benzoate, 2-(1H-indol-6-yl)-3-(3-(4-(2-oxo-2-(pyridine-3-ylamino)ethyl)phenoxy)azetidin-1-yl) benzoic acid, methyl 6-(3-(4-(2-(pyridin-3-yl)acetamido)phenoxy)azetidin-1-yl)-[1,1 '-biphenyl]-2-carboxylate, 6-(3-(4-(2-(pyridin-3-yl)acetamido)phenoxy)azetidin-1-yl)-[1,1'-biphenyl]-2-carboxylic acid, N-hydroxy-6-(3-(4-(2-(pyridin-3-yl)acetamido)phenoxy)azetidin-1-yl)-[1,1'-biphenyl]-2-carb oxamide, N-methyl-6-(3-(4-(2-(pyridin-3-yl)acetamido)phenoxy)azetidin-1-yl)-[1,1'-biphenyl]-2-carbo xamide, N-methoxy-6-(3-(4-(2-(pyridin-3-yl)acetamido)phenoxy)azetidin-1-yl)-[1,1'-biphenyl]-2-carb oxamide, N-(4-((1-([1,1'-biphenyl]-2-yl)azetidin-3-yl)oxy)phenyl)-2-(pyridin-3-yl)acetamide, methyl 3 -(3 -(4-(3 -(5 -fluoropyridin-3 -yl)ureido)phenoxy)azetidin-1 -yl)-2-(1H-pyrrol-1 -yl) benzoate, 3-(3-(4-(3-(5-fluoropyridin-3-yl)ureido)phenoxy)azetidin-1-yl)-2-(1H-pyrrol-1-yl) benzoic acid, methyl 3-(3-(4-(3-(5-methylpyridin-3-yl)ureido)phenoxy)azetidin-1-yl)-2-(1H-pyrrol-1-yl) benzoate, 3-(3-(4-(3-(5-methylpyridin-3-yl)ureido)phenoxy)azetidin-1-yl)-2-(1H-pyrrol-1-yl) benzoic acid, methyl 3'-chloro-6-(3-(4-(2-(pyridin-3-yl)acetamido)phenoxy)azetidin-1-yl)-[1,1'-biphenyl]-2-carbox ylate, 3'-chloro-6-(3-(4-(2-(pyridin-3-yl)acetamido)phenoxy)azetidin-1-yl)-[1,1'-biphenyl]-2-carbox ylic acid, methyl 3-(3-(4-(2-(pyridin-3-yl)acetamido)phenoxy)azetidin-1-yl) benzoate, 3-(3-(4-(2-(pyridin-3-yl)acetamido)phenoxy)azetidin-1-yl) benzoic acid, methyl 3-(3-(4-(3-(pyridin-3-yl)ureido)phenoxy)azetidin-1-yl) benzoate, 3-(3-(4-(3-(pyridin-3-yl)ureido)phenoxy)azetidin-1-yl) benzoic acid, methyl 3-(3-(4-(2-(pyridin-3-yl)acetamido)phenoxy)azetidin-1-yl)-2-(1H-pyrrol-1-yl) benzoate, 3-(3-(4-(2-(pyridin-3-yl)acetamido)phenoxy)azetidin-1-yl)-2-(1H-pyrrol-1-yl) benzoic acid, methyl 3 -(3 -(3 -fluoro-4-(2-(pyridin-3 -yl)acetamido)phenoxy)azetidin-1-yl)-2-(1H-pyrrol-1 -yl) benzoate, 3-(3-(3-fluoro-4-(2-(pyridin-3-yl)acetamido)phenoxy)azetidin-1-yl)-2-(1H-pyrrol-1-yl) benzoic acid, methyl 2-methyl-3-(3-(4-(2-(pyridin-3-yl)acetamido)phenoxy)azetidin-1-yl) benzoate, 2-methyl-3-(3-(4-(2-(pyridin-3-yl)acetamido)phenoxy)azetidin-1-yl) benzoic acid, methyl 6-(3-(3-fluoro-4-(2-(pyridin-3-yl)acetamido)phenoxy)azetidin-1 -yl)-[1,1'-biphenyl]-2-carboxy late, 6-(3-(3-fluoro-4-(2-(pyridin-3-yl)acetamido)phenoxy)azetidin-1-yl)-[1,1'-biphenyl]-2-carboxy lic acid, methyl 2-chloro-3-(3-(4-(2-(pyridin-3-yl)acetamido)phenoxy)azetidin-1-yl) benzoate, 2-chloro-3-(3-(4-(2-(pyridin-3-yl)acetamido)phenoxy)azetidin-1-yl) benzoic acid, methyl 3-(3-(4-((1H-imidazol-1-yl)methyl)phenoxy)azetidin-1-yl)-2-(1H-pyrrol-1-yl) benzoate, 3-(3-(4-((1H-imidazol-1-yl)methyl)phenoxy)azetidin-1-yl)-2-(1H-pyrrol-1-yl) benzoic acid, methyl 3'-(2-hydroxyethoxy)-6-((4-(3-(pyridin-3-yl)ureido)phenyl)ethynyl)-[1,1'-biphenyl]-2-carbox ylate, methyl 6-((4-(3-(pyridin-3-yl)ureido)phenyl)ethynyl)-[1,1'-biphenyl]-2-carboxylate, 6-((4-(3-(pyridin-3-yl)ureido)phenyl)ethynyl)-[1,1'-biphenyl]-2-carboxylic acid, N-hydroxy-6-((4-(3-(pyridin-3-yl)ureido)phenyl)ethynyl)-[1,1'-biphenyl]-2-carboxamide, methyl 2-(1H-indol-6-yl)-3-((4-(3-(pyridin-3-yl)ureido)phenyl)ethynyl) benzoate, 2-(1H-indol-6-yl)-3-((4-(3-(pyridin-3-yl)ureido)phenyl)ethynyl) benzoic acid, methyl 6-((4-(2-oxo-2-(pyridin-3-ylamino)ethyl)phenyl)ethynyl)-[1,1'-biphenyl]-2-carboxylate, 6-((4-(2-oxo-2-(pyridin-3-ylamino)ethyl)phenyl)ethynyl)-[1,1'-biphenyl]-2-carboxylic acid, N-hydroxy-6-((4-(2-oxo-2-(pyridin-3-ylamino)ethyl)phenyl)ethynyl)-[1,1'-biphenyl]-2-carbox amide, methyl 2-(1H-indol-6-yl)-3-((4-(2-oxo-2-(pyridin-3-ylamino)ethyl)phenyl)ethynyl) benzoate, 2-(1H-indol-6-yl)-3-((4-(2-oxo-2-(pyridin-3-ylamino)ethyl)phenyl)ethynyl) benzoic acid, methyl 6-((4-(2-(pyridin-3-yl)acetamido)phenyl)ethynyl)-[1,1'-biphenyl]-2-carboxylate, 6-((4-(2-(pyridin-3-yl)acetamido)phenyl)ethynyl)-[1,1'-biphenyl]-2-carboxylic acid, and methyl 3'-hydroxyl-6-((4-(3-(pyridin-3-yl)ureido)phenyl)ethynyl)-[1,1'-biphenyl]-2-carboxylate.

6. A pharmaceutical composition, comprising the compound of general formula (I), or its enantiomer, diastereomer, tautomer, salt, crystal form, solvate and/or isotope-substituted derivative according to any one of claims 1 to 5, and/or pharmaceutically acceptable carrier, excipient or diluent.

7. A method for treating and/or preventing a disease or disorder caused by EBNA1 activity, the method comprising administering to a subject an effective amount of at least one compound, or its enantiomer, diastereomer, tautomer, salt, crystal form, solvate and/or isotope-substituted derivative according to claims 1 to 5, or administering to a subject an effective amount of at least one pharmaceutical composition according to claim 6.

8. A method for treating and/or preventing nasopharyngeal carcinoma, the method comprising administering to a subject an effective amount of at least one compound, or its enantiomer, diastereomer, tautomer, salt, crystal form, solvate and/or isotope-substituted derivative according to claims 1 to 5, or administering to a subject an effective amount of at least one pharmaceutical composition according to claim 6.

9. The method according to claim 7, wherein the disease or disorder caused by EBNA1 activity is cancer, infectious mononucleosis, chronic fatigue syndrome, multiple sclerosis, systemic lupus erythematosus or rheumatoid arthritis.

10. The method according to claim 9, wherein the cancer is nasopharyngeal carcinoma, non-Hodgkin's lymphoma, anaplastic large cell lymphoma, angioimmunoblastic T-cell lymphoma, hepatosplenic T-cell lymphoma, B-cell lymphoma, Burkitt's lymphoma, reticuloendothelial proliferation, reticulocytosis, diffuse large B-cell lymphoma, extranodal T/NK lymphoma/angiocentric lymphoma, follicular lymphoma, immunoblastic lymphoma, mucosa-associated lymphoma tissue lymphoma, B-cell chronic lymphocytic leukemia, mantle cell lymphoma, mediastinal large B-cell lymphoma, lymphoplasmacytic lymphoma, lymph node marginal zone B-cell lymphoma, splenic marginal zone lymphoma, intravascular large B-cell lymphoma, primary effusion lymphoma, lymphomatoid granuloma, angioimmunoblastic lymphadenopathy, X-linked lymphoproliferative disease, post-transplant lymphoproliferative disease, or Hodgkin lymphoma.
